(19) Europäisches Patentamt / European Patent Office / Office européen des brevets

(11) **EP 2 927 692 B1**

(12) **EUROPEAN PATENT SPECIFICATION**

(45) Date of publication and mention
of the grant of the patent:
**27.09.2017 Bulletin 2017/39**

(51) Int Cl.:
*G01N 33/68* (2006.01)    *G06F 19/00* (2011.01)

(21) Application number: **14163631.6**

(22) Date of filing: **04.04.2014**

(54) **Multiple biomarker approach for prediction of mortality in dialysis patients**

Ansatz mit mehreren Biomarkern zur Vorhersage der Mortalität bei Dialysepatienten

Approche de biomarqueurs multiples pour la prédiction de la mortalité chez des patients dialysés

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**07.10.2015 Bulletin 2015/41**

(73) Proprietors:
• **März, Winfried**
**69493 Hirschberg/Leutershausen (DE)**
• **Wanner, Christoph**
**97204 Höchberg (DE)**
• **Drechsler, Christiane**
**97078 Würzburg (DE)**

(72) Inventors:
• **März, Winfried**
**69493 Hirschberg/Leutershausen (DE)**
• **Wanner, Christoph**
**97204 Höchberg (DE)**
• **Drechsler, Christiane**
**97078 Würzburg (DE)**

(74) Representative: **Nottrott, Stephanie**
**Isenbruck Bösl Hörschler LLP**
**Patentanwälte**
**Prinzregentenstraße 68**
**81675 München (DE)**

(56) References cited:
**WO-A1-2007/001969    WO-A1-2011/098519**

**WO-A2-2012/075222    WO-A2-2013/010085**
**US-A1- 2012 103 072**

• JUAN J CARRERO ET AL: "Protein-energy wasting modifies the association of ghrelin with inflammation, leptin, and mortality in hemodialysis patients", KIDNEY INTERNATIONAL, vol. 79, no. 7, 22 December 2010 (2010-12-22), pages 749-756, XP055145944, ISSN: 0085-2538, DOI: 10.1038/ki.2010.487
• BABER U ET AL: "Thrombolysis In Myocardial Infarction (TIMI) Risk Score and Mortality in Patients With Advanced Chronic Kidney Disease and on Dialysis", AMERICAN JOURNAL OF CARDIOLOGY, CAHNERS PUBLISHING CO., NEWTON, MA, US, vol. 103, no. 11, 1 June 2009 (2009-06-01) , pages 1513-1517, XP026130635, ISSN: 0002-9149, DOI: 10.1016/J.AMJCARD.2009.01.364 [retrieved on 2009-04-08]
• BREDA PEÄ OVNIK BALON ET AL: "Fetuin-A as a risk factor for mortality in hemodialysis patients", WIENER KLINISCHE WOCHENSCHRIFT ; THE MIDDLE EUROPEAN JOURNAL OF MEDICINE, SPRINGER-VERLAG, VI, vol. 122, no. 2, 1 May 2010 (2010-05-01), pages 63-67, XP019850828, ISSN: 1613-7671
• HUANG CHIN-CHOU ET AL: "The beneficial effects of statins in patients undergoing hemodialysis", INTERNATIONAL JOURNAL OF CARDIOLOGY, vol. 168, no. 4, 5 August 2013 (2013-08-05), pages 4155-4159, XP028757300, ISSN: 0167-5273, DOI: 10.1016/J.IJCARD.2013.07.115

EP 2 927 692 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to the field of laboratory diagnostics. Specifically, the present invention relates to methods for determining the risk of mortality in dialysis patients by investigating biomarkers from various pathophysiological pathways. These methods allow for the reduction of the patient's risk of mortality by providing suitable and improved medical treatments to the patient.

An aim of modern medicine is to provide personalized or individualized treatment regimens. Those are treatment regimens which take into account a patient's individual needs or risks. Individualized treatment regimens offer benefits for the individual patient as well as for society as a whole. For the individual patient personalized treatment avoids excessive therapy while ensuring that necessary measures are taken. As every therapy may cause undesired harmful side effects, the avoidance of unnecessary therapies saves the patient from potentially harmful side effects. On the other hand, the identification of patients with special needs ensures that these individuals receive the appropriate treatment. For the health system as a whole the avoidance of unnecessary therapies allows for a more economic use of resources.

Individualized treatment regimens require appropriate diagnostic tools in order to separate those patients who benefit from certain therapeutic measures from the patients who do not. Therefore, the development of individualized treatment regimens critically depends on the development of novel diagnostic tools and procedures. Because the prevention of future disease is often more effective than the therapy of already existing disease, diagnostic tools and methods for risk stratification with respect to future diseases are especially desirable.

There is broad evidence that dialysis patients are at high risk for cardiovascular disease and mortality. So far, the risk assessment of mortality in dialysis patients relied on traditional cardiovascular risk factors. However, numerous novel biomarkers have been found to be independent predictors of cardiovascular disease, which might significantly improve risk prediction in patients undergoing hemodialysis. Cardiovascular diseases are amongst the major causes of death in industrialized countries and often a cause of death in dialysis patients. However, despite highly effective measures to control conventional risk factors the incidence of cardiovascular events remains high. This highlights the need to identify independent non-conventional risk factors for calculating and determining the risk of mortality in dialysis patients.

A multicenter, randomized, double-blind prospective study of subjects with type 2 diabetes mellitus receiving maintenance hemodialysis who were randomly assigned to receive atorvastatin per day or matching placebo has been described (Wanner et al., 2005, N Engl J Med 353:3, 238-248).

Methods for determining the overall survival of maintenance hemodialysis patients including measuring low density lipoprotein (LDL) particle size and subfraction concentrations as prognostic tools for early mortality risk detection have been described (Kamyar Kalantar-Zadeh et al. (2012) US 2012/0103072 A1). Clinical and biochemical characteristics in hemodialysis patients, according to ghrelin and nutritional status have been disclosed (Carrero et al., Kidney International (2011) 79: 749-756). Homoarginine as a biomarker for the risk of mortality is known in the art (Maerz et al. (2011) WO 2011/098519 A1). Thrombolysis in myocardial infarction (TIMI) risk score and mortality in patients with advanced chronic kidney disease an on dialysis has been described (Baber et al. (2009), Am J of Cardiology, 103: 1513-1517). Also described in the art is a method of identifying a patient undergoing periodic hemodialysis treatments at increased risk for death comprising determining clinical or biochemical parameters (Kotanko et al. (2012) WO 2012/075222 A2), including methods and kits for reducing the risk of mortality of dialysis by assaying endogenous vitamin D levels in the serum or plasma of dialysis patients or in patients in need of dialysis (Thadhani et al. (2007) WO 2007/001969 A1). Further, a method for diagnosing kidney disease, determining the likelihood of developing kidney disease or determining the severity of kidney disease in a subject comprising determining the fraction of carbamylated albumin in a subject has been described (Berg et al. (2013) WO 2013/010085 A2). Fetuin-A has been implicated as a risk factor for mortality in hemodialysis patients (Balon et al. (2010) Wien Klin Wochenschr 122: 63-67), while the beneficial effects of statins in patients undergoing hemodialysis have further been described in the art (Huang et al. (2013), International Journal of Cardiology 168: 4155-4159).

Despite advances in renal replacement therapy, mortality of dialysis patients remains excessive. Sudden cardiac death (SCD) has been described as a major contributor to the excess mortality of patients on maintenance dialysis (Drechsler et al., 2011, Eur J Heart Failure 13, 852-859). Risk stratification is in general done by using classic, Framingham-type risk factors such as hypertension, diabetes mellitus, hypercholesterolemia, family history of cardiovascular disease and smoking. These predictors allow for a good stratification of individuals regarding their risk of cardiovascular disease and death. Risk prediction in dialysis patients, however, may not be based on the classic risk factors alone, as different and uremia-specific risk factors seem to play a more important role.

Therefore, there is always a need for providing improved methods of risk prediction including improved methods of risk assessment in patients undergoing hemodialysis.

In a first aspect, the invention provides a method for determining the risk of mortality in dialysis patients, comprising the steps of

a) analysing a set of biological parameters obtained from the patient or from the patient's file history, wherein the biological parameters are selected from the group consisting of:

the patient's age,
the patient's gender,
the patient's body mass index,
the patient's use of statin,
at least one indicator of oxygen transport in the blood,
at least one indicator of calcium-phosphate homeostasis,
at least one indicator of bone metabolism,
at least one indicator of immune function,
at least one indicator of skeletal muscle metabolism,
at least one indicator of lipid metabolism,
at least one indicator of carbohydrate metabolism,
at least one indicator of hormonal blood pressure regulation,
at least one indicator of vascular inflammation,
at least one indicator of myocardial performance,
at least one indicator of myocardial integrity,
at least one indicator of sudden cardiac death,
at least one indicator of renal function,
at least one indicator of blood pressure regulation, and
at least one indicator of hepatic function,
or a subset thereof;

b) setting the biological parameters or a subset thereof into correlation, wherein the correlation of a subset of biological parameters provides a risk score indicative for the patient's mortality within the next year, wherein the biological parameters are selected from the group consisting of

the patient's age,
the patient's gender,
the patient's body mass index,
at least one indicator of skeletal muscle metabolism,
at least one indicator of sudden cardiac death,
at least one indicator of renal function,
at least one indicator of hormonal blood pressure regulation,
at least one indicator of bone metabolism,
at least two indicators of hepatic function,

wherein
the at least one indicator of skeletal muscle metabolism is defined by the patient's blood concentration or activity of creatine kinase,
the at least one indicator of sudden cardiac death is defined by the patient's blood concentration of homoarginine,
the at least one indicator of renal function is defined by the patient's blood concentration of carbamylated albumin,
the at least one indicator of hormonal blood pressure regulation is defined by the patient's blood concentration of aldosterone or copeptin (carboxy-terminal-pro arginin vasopressin),
the at least one indicator of bone metabolism is defined by the patient's blood concentration or activity of alkaline phosphatase, and
the at least two indicators of hepatic function are defined by the patient's blood concentration of transthyretin and fetuin,
wherein the risk score is a weighted risk score $Y_1$ indicative for the patient's risk of mortality within the next year, and wherein the biological parameters are set into correlation by using the following formula, thereby providing the weighted risk score $Y_1$:

$$Y_1 = 0.51 * \text{variable 1} + (-0.49 * \text{variable 2}) + (-0.32 * \text{variable 3}) + 0.44 * \text{variable 4}$$

$$+ (-0.32 * \text{variable 5}) + (-0.29 * \text{variable 6}) + (-0.33 * \text{variable 7}) + 0.35 * \text{variable 8}$$

$$+ (-0.4 * \text{variable 9}) + 0.31 * \text{variable 10}$$

wherein

variable 1 is defined by the log value of the patient's age in years,

variable 2 is defined as equal to 1 if the patient's gender is female and as equal to 0 if the patient's gender is male,

variable 3 is defined by the log value of the patient's body mass index in kg/m$^2$,

variable 4 is defined by the log value of the patient's blood activity of alkaline phosphatase in U/L,

variable 5 is defined by the log value of the patient's blood activity of creatine kinase in U/L,

variable 6 is defined by the log value of the patient's blood concentration of homoarginine in micromol/L,

variable 7 is defined by the log value of the patient's blood concentration of fetuin in ng/mL,

variable 8 is defined by the log value of the patient's blood concentration of carbamylated albumin in percent (%),

variable 9 is defined by the log value of the patient's blood concentration of transthyretin in micromol/L, and

variable 10 is defined by the log value of the patient's blood concentration of aldosterone in pg/mL,

wherein all log values are calculated to the basis e, and wherein all numeric values in the formula providing the weighted risk score $Y_1$ are calculated per standard deviation, preferably wherein the numeric values are defined with a deviation of 50 %, preferably of 20 %, more preferably of 10 %, and most preferably of 5 %,

and wherein the risk score is indicative for a medical treatment to reduce the patient's risk of mortality.

The term "determining the risk of mortality" as used herein refers to assessing the probability according to which a subject will die within a certain time window, i.e. the predictive window. In accordance with the present invention, the predictive window is preferably within 1 year, 3 years, 5 years, 8 years, or 10 years or more after determination of the risk of mortality. However, as will be understood by those skilled in the art, such an assessment is usually not intended to be correct for 100 % of the subjects to be investigated. The term, however, requires that prediction can be made for a statistically significant portion of subjects in a proper and correct manner. Whether a portion is statistically significant can be determined without further ado by the person skilled in the art using various well known statistic evaluation tools, e.g., the Mann-Whitney-U test, Cox proportional hazard regression analysis, Pearson correlation and Harrell's C-statistic. Details are, for example, found in Dowdy and Wearden, Statistics for Research, John Wiley & Sons, New York 1983. Continuous data are presented as median and interquartile range, discrete data as counts and percentages. Continuous variables preferably undergo log transformation before entering analysis. Preferred confidence intervals are at least 95 %, at least 97 %, at least 98 % or at least 99 %. The p-values are, preferably, 0.005, or 0.0001. The discriminatory power of the designed risk score is assessed using Harrell's C-statistic. The improvement of individual risk prediction is preferably examined by net reclassification improvement. Preferably, the probability envisaged by the present invention allows that the prediction of an increased, normal or decreased risk will be correct for at least 60 %, at least 70 %, at least 80 %, or at least 90 %, and preferably of at least 95 % of the subjects of a given cohort or population. Determining the risk of mortality preferably relates to determining whether or not there is an increased risk of mortality compared to the average risk of mortality in a population of subjects rather than giving a precise probability for the said risk.

The term "risk score" as used herein generally means an indicator for the risk of mortality with respect to a certain period of time. In particular, the risk score of the present invention provides an estimate of how long the patient is expected to survive a certain period of time, or alternatively, of how likely it is that the patient will die within this time window. Therefore, in the context of the present invention, the risk score can either be seen as an indicator of a particular survival or mortality rate.

The method of the present invention is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison in step (b).

In the context of the present invention, the risk score is indicative for all-cause mortality of the patient. The term "all-cause mortality" generally refers to all kinds of death due to any cause.

In a preferred embodiment, the risk score determined by the methods of the present invention is indicative for the patient's mortality due to cardiovascular events or due to combined fatal and non-fatal cardiovascular events.

The term "cardiovascular events" generally means all cases of death caused and/or related to cardiovascular diseases, preferably caused by diseases of the arterial tree such as, for example, atherosclerosis and/or thrombosis.

The term "patient" as used herein generally refers to a mammal, preferably to a human.

The term "biological parameter" as used in the context of the present invention generally includes all kind of gene expression products which are differentially expressed, i.e. up regulated or down regulated in presence or absence of a certain condition, disease, or physical situation. A biological parameter of the present invention is also referred to as a biomarker. A biological parameter according to the present invention may also mean a variable in form of the patient's age, the patient's gender, the patient's body mass index and/or the patient's heart rate or the patient's intake of drugs. Equally preferred is that a biological parameter according to the present invention may be in form of a protein product, preferably in form of an enzyme, a nucleic acid (including mRNA), a peptide or protein, or a small molecule compound, in particular in form of its concentration or amount. The amount of a suitable biological parameter can indicate the presence or absence of the condition, disease, or a physical state, and thus allow for a particular diagnosis. For example, biological parameters reflecting myocardial performance, inflammation, lipid metabolism, renal function, hepatic function and/or cardiovascular function including their analysis by means of commercially available routine assay systems and antibodies are well known in the art (see, for example, Schnabel et al., 2010. Eur Heart J, 31: 3024-3031).

Biological parameters according to the present invention also include, but are not limited to, indicators of oxygen transport in the blood, indicators of calcium-phosphate homeostasis, indicators of bone metabolism, indicators of immune function, indicators of skeletal muscle metabolism, indicators of lipid or lipoprotein metabolism, indicators of carbohydrate metabolism, indicators of hormonal blood pressure regulation, indicators of vascular inflammation, indicators of myocardial performance, indicators of myocardial integrity, indicators of sudden cardiac death, indicators of renal function, indicators of blood pressure regulation, and indicators of hepatic function. Such definition of indictors is well known to the skilled person and these indicators are analysed on a routine basis by physicians and/or in clinical assays.

The term "indicator of myocardial performance" as used in the context of the present invention, for example, generally refers to any biological parameter which provides information about the medical condition of the patient's heart. In particular, an indicator of myocardial performance means a biological parameter that provides information about the patient's cardiovascular function. Examples of biological parameters which indicate myocardial performance according to the present invention include, but are not limited to, copeptin, C-terminal-pro-endothelin-1, mid-regional-pro-adrenomedullin (MR-proADM), mid-regional-pro-atrial natriuretic peptide (MR-proANP), and N-terminal-pro-B-type natriuretic peptide (NT-proBNP).

Alternatively preferred is that the "indicator of myocardial performance" is defined by the patient's blood concentration of natriuretic peptides. The term "natriuretic peptides" generally refers all known atrial natriuretic peptides (ANP) and brain natriuretic peptides (BNP) described in the art (Bonow 1996, Circulation 93, 1946), including, for example, pre-proANP, proANP, NT-proANP, ANP and pre-proBNP, proBNP, NT-proBNP, and BNP.

The pre-pro peptide (134 amino acids in the case of pre-proBNP) comprises a short signal peptide, which is enzymatically cleaved releasing the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferably, natriuretic peptides according to the present invention are NT-proANP, ANP, and, more preferably, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol 167, 239). Preanalytics are more robust with NT-proBNP allowing easy transportation of the sample to a central laboratory (Mueller 2004, Clin Chem Lab Med 42, 942). Blood samples can be stored at room temperature for several days or may be mailed or shipped without recovery loss. In contrast, storage of BNP for 48 hours at room temperature or at 4° Celsius leads to a concentration loss of at least 20 % (Wu 2004, Clin Chem 50, 867). Therefore, depending on the time-course or properties of interest, either measurement of the active or the inactive forms of the natriuretic peptide can be advantageous. Preferred natriuretic peptides according to the present invention are NT-proBNP or variants thereof. The human NT-proBNP, is a polypeptide comprising, preferably, 76 amino acids in length corresponding to the N-terminal portion of the human NT-proBNP molecule. An alternatively preferred natriuretic peptide is the mid-regional-pro-atrial natriuretic peptide (MR-proANP). Methods for measurement natriuretic peptides have been described (Ala-Kopsala 2004, Clin Chem 50, 1576), and the concentration of the natriuretic peptides is preferably calculated in pg/ml, but may also be indicated by any other suitable concentration format, such as, for example, pmol/dl.

An "indicator of renal function" as used herein generally means any biological parameter which provides information about the medical condition of the patient's kidney(s). In particular, an indicator of renal function is a biological parameter or a biomarker that provides information about the function of the patient's kidney and/or about the quality of dialysis. Examples of such biological parameters include, but are not limited to, cystatin C, serum creatinine, renin and carbamylated albumin (Schnabel et al., 2010. Eur Heart J; 31: 2024-3031).

Preferably, the indicator of renal function is defined by the patient's blood concentration of carbamylated albumin. Carbamylated serum albumin is a risk factor for mortality in patients with end-stage renal disease (ESRD), which may be modifiable with amino acid therapy. Urea is known to promote the carbamylation of proteins, including human albumin. The concentration of carbamylated albumin can be measured in a blood sample by standard methods. The concentration of carbamylated albumin preferably calculated in mg/dL or g/dL, but may be indicated by any other suitable concentration format, such as, for example, pmol/dL.

Equally preferred is that the indicator of renal performance is defined by the patient's blood concentration of cystatin C and/or renin. Cystatin C is also referred to in the art as cystatin 3. Renin is an enzyme produced by the kidney. Cystatin C and renin are routinely used biomarkers for evaluating the kidney function and/or performance of a patient. The concentration of cystatin C in serum can be defined by standard methods known in the art and is preferably measured, calculated and indicated in mg/L. The same applies for the concentration of renin in a patient's blood sample, the concentration of which is preferably indicated in pg/mL, μg/mL, or equivalently in mU/L, or U/L.

The patient's gender may be male or female.

The patient's age is defined by the patient's age in years.

The patient's body mass index defines a measure for the patient's body shape based on the patient's individual's mass and height. The body mass index is defined as the individual's body mass divided by the square of their height with the value universally being given in units of $kg/m^2$.

The patient's use of statin means the use of medical drugs in form of statins or HMG-CoA reductase inhibitors which are a class of drugs used to lower cholesterol levels inhibiting the enzyme HMG-CoA reductase, which plays a central role in the production of cholesterol in the liver. Statins including their administration to patients is medial routine and known to the skilled person and physician.

In a preferred embodiment, the present invention provides a method for determining the risk of mortality in dialysis patients, wherein the correlation of a subset of biological parameters indicated above provides a risk score indicative for the patient's mortality within the next year, wherein the biological parameters are selected from the group consisting of

> the patient's age,
> the patient's gender,
> the patient's body mass index,
> at least one indicator of skeletal muscle metabolism,
> at least one indicator of sudden cardiac death,
> at least one indicator of renal function,
> at least one indicator of hormonal blood pressure regulation,
> at least one indicator of bone metabolism,
> at least two indicators of hepatic function,

wherein
the at least one indicator of skeletal muscle metabolism is defined by the patient's blood concentration or activity of creatine kinase,
the at least one indicator of sudden cardiac death is defined by the patient's blood concentration of homoarginine,
the at least one indicator of renal function is defined by the patient's blood concentration of carbamylated albumin,
the at least one indicator of hormonal blood pressure regulation is defined by the patient's blood concentration of aldosterone or copeptin (carboxy-terminal-pro arginine vasopressin),
the at least one indicator of bone metabolism is defined by the patient's blood concentration or activity of alkaline phosphatase, and
the at least two indicators of hepatic function are defined by the patient's blood concentration of transthyretin and fetuin.

The "patient's blood concentration" as referred to herein preferably means the concentration of a biological parameter as determined from a sample of blood which has been obtained from the patient by standard routine practice. It the context of the present invention, however, it is also included that the biological parameter(s) of interest is/are determined by the use of any other sample of the patient as input material, such as, for example, a sample of a body fluid, a sample of separated cells or a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which produce the marker referred to herein.

It is also envisaged that the risk score is provided by means of a simplified risk score, wherein the biological parameter indicated above or a subset thereof are set into correlation by a sum of individual quantities, wherein the number of individual quantities is defined by the number of biological parameter and wherein the sum of this quantities provides the simplified risk score. For defining such a simplified risk score, the risk of mortality is calculated by dividing the number of patients who died within a certain time window, in particular within the time window of 1, 3, 5, or 10 years, by the number of patients which had initially been classified with this risk score. Thereby, different risk scores could be correlated with different risks of mortality, in particular with different risk score classes.

The invention provides a method for determining the risk of mortality in dialysis patient, wherein the risk score is a weighted risk score $Y_1$ indicative for the patient's risk of mortality within the next year, and wherein the biological parameters are set into correlation by using the following formula, thereby providing the weighted risk score $Y_1$:

$$Y_1 = 0.51 * \text{variable 1} + (-0.49 * \text{variable 2}) + (-0.32 * \text{variable 3}) + 0.44 * \text{variable 4} + (-0.32 * \text{variable 5}) + (-0.29 * \text{variable 6}) + (-0.33 * \text{variable 7}) + 0.35 * \text{variable 8} + (-0.4 * \text{variable 9}) + 0.31 * \text{variable 10}$$

wherein

variable 1 is defined by the log value of the patient's age in years,

variable 2 is defined as equal to 1 if the patient's gender is female and as equal to 0 if the patient's gender is male,

variable 3 is defined by the log value of the patient's body mass index in kg/m$^2$,

variable 4 is defined by the log value of the patient's blood activity of alkaline phosphatase in U/L,

variable 5 is defined by the log value of the patient's blood activity of creatine kinase in U/L,

variable 6 is defined by the log value of the patient's blood concentration of homoarginine in micromol/L,

variable 7 is defined by the log value of the patient's blood concentration of fetuin in ng/mL,

variable 8 is defined by the log value of the patient's blood concentration of carbamylated albumin in percent (%),

variable 9 is defined by the log value of the patient's blood concentration of transthyretin in micromol/L, and

variable 10 is defined by the log value of the patient's blood concentration of aldosterone in pg/mL,

wherein all log values are calculated to the basis e, and wherein all numeric values in the formula providing the weighted risk score $Y_1$ are calculated per standard deviation, preferably wherein the numeric values are defined with a deviation of 50 %, preferably of 20 %, more preferably of 10 %, and most preferably of 5 %.

Here, the "numeric values in the formula providing the weighted risk score $Y_1$" mean the following numeric values: 0.51, 0.49, 0.32, 0.44, 0.32, 0.29, 0.33, 0.35, 0.4, and 0.31.

Equally preferred is that all numeric values in the formula providing the weighted risk score $Y_1$ are defined without any deviation.

Moreover, in the context of the present invention, all log values are calculated to the basis e.

It has surprisingly been found in the context of the present invention that the weighted risk score $Y_1$ can be correlated with different classes of the risk of mortality, preferably with a class of low risk of mortality, a class of moderate risk of mortality, and a class of high risk of mortality.

Therefore, in a preferred embodiment, a weighted risk score $Y_1$ in the range of less than or equal to 0 indicates a low risk of mortality, a weighted risk score $Y_1$ in the range of 0 to 1 indicates a moderate risk of mortality, and a weighted risk score $Y_1$ in the range of equal to or more than 1 indicates a high risk or mortality.

In the context of the present invention, it is to be understood that the term "equal to" may comprise a deviation of $\pm$ 0.5, preferably of $\pm$ 0.25, more preferably of $\pm$ 0.1.

Moreover, it is to be understood that those patients with a weighted risk score $Y_1$ in the range of less than or equal to 0 fall into the 1st tertile of the given cohort or population (minimum to 33rd percentile), those patients with a weighted risk score $Y_1$ in the range of 0 to 1 fall into the 2nd tertile of the cohort (33rd percentile to 66th percentile), while

those patients with a weighted risk score $Y_1$ in the range of more than or equal to 1 fall into the 3rd tertile of the cohort (66th percentile to maximum).

The term "percentile" generally means the value of a variable below which a certain percent of observations fall. For example, the 33rd percentile is the value below which 33 percent of the observations may be found. In the present invention, the cut off values off the 1st, 2nd and 3rd tertile have been determined as exemplified in the examples of the present invention.

In the context of the present invention, is has been found that a particular risk score can be correlated with a particular risk class of mortality, preferably with a class of very low risk of mortality, a class of low risk of mortality, a class of intermediate (or moderate) risk of mortality and/or with a class of high risk of mortality.

This classification is applicable to all mortality risk scores of the present invention, in particular to the weighted risk scores $Y_1$ and $Y_2$ as exemplified by the examples of the present invention.

Therefore, a risk score of equal to 0 or equal to 1 indicates a low risk of mortality, while a simplified risk score of equal to 2 or equal to 3 indicates an moderate risk of mortality, and a simplified risk score of equal to or more than 4 indicates a high risk of mortality.

Equally preferred is that a calculated risk score of equal to 1 or less indicates a very low risk of mortality, of equal to 2 indicates a low risk of mortality, of equal to 3 indicates an intermediate risk of mortality, and of equal to 4 or more indicates a high risk of mortality.

Here, a low risk of mortality preferably means a risk of mortality of equal to or below 20 %, an intermediate risk of mortality means a risk of mortality of equal to or below 40 %, while a high risk of mortality means a risk of mortality of at least 50 %, preferably of between 50 % and 60 %.

In a further preferred embodiment, the present invention provides a method for determining the risk of mortality in dialysis patients, wherein the correlation of a subset of the biological parameters indicated above provides a risk score indicative for the patient's risk of mortality within the next five year, wherein the biological parameters are selected from the group consisting of

the patient's age,
the patient's gender,
at least one indicator of bone metabolism,
at least one indicator of skeletal muscle metabolism,
at least one indicator of myocardial performance,
at least one indicator of myocardial integrity,
at least one indicator of renal function, and
at least one indicator of inflammation,

wherein
the at least one indicator of bone metabolism is defined by the patient's blood concentration or activity of alkaline phosphatase,
the at least one indicator of skeletal muscle metabolism is defined by the patient's blood concentration or activity of creatine kinase,
the at least one indicator of myocardial performance is defined by the patient's blood concentration of NT-proBNP,
the at least one indicator of myocardial integrity is defined by the patient's blood concentration of troponin T or troponin I,
the at least one indicator of renal function is defined by the patient's blood concentration of carbamylated albumin,
the at least one indicator of inflammation is defined by the patient's blood concentration of c-reactive protein (CRP,
wherein the risk score is a weighted risk score $Y_2$ indicative for the patient's risk of mortality within the next five years,
and wherein the biological parameters are set into correlation by using the following formula, thereby providing the weighted risk score $Y_2$:

$$Y_2 = 0.38 * \text{variable 1} + (-0.1 * \text{variable 2}) + 0.21 * \text{variable 3} + (-0.34 * \text{variable 4}) + 0.18 * \text{variable 5} + 0.16 * \text{variable 6} + 0.26 * \text{variable 7} + 0.16 * \text{variable 8}$$

wherein

variable 1 is defined by the log value of the patient's age in years,

variable 2 is defined as equal to 1 if the patient's gender is female and as equal to 0 if the patient's gender is male,

variable 3 is defined by the log value of the patient's blood activity of alkaline phosphatase in U/L,

variable 4 is defined by the log value of the patient's blood activity of creatine kinase in U/L,

variable 5 is defined by the log value of the patient's blood concentration of NT-proBNP in pg/mL,

variable 6 is defined by the log value of the patient's blood concentration of c-reactive protein (CRP) in mg/L,

variable 7 is defined by the log value of the patient's blood concentration of troponin T in ng/mL, and

variable 8 is defined by the log value of the patient's blood concentration of carbamylated albumin in percent (%),

wherein all log values are calculated to the basis e, and wherein all numeric values in the formula providing the weighted risk score $Y_2$ are calculated per standard deviation, preferably wherein the numeric values are defined with a deviation of 50 %, preferably of 20 %, more preferably of 10 %, and most preferably of 5 %.

Here, the "numeric values in the formula providing the weighted risk score $Y_2$" mean the following numeric values: 0.38, 0.21, 0.34, 0.18, 0.16, 0.26, and 0.16.

Equally preferred is that all numeric values in the formula providing the weighted risk score $Y_2$ are defined without any deviation.

Moreover, in the context of the present invention, all log values are calculated to the basis e.

In a preferred embodiment, the invention provides a method for determining the risk of mortality in dialysis patients, comprising the steps of

a) analysing a set of biological parameters obtained from the patient or from the patient's file history, wherein the biological parameters are selected from the group consisting of

the patient's age,
the patient's gender,
the patient's body mass index,
the patient's use of statin,
at least one indicator of oxygen transport in the blood,
at least one indicator of calcium-phosphate homeostasis,
at least one indicator of bone metabolism,
at least one indicator of immune function,
at least one indicator of skeletal muscle metabolism,
at least one indicator of lipid metabolism,
at least one indicator of carbohydrate metabolism,
at least one indicator of hormonal blood pressure regulation,
at least one indicator of vascular inflammation,
at least one indicator of myocardial performance,
at least one indicator of myocardial integrity,
at least one indicator of sudden cardiac death,
at least one indicator of renal function,
at least one indicator of blood pressure regulation, and
at least one indicator of hepatic function;

b) setting the biological parameters or a subset thereof into correlation, thereby providing a risk score, wherein the risk score is indicative for all-cause mortality of the patient, and wherein the risk score is indicative for a medical treatment to reduce the patient's risk of all-cause mortality,
wherein the correlation of a subset of biological parameters provides a risk score indicative for the patient's risk of mortality within the next year, wherein the biological parameters are selected from the group consisting of

the patient's age,
the patient's gender,
the patient's body mass index,
the patient's use of a statin,
at least one indicator of oxygen transport in the blood,
at least one indicator of calcium-phosphate homeostasis,
at least one indicator of electrolyte homoestasis,

at least one indicator of bone metabolism,
at least one indicator of immune function,
at least one indicator of skeletal muscle metabolism,
at least one indicator of lipid metabolism,
at least one indicator of carbohydrate metabolism,
at least one indicator of hormonal blood pressure regulation,
at least one indicator of vascular inflammation,
at least one indicator of myocardial performance,
at least one indicator of myocardial integrity,
at least one indicator of sudden cardiac death,
at least one indicator of renal function, and
at least one indicator of hepatic function,

wherein

the at least one indicator of lipid metabolism is defined by the patient's blood concentration of LDL cholesterol,
the at least one indicator of oxygen transport is defined by the patient's blood concentration of hemoglobin,
the at least one indicator of carbohydrate metabolism is defined by the patient's blood concentration of glycated haemoglobin or blood, serum or plasma glucose,
the at least one indicator of calcium-phosphate homeostasis is defined by the patient's blood concentration of phosphate,
the at least one indicator of bone metabolism is defined by the patient's blood concentration of alkaline phosphatase and/or by the patient's blood concentration of osteoprotegerin and/or by the patient's blood concentration of vitamin D,
the at least one indicator of skeletal muscle metabolism is defined by the patient's blood concentration or activity of creatine kinase,
the at least one indicator of immune function is defined by the patient's blood concentration of white blood cells and/or cortisol and/or soluble suppression of Tumorigenicity 2 (sST2),
the at least one indicator of hepatic function is defined by the patient's blood concentrations or activities of alanine aminotransferase (ALAT) and/or aspartate aminotransferase (ASAT) and/or fetuin and/or transthyretin,
the at least one indicator of electrolyte homeostasis is defined by the patient's blood concentration of potassium,
the at least one indicator of myocardial function is defined by the patient's blood concentration of a natriuretic peptide,
the at least one indicator of vascular inflammation is defined by the patient's blood concentration or activity of lipoprotein associated phospholipase A2,
the at least one indicator of sudden cardiac death is defined by the patient's blood concentration of homoarginine,
the at least one indicator of myocardial integrity is defined by the patient's blood concentration of troponin T or troponin I,
the at least one indicator of hormonal blood pressure regulation is defined by the patient's blood concentration of aldosterone and or of copeptin,
the at least one indicator of renal function is defined by the patient's blood concentration of carbamylated albumin,
wherein the risk score is a weighted risk score $Y_3$ indicative for the patient's risk of mortality within the next year,
and wherein the biological parameters are set into correlation by using the following formula, thereby providing the weighted risk score $Y_3$:

$Y_3$ = 0,057410212 * variable 1 - 0,543915506 * variable 2 - 0,143998352 * variable 3 - 0,102420371 * variable 4 + 0,002126855 * variable 4 * variable 4 + 0,028997043 * variable 5 - 0,000102574 * variable 5 * variable 5 - 0,144446014* variable 6 + 0,500608722 * variable 7 - 0,032782705 * variable 7 * variable 7 + 0,141166863 * variable 8 - 0,004400439 * variable 9 + 4,87592E-05 * variable 9 * variable 9 + -4,08374E-08 * variable 9 * variable 9 * variable 9 - 0,007492671* variable 10 + 8,65369E-06 * variable 10 * variable 10 + 0,23222716 * variable 11 - 0,006951655 * variable 11 * variable 11 - 0,073850191 * variable 12 + 0,001124082 * variable 12 * variable 12 + 2,88445E-05 * variable 12 * variable 12 * variable 12 + 0,020974682 * variable 13 - 0,001555684 * variable 13 * variable 13 + 0,064258442 * variable 14 - 0,000208666 * variable 14 * variable 14 -0,439032703 * variable 15 + 0,064866606 * variable 15 * variable 15 + 5,67869E-05 * variable 16 -1,15087E-09 * variable 16 * variable 16 - 0,043785154 * variable 17 + 6,01674E-05 variable 17 * variable 17 - 2,2989E-08 * variable 17 * variable 17 * variable 17 - 0,035837307 * variable 18 + 0,000195508 * variable 18 * variable 18 + 1,747437912 * variable 19 - 0,30324001 * variable 19 *

variable 19 + 3,652306243 * variable 20 - 1,722577314 * variable 20 * variable 20 - 0,069675 * variable 21 + 0,003134 * variable 21 * variable 21 - 0,054227857 * variable 22 + 0,026799499 * variable 23 - 0,000456787 * variable 23 * variable 23 - 1,12341E-05 * variable 23 * variable 23 * variable 23 - 0,373490667 * variable 24 - 0,006961823 * variable 24 * variable 24 + 0,000774153 * variable 25 + 0,238619449 * variable 26 - 0,006139105 * variable 26 * variable 26 + 8,53488E-05 * variable 27 + 0,016262 * variable 28 + 1,579488608 * variable 29,

wherein

variable 1 is defined by the patient's age in years,

variable 2 is defined as equal to 1 if the patient's gender is female and as equal to 0 if the patient's gender is male,

variable 3 is defined as equal to 2 if the patient uses a statin and as equal to 1 if the patient does not use a statin,

variable 4 as the patient's body mass index in kg/m$^2$

variable 5 is defined by the value of the patient's serum or plasma concentration of low density lipoprotein (LDL) cholesterol in mg/dl,

variable 6 is defined by the value of the patient's blood concentration of haemoglobin in g/dl,

variable 7 is defined by the value of the patient's blood serum or plasma concentration of glycated haemoglobin in percent (%),

variable 8 is defined by the value of the patient's blood serum or plasma concentration of phosphate in mg/dl,

variable 9 is defined by the value of the patient's serum or plasma activity of alkaline phosphatase in U/L,

variable 10 is defined by the value of the patient's serum or plasma activity of creatine kinase in U/L,

variable 11 is defined by the value of the patient's blood concentration of white blood cells (leucocytes) in 1000/$\mu$l,

variable 12 is defined by the value of patient's serum or plasma activity of aspartate aminotransferase (ASAT) in U/L,

variable 13 is defined by the value of patient's serum or plasma activity of alanine aminotransferase (ALAT) in U/L,

variable 14 is defined by the value of the patient's blood, plasma or serum concentration of glucose in mg/dl,

variable 15 is defined by the value of the patient's plasma or serum potassium in mmol/L,

variable 16 is defined by the value of the patient's plasma or serum concentration of N-terminal pro B-type natriuretic peptide in pg/ml,

variable 17 is defined by the value of the patient's plasma or serum concentration of lipoprotein associated phospholipase A2 in U/L,

variable 18 is defined by the value of the patient's plasma or serum concentration of C-reactive protein in mg/L,

variable 19 is defined by the value of the patient's plasma or serum concentration of troponin T in ng/mL,

variable 20 is defined by the value of the patient's plasma or serum concentration of homoarginine in micromol/L,

variable 21 is defined by the value of the patient's plasma or serum concentration of osteoprotegerin in picomol/L,

variable 22 is defined by the value of the patient's plasma or serum concentration of fetuin in ng/mL,

variable 23 is defined by the value of the patient's plasma or serum concentration of vitamin D in ng/ml,

variable 24 is defined by the value of the patient's plasma or serum concentration of transthyretin in micromol/L,

variable 25 is defined by the value of the patient's plasma or serum concentration of aldosterone in pg/mL,

variable 26 is defined by the value of the patient's plasma or serum concentration of cortisol microgram/dL,

variable 27 is defined by the value of the patient's plasma or serum concentration of copeptin (carboxy-terminal-pro arginin vasopressin) in pmol/L,

variable 28 is defined by the value of the patient's plasma or serum concentration of soluble suppression of tumorigenicity 2 (sST2) in ng/mL,

variable 29 is defined by the value of the patient's plasma or serum concentration of carbamylated albumin in percent (%),

wherein all numeric values are calculated per standard deviation, preferably wherein the numeric values are defined with a deviation of 50 %, preferably of 20 %, more preferably of 10 %, and most preferably of 5 %.

In another preferred embodiment, the invention provides a method wherein the correlation of a subset of biological parameters provides a risk score indicative for the patient's risk of mortality within the next five years, wherein the biological parameters are selected from the group consisting of

the patient's age,
the patient's gender,
the patient's body mass index,

the patient's use of a statin,
at least one indicator of oxygen transport in the blood,
at least one indicator of calcium-phosphate homeostasis,
at least one indicator of electrolyte homoestasis,
at least one indicator of bone metabolism,
at least one indicator of immune function,
at least one indicator of skeletal muscle metabolism,
at least one indicator of lipid metabolism,
at least one indicator of carbohydrate metabolism,
at least one indicator of hormonal blood pressure regulation,
at least one indicator of vascular inflammation,
at least one indicator of myocardial performance,
at least one indicator of myocardial integrity,
at least one indicator of sudden cardiac death,
at least one indicator of renal function, and
at least one indicator of hepatic function,

wherein
the at least one indicator of lipid metabolism is defined by the patient's blood concentration of LDL cholesterol,
the at least one indicator of oxygen transport is defined by the patient's blood concentration of hemoglobin,
the at least one indicator of carbohydrate metabolism is defined by the patient's blood concentration of glycated haemoglobin or blood, serum or plasma glucose,
the at least one indicator of calcium-phosphate homeostasis is defined by the patient's blood concentration of phosphate,
the at least one indicator of bone metabolism is defined by the patient's blood concentration of alkaline phosphatase and/or by the patient's blood concentration of osteoprotegerin and/or by the patient's blood concentration of vitamin D,
the at least one indicator of skeletal muscle metabolism is defined by the patient's blood concentration or activity of creatine kinase,
the at least one indicator of immune function is defined by the patient's blood concentration of white blood cells and/or cortisol and/or soluble suppression of Tumorigenicity 2 (sST2),
the at least one indicator of hepatic function is defined by the patient's blood concentrations or activities of alanine aminotransferase (ALAT) and/or aspartate aminotransferase (ASAT) and/or fetuin and/or transthyretin,
the at least one indicator of electrolyte homeostasis is defined by the patient's blood concentration of potassium,
the at least one indicator of myocardial function is defined by the patient's blood concentration of a natriuretic peptide,
the at least one indicator of vascular inflammation is defined by the patient's blood concentration or activity of lipoprotein associated phospholipase A2,
the at least one indicator of sudden cardiac death is defined by the patient's blood concentration of homoarginine,
the at least one indicator of myocardial integrity is defined by the patient's blood concentration of troponin T or troponin I,
the at least one indicator of hormonal blood pressure regulation is defined by the patient's blood concentration of aldosterone and or of copeptin,
the at least one indicator of renal function is defined by the patient's blood concentration of carbamylated albumin, wherein the risk score is a weighted risk score $Y_4$ indicative for the patient's risk of mortality within the next five years, and wherein the biological parameters are set into correlation by using the following formula, thereby providing the weighted risk score $Y_4$:

$$Y_4 = x1 * \text{variable 1} + x2 * \text{variable 2} + x3 * \text{variable 3} + x4 * \text{variable 4} + x5 * \text{variable 4} * \text{variable 4} + x6 * \text{variable 5} + x7 * \text{variable 5} * \text{variable 5} + x8 * \text{variable 6} + x9 * \text{variable 7} + x10 * \text{variable 7} * \text{variable 7} + x11 * \text{variable 8} + x12 * \text{variable 9} + x13 * \text{variable 9} * \text{variable 9} + x14 * \text{variable 9} * \text{variable 9} * \text{variable 9} + x15 * \text{variable 10} + x16 * \text{variable 10} * \text{variable 10} + x17 * \text{variable 11} + x18 * \text{variable 11} * \text{variable 11} + x19 * \text{variable 12} + x20 * \text{variable 12} * \text{variable 12} + x21 * \text{variable 12} * \text{variable 12} * \text{variable 12} + x22 * \text{variable 13} + x23 * \text{variable 13} * \text{variable 13} + x24 * \text{variable 14} + x25 * \text{variable 14} *$$

variable 14  + x26 * variable 15 + x27 * variable 15 * variable 15 + x28 * variable 16 + x29 * variable 16 * variable 16 + x30 * variable 17 + x31 variable 17 * variable 17 + x32 * variable 17 * variable 17 * variable 17 + x33 * variable 18 + x34 * variable 18 * variable 18 + x35 * variable 19 + x36 * variable 19 * variable 19 + x37 * variable 20 + x38 * variable 20 * variable 20  + x39 * variable 21 + x40 * variable 21 * variable 21 + x41 * variable 22 + x42 * variable 23 + x43 * variable 23 * variable 23 + x44 * variable 23 * variable 23 * variable 23 + x45 * variable 24 + x46 * variable 24 * variable 24 + x47 * variable 25 + x48 * variable 26 + x49 * variable 26 * variable 26 + x50 * variable 27 + x51 * variable 28 + x52 * variable 29,

wherein

variable 1 is defined by the patient's age in years,

variable 2 is defined as equal to 1 if the patient's gender is female and as equal to 0 if the patient's gender is male,

variable 3 is defined as equal to 2 if the patient uses a statin and as equal to 1 if the patient does not use a statin,

variable 4 as the patient's body mass index in kg/m$^2$

variable 5 is defined by the value of the patient's serum or plasma concentration of low density lipoprotein (LDL) cholesterol in mg/dl,

variable 6 is defined by the value of the patient's blood concentration of haemoglobin in g/dl,

variable 7 is defined by the value of the patient's blood serum or plasma concentration of glycated haemoglobin in percent (%),

variable 8 is defined by the value of the patient's blood serum or plasma concentration of phosphate mg/dl,

variable 9 is defined by the value of the patient's serum or plasma activity of alkaline phosphatase in U/L,

variable 10 is defined by the value of the patient's serum or plasma activity of creatine kinase in U/L.

variable 11 is defined by the value of the patient's blood concentration of white blood cells (leucocytes) in 1000/ μL,

variable 12 is defined by the value of patient's serum or plasma activity of aspartate aminotransferase (ASAT) in U/L,

variable 13 is defined by the value of patient's serum or plasma activity of alanine aminotransferase (ALAT) in U/L,

variable 14 is defined by the value of the patient's blood, plasma or serum concentration of glucose in mg/dl,

variable 15 is defined by the value of the patient' plasma or serum potassium in mmol/L,

variable 16 is defined by the value of the patient's plasma or serum concentration of N-terminal pro B-type natriuretic peptide in pg/mL,

variable 17 is defined by the value of the patient's plasma or serum concentration of lipoprotein associated phospholipase A2 in U/L,

variable 18 is defined by the value of the patient's plasma or serum concentration of C-reactive protein in mg/L,

variable 19 is defined by the value of the patient's plasma or serum concentration of troponin T in ng/mL,

variable 20 is defined by the value of the patient's plasma or serum concentration of homoarginine in micromol/L,

variable 21 is defined by the value of the patient's plasma or serum concentration of osteoprotegerin in pmol/L,

variable 22 is defined by the value of the patient's plasma or serum concentration of fetuin in ng/mL,

variable 23 is defined by the value of the patient's plasma or serum concentration of vitamin D in ng/ml,

variable 24 is defined by the value of the patient's plasma or serum concentration of transthyretin in micromol/L,

variable 25 is defined by the value of the patient's plasma or serum concentration of aldosterone in pg/mL,

variable 26 is defined by the value of the patient's plasma or serum concentration of cortisol in microgram/dL,

variable 27 is defined by the value of the patient's plasma or serum concentration of copeptin (carboxy-terminal-pro arginin vasopressin) in pmol/L,

variable 28 is defined by the value of the patient's plasma or serum concentration of soluble suppression of Tumorigenicity 2 (sST2) in ng/mL.

variable 29 is defined by the value of the patient's plasma or serum concentration of carbamylated albumin in percent,

wherein all numeric values are calculated per standard deviation, preferably wherein the numeric values are defined with a deviation of 50 %, preferably of 20 %, more preferably of 10 %, and most preferably of 5 %, and

wherein the coefficients of the risk equation are defined as

$x1 = 0,047702681;$
$x2 = -0,385079479;$
$x3 = -0,170679763;$
$x4 = -0,19424517;$
$x5 = 0,003364687;$
$x6 = 0,038836794;$
$x7 = -0,000144754;$
$x8 = -0,106157347;$
$x9 = 0,286153578;$
$x10 = -0,012747406;$
$x11 = 0,085284785;$
$x12 = -0,001889941;$
$x13 = 2,72969E-05;$
$x14 = -2,22659E-08;$
$x15 = -0,004498075;$
$x16 = 1,69671E-06;$
$x17 = -0,099378831;$
$x18 = 0,006740262;$
$x19 = -0,096168983;$
$x20 = 0,003300182;$
$x21 = -1,37846E-05;$
$x22 = 0,021377942;$
$x23 = -0,000652653;$
$x24 = 0,008605366;$
$x25 = -2,35966E-05;$
$x26 = -0,965745662;$
$x27 = 0,089391895;$

x28 = 2,73536E-06;
x29 = 3,81425E-11;
x30 = -0,005449139;
x31 = 6,77085E-06;
x32 = -1,35998E-09;
x33 = 0,013156362;
x34 = -0,000156612;
x35 = 1,073159193;
x36 = 0,080792566;
x37 = 0,018852403;
x38 = -0,003042769;
x39 = -0,068736703;
x40 = 0,003368983;
x41 = -0,011378031;
x42 = -0,073762152;
x43 = 0,00204079;
x44 = -1,29568E-05;
x45 = -0,272897733;
x46 = 0,019087226;
x47 = 0,00048282;
x48 = 0,039697765;
x49 = -0,000875904;
x50 = 0,000956853;
x51 = 0,010142565;
x52 = 0,101680363.

In yet another preferred embodiment, the invention provides a method for determining the risk of mortality in dialysis patients, wherein the correlation of a subset of biological parameters provides a risk score indicative for the patient's risk of mortality within the next year, wherein the biological parameters are selected from the group consisting of

the patient's age,
the patient's gender,
the patient's body mass index,
the patient's use of a statin,
at least one indicator of oxygen transport in the blood,
at least one indicator of calcium-phosphate homeostasis,
at least one indicator of electrolyte homoestasis,
at least one indicator of bone metabolism,
at least one indicator of immune function,
at least one indicator of skeletal muscle metabolism,
at least one indicator of lipid metabolism,
at least one indicator of carbohydrate metabolism,
at least one indicator of vascular inflammation,
at least one indicator of myocardial performance,
at least one indicator of myocardial integrity,
at least one indicator of sudden cardiac death,
at least one indicator of renal function,
at least one indicator of hepatic function,

wherein
the at least one indicator of lipid or lipoprotein metabolism is defined by the patient's blood concentration of LDL cholesterol,
the at least one indicator of oxygen transport is defined by the patient's blood concentration of hemoglobin,
the at least one indicator of carbohydrate metabolism is defined by the patient's blood concentration of glycated haemoglobin or blood, serum or plasma glucose,
the at least one indicator of calcium-phosphate homeostasis is defined by the patient's blood concentration of phosphate,
the at least one indicator of bone metabolism is defined by the patient's blood concentration of alkaline phosphatase and/or by the patient's blood concentration of vitamin D,

the at least one indicator of skeletal muscle metabolism is defined by the patient's blood concentration or activity of creatine kinase,

the at least one indicator of immune function is defined by the patient's blood concentration of white blood cells and/or soluble suppression of tumorigenicity 2 (sST2),

the at least one indicator of hepatic function is defined by the patient's blood concentrations or activities of alanine aminotransferase (ALAT) and/or aspartate aminotransferase (ASAT) and/or fetuin and/or transthyretin,

the at least one indicator of electrolyte homeostasis is defined by the patient's blood concentration of potassium,

the at least one indicator of myocardial function is defined by the patient's blood concentration of a natriuretic peptide,

the at least one indicator of vascular inflammation is defined by the patient's blood concentration or activity of lipoprotein associated phospholipase A2,

the at least one indicator of sudden cardiac death is defined by the patient's blood concentration of homoarginine,

the at least one indicator of myocardial integrity is defined by the patient's blood concentration of troponin T or troponin I,

the at least one indicator of renal function is defined by the patient's blood concentration of carbamylated albumin, wherein the method provides a weighted risk score $Y_5$ indicative for the patient's risk of mortality within the next year, and wherein the biological parameters are set into correlation by using the following formula, thereby providing the weighted risk score $Y_5$:

$$
\begin{aligned}
Y_5 =\ & x1 * \text{variable 1} + x2 * \text{variable 2} + x3 * \text{variable 3} + x4 * \text{variable 4} + x5 * \text{variable 4} * \\
& \text{variable 4} + x6 * \text{variable 5} + x7 * \text{variable 5} * \text{variable 5} + x8 * \text{variable 6} + x9 * \text{variable 7} \\
& + x10 * \text{variable 7} * \text{variable 7} + x11 * \text{variable 8} + x12 * \text{variable 9} + x13 * \text{variable 9} * \\
& \text{variable 9} + x14 * \text{variable 9} * \text{variable 9} * \text{variable 9} + x15 * \text{variable 10} + x16 * \text{variable} \\
& 10 * \text{variable 10} + x17 * \text{variable 11} + x18 * \text{variable 11} * \text{variable 11} + x19 * \text{variable 12} + \\
& x20 * \text{variable 12} * \text{variable 12} + x21 * \text{variable 12} * \text{variable 12} * \text{variable 12} + x22 * \\
& \text{variable 13} + x23 * \text{variable 13} * \text{variable 13} + x24 * \text{variable 14} + x25 * \text{variable 14} * \\
& \text{variable 14} + x26 * \text{variable 15} + x27 * \text{variable 15} * \text{variable 15} + x28 * \text{variable 16} + x29 \\
& * \text{variable 16} * \text{variable 16} + x30 * \text{variable 17} + x31 \text{ variable 17} * \text{variable 17} + x32 * \\
& \text{variable 17} * \text{variable 17} * \text{variable 17} + x33 * \text{variable 18} + x34 * \text{variable 18} * \text{variable 18} \\
& + x35 * \text{variable 19} + x36 * \text{variable 19} * \text{variable 19} \quad + x37 * \text{variable 20} + x38 * \\
& \text{variable 20} * \text{variable 20} + x39 * \text{variable 21} + x40 * \text{variable 22} + x41 * \text{variable 22} * \\
& \text{variable 22} + x42 * \text{variable 22} * \text{variable 22} * \text{variable 22} + x43 * \text{variable 23} + x44 * \\
& \text{variable 23} * \text{variable 23} + x45 * \text{variable 24} + x46 * \text{variable 25},
\end{aligned}
$$

wherein

variable 1 is defined by the patient's age in years,

variable 2 is defined as equal to 1 if the patient's gender is female and as equal to 0 if the patient's gender is male,

variable 3 is defined as equal to 2 if the patient uses a statin and as equal to 1 if the patient does not use a statin,

variable 4 as the patient's body mass index in $kg/m^2$

variable 5 is defined by the value of the patient's serum or plasma concentration of low density lipoprotein (LDL) cholesterol in mg/dl,

variable 6 is defined by the value of the patient's blood concentration of haemoglobin in g/dl,

variable 7 is defined by the value of the patient's blood serum or plasma concentration of glycated haemoglobin in percent,

variable 8 is defined by the value of the patient's blood serum or plasma concentration of phosphate in mg/dl,

variable 9 is defined by the value of the patient's serum or plasma activity of alkaline phosphatase in U/L,

variable 10 is defined by the value of the patient's serum or plasma activity of creatine kinase in U/L,

variable 11 is defined by the value of the patient's blood concentration of white blood cells (leucocytes) in $1000/\mu\text{L}$,

variable 12 is defined by the value of patient's serum or plasma activity of aspartate aminotransferase (ASAT) in U/L,

variable 13 is defined by the value of patient's serum or plasma activity of alanine aminotransferase (ALAT) in U/L,

variable 14 is defined by the value of the patient's blood, plasma or serum concentration of glucose in mg/dl,

variable 15 is defined by the value of the patient' plasma or serum potassium in mmol/L,

variable 16 is defined by the value of the patient's plasma or serum concentration of N-terminal pro B-type natriuretic peptide in pg/mL,

variable 17 is defined by the value of the patient's plasma or serum concentration of lipoprotein associated phospholipase A2 in U/L,

variable 18 is defined by the value of the patient's plasma or serum concentration of C-reactive protein in mg/L,

variable 19 is defined by the value of the patient's plasma or serum concentration of troponin T in ng/mL,

variable 20 is defined by the value of the patient's plasma or serum concentration of homoarginine in micromol/L,

variable 21 is defined by the value of the patient's plasma or serum concentration of fetuin in ng/mL,

variable 22 is defined by the value of the patient's plasma or serum concentration of vitamin D in ng/ml,

variable 23 is defined by the value of the patient's plasma or serum concentration of transthyretin in micromol/L,

variable 24 is defined by the value of the patient's plasma or serum concentration of soluble suppression of Tumorigenicity 2 (sST2) in ng/mL,

variable 25 is defined by the value of the patient's plasma or serum concentration of carbamylated albumin in percent,

wherein all numeric values are calculated per standard deviation, preferably wherein the numeric values are defined with a deviation of 50 %, preferably of 20 %, more preferably of 10 %, and most preferably of 5 %, and

wherein the coefficients of the risk equation are defined as

$x1 = 0{,}053386018;$
$x2 = -0{,}507359962;$
$x3 = -0{,}159672995;$
$x4 = -0{,}077278203;$
$x5 = 0{,}001675282;$
$x6 = 0{,}035553017;$
$x7 = -0{,}000122901;$
$x8 = -0{,}139558759;$
$x9 = 0{,}63289541;$
$x10 = -0{,}040388869;$
$x11 = 0{,}161839595;$
$x12 = -0{,}002408882;$
$x13 = 4{,}3439\text{E-}05;$
$x14 = -3{,}73529\text{E-}08;$

x15 = -0,009348889;
x16 = 1,00102E-05;
x17 = 0,259087904;
x18 = -0,010106474;
x19 = -0,090758507;
x20 = 0,002770596;
x21 =-3,26291E-06;
x22 = 0,026311865;
x23 = -0,00170523;
x24 = 0,057829917;
x25 = -0,000190269;
x26 = -0,278450291;
x27 = 0,050511166;
x28 = 5,86621E-05;
x29 =-1,08488E-09;
x30 = -0,041792948;
x31 = 5,7281E-05;
x32 = -2,18132E-08;
x33 = -0,033933381;
x34 = 0,000184845;
x35 = 1,971444416;
x36 = -0,401763801;
x37 = 3,417807829;
x38 = -1,60701075;
x39 = -0,048572426;
x40 = -0,001714864;
x41 = 0,000628046;
x42 = 1,71234E-07;
x43 = -0,422328754;
x44 = -0,002316355;
x45 =0,016454073.
x46 =1,597285182.

The invention also describes a method for determining the risk of mortality in dialysis patients, wherein the correlation of a subset of biological parameters provides a risk score indicative for the patient's risk of mortality within the next year, wherein the biological parameters are selected from the group consisting of

the patient's age,
the patient's gender,
the patient's body mass index,
the patient's use of a statin,
at least one indicator of oxygen transport in the blood,
at least one indicator of calcium-phosphate homeostasis,
at least one indicator of electrolyte homoestasis,
at least one indicator of bone metabolism,
at least one indicator of immune function,
at least one indicator of skeletal muscle metabolism,
at least one indicator of lipid metabolism,
at least one indicator of carbohydrate metabolism,
at least one indicator of vascular inflammation,
at least one indicator of myocardial performance,
at least one indicator of myocardial integrity,
at least one indicator of sudden cardiac death,
at least one indicator of hepatic function,

wherein
the at least one indicator of lipid or lipoprotein metabolism is defined by the patient's blood concentration of LDL cholesterol,

the at least one indicator of oxygen transport is defined by the patient's blood concentration of hemoglobin,

the at least one indicator of carbohydrate metabolism is defined by the patient's blood concentration of glycated haemoglobin or blood, serum or plasma glucose,

the at least one indicator of calcium-phosphate homeostasis is defined by the patient's blood concentration of phosphate

the at least one indicator of bone metabolism is defined by the patient's blood concentration of alkaline phosphatase and/or by the patient's blood concentration of vitamin D

the at least one indicator of skeletal muscle metabolism is defined by the patient's blood concentration or activity of creatine kinase

the at least one indicator of immune function is defined by the patient's blood concentration of white blood cells

the at least one indicator of hepatic function is defined by the patient's blood concentrations or activities of alanine aminotransferase (ALAT) and/or aspartate aminotransferase (ASAT) and/or fetuin and/or transthyretin

the at least one indicator of electrolyte homeostasis is defined by the patient's blood concentration of potassium,

the at least one indicator of myocardial function is defined by the patient's blood concentration of a natriuretic peptide,

the at least one indicator of vascular inflammation is defined by the patient's blood concentration or activity of lipoprotein associated phospholipase A2,

the at least one indicator of sudden cardiac death is defined by the patient's blood concentration of homoarginine

the at least one indicator of myocardial integrity is defined by the patient's blood concentration of troponin T or troponin I.

The method describes a weighted risk score $Y_7$ indicative for the patient's risk of mortality within the next year, and wherein the biological parameters are set into correlation by using the following formula, thereby providing the weighted risk score $Y_7$:

$$Y_7 = x1 * \text{variable } 1 + x2 * \text{variable } 2 + x3 * \text{variable } 3 + x4 * \text{variable } 4 + x5 * \text{variable } 4 * \text{variable } 4 + x6 * \text{variable } 5 + x7 * \text{variable } 5 * \text{variable } 5 + x8 * \text{variable } 6 + x9 * \text{variable } 7 + x10 * \text{variable } 7 * \text{variable } 7 + x11 * \text{variable } 8 + x12 * \text{variable } 9 + x13 * \text{variable } 9 * \text{variable } 9 + x14 * \text{variable } 9 * \text{variable } 9 * \text{variable } 9 + x15 * \text{variable } 10 + x16 * \text{variable } 10 * \text{variable } 10 + x17 * \text{variable } 11 + x18 * \text{variable } 11 * \text{variable } 11 + x19 * \text{variable } 12 + x20 * \text{variable } 12 * \text{variable } 12 + x21 * \text{variable } 12 * \text{variable } 12 * \text{variable } 12 + x22 * \text{variable } 13 + x23 * \text{variable } 13 * \text{variable } 13 + x24 * \text{variable } 14 + x25 * \text{variable } 14 * \text{variable } 14 + x26 * \text{variable } 15 + x27 * \text{variable } 15 * \text{variable } 15 + x28 * \text{variable } 16 + x29 * \text{variable } 16 * \text{variable } 16 + x30 * \text{variable } 17 + x31 \text{ variable } 17 * \text{variable } 17 + x32 * \text{variable } 17 * \text{variable } 17 * \text{variable } 17 + x33 * \text{variable } 18 + x34 * \text{variable } 18 * \text{variable } 18 + x35 * \text{variable } 19 + x36 * \text{variable } 19 * \text{variable } 19 + x37 * \text{variable } 20 + x38 * \text{variable } 20 * \text{variable } 20 + x39 * \text{variable } 21 + x40 * \text{variable } 22 + x41 * \text{variable } 22 * \text{variable } 22 + x42 * \text{variable } 22 * \text{variable } 22 * \text{variable } 22 + x43 * \text{variable } 23 + x44 * \text{variable } 23 * \text{variable } 23,$$

wherein

variable 1 is defined by the patient's age in years,

variable 2 is defined as equal to 1 if the patient's gender is female and as equal to 0 if the patient's gender is male,

variable 3 is defined as equal to 2 if the patient uses a statin and as equal to 1 if the patient does not use a statin

variable 4 as the patient's body mass index in kg/m$^2$

variable 5 is defined by the value of the patient's serum or plasma concentration of low density lipoprotein (LDL) cholesterol in mg/dl,

variable 6 is defined by the value of the patient's blood concentration of haemoglobin in g/dl,

variable 7 is defined by the value of the patient's blood serum or plasma concentration of glycated haemoglobin in percent,

variable 8 is defined by the value of the patient's blood serum or plasma concentration of phosphate mg/dL

variable 9 is defined by the value of the patient's serum or plasma activity of alkaline phosphatase in U/L,

variable 10 is defined by the value of the patient's serum or plasma activity of creatine kinase in U/L,

variable 11 is defined by the value of the patient's blood concentration of white blood cells (leucocytes) in 1000/$\mu$L,

variable 12 is defined by the value of patient's serum or plasma activity of aspartate aminotransferase (ASAT) in U/L,

variable 13 is defined by the value of patient's serum or plasma activity of alanine aminotransferase (ALAT) in U/L,

variable 14 is defined by the value of the patient's blood, plasma or serum concentration of glucose in mg/dl,

variable 15 is defined by the value of the patient' plasma or serum potassium in mmol/L,

variable 16 is defined by the value of the patient's plasma or serum concentration of N-terminal pro B-type natriuretic peptide in pg/mL

variable 17 is defined by the value of the patient's plasma or serum concentration of lipoprotein associated phospholipase A2 in U/L,

variable 18 is defined by the value of the patient's plasma or serum concentration of C-reactive protein in mg/L,

variable 19 is defined by the value of the patient's plasma or serum concentration of troponin T in ng/mL,

variable 20 is defined by the value of the patient's plasma or serum concentration of homoarginine in micromol/L,

variable 21 is defined by the value of the patient's plasma or serum concentration of fetuin in ng/mL,

variable 22 is defined by the value of the patient's plasma or serum concentration of vitamin D in ng/ml,

variable 23 is defined by the value of the patient's plasma or serum concentration of transthyretin in micromol/L,

wherein all numeric values are calculated per standard deviation, preferably wherein the numeric values are defined with a deviation of 50 %, preferably of 20 %, more preferably of 10 %, and most preferably of 5 %,

wherein the coefficients of the risk equation are defined as

$x1 = 0{,}059816768;$
$x2 = -0{,}632527694;$
$x3 = -0{,}133931049;$
$x4 = -0{,}139784069;$
$x5 = 0{,}002552879;$
$x6 = 0{,}027086755;$
$x7 = -8{,}74329E\text{-}05;$
$x8 = -0{,}1647803;$
$x9 = 0{,}542220121;$
$x10 = -0{,}032822594;$
$x11 = 0{,}229002018;$
$x12 = -0{,}006658052;$
$x13 = 5{,}58519E\text{-}05;$
$x14 = -4{,}5851E\text{-}08;$

x15 = -0,008655943;
x16 = 8,96476E-06;
x17 = 0,154419666;
x18 = -0,002701144;
x19 = -0,203651576;
x20 = 0,009147857;
x21 = -0,000105614;
x22 = 0,021640305;
x23 = -0,001657076;
x24 = 0,063288523;
x25 = -0,000207161;
x26 = -0,457789633;
x27 = 0,054176218;
x28 = 7,17406E-05;
x29 = -1,16875E-09;
x30 = -0,034845209;
x31 = 4,73402E-05;
x32 = -1,7833E-08;
x33 = -0,027169189;
x34 = 0,000137061;
x35 = 1,370859193;
x36 = -0,249497496;
x37 = 3,264453064;
x38 = -1,478672641;
x39 = -0,050488015;
x40 = -0,026982388;
x41 = 0,001430112;
x42 = -8,79861E-06;
x43 = -0,30275296
x44 = -0,012001712.

The invention also describes a method for determining the risk of mortality in dialysis patients, wherein the correlation of a subset of biological parameters provides a risk score indicative for the patient's risk of mortality within the next five years, wherein the biological parameters are selected from the group consisting of

the patient's age,
the patient's gender,
the patient's body mass index,
the patient's use of a statin,
at least one indicator of oxygen transport in the blood,
at least one indicator of calcium-phosphate homeostasis,
at least one indicator of electrolyte homoestasis,
at least one indicator of bone metabolism,
at least one indicator of immune function,
at least one indicator of skeletal muscle metabolism,
at least one indicator of lipid metabolism,
at least one indicator of carbohydrate metabolism,
at least one indicator of vascular inflammation,
at least one indicator of myocardial performance,
at least one indicator of myocardial integrity,
at least one indicator of sudden cardiac death,
at least one indicator of hepatic function.

wherein
the at least one indicator of lipid or lipoprotein metabolism is defined by the patient's blood concentration of LDL cholesterol,
the at least one indicator of oxygen transport is defined by the patient's blood concentration of hemoglobin,
the at least one indicator of carbohydrate metabolism is defined by the patient's blood concentration of glycated haemoglobin or blood, serum or plasma glucose,
the at least one indicator of calcium-phosphate homeostasis is defined by the patient's blood concentration of phosphate
the at least one indicator of bone metabolism is defined by the patient's blood concentration of alkaline phosphatase and/or by the patient's blood concentration of vitamin D
the at least one indicator of skeletal muscle metabolism is defined by the patient's blood concentration or activity of creatine kinase
the at least one indicator of immune function is defined by the patient's blood concentration of white blood cells
the at least one indicator of hepatic function is defined by the patient's blood concentrations or activities of alanine aminotransferase (ALAT) and/or aspartate aminotransferase (ASAT) and/or fetuin and/or transthyretin
the at least one indicator of electrolyte homeostasis is defined by the patient's blood concentration of potassium,
the at least one indicator of myocardial function is defined by the patient's blood concentration of a natriuretic peptide,
the at least one indicator of vascular inflammation is defined by the patient's blood concentration or activity of lipoprotein associated phospholipase A2,
the at least one indicator of sudden cardiac death is defined by the patient's blood concentration of homoarginine
the at least one indicator of myocardial integrity is defined by the patient's blood concentration of troponin T or troponin I.

Further, the methods describe a weighted risk score $Y_8$ indicative for the patient's risk of mortality within the next five years, and wherein the biological parameters are set into correlation by using the following formula, thereby providing the weighted risk score $Y_8$:

$$
\begin{aligned}
Y_8 = {}& x1 * \text{variable 1} + x2 * \text{variable 2} + x3 * \text{variable 3} + x4 * \text{variable 4} + x5 * \text{variable 4} * \\
& \text{variable 4} + x6 * \text{variable 5} + x7 * \text{variable 5} * \text{variable 5} + x8 * \text{variable 6} + x9 * \text{variable 7} \\
& + x10 * \text{variable 7} * \text{variable 7} + x11 * \text{variable 8} + x12 * \text{variable 9} + x13 * \text{variable 9} * \\
& \text{variable 9} + x14 * \text{variable 9} * \text{variable 9} * \text{variable 9} + x15 * \text{variable 10} + x16 * \text{variable} \\
& 10 * \text{variable 10} + x17 * \text{variable 11} + x18 * \text{variable 11} * \text{variable 11} + x19 * \text{variable 12} + \\
& x20 * \text{variable 12} * \text{variable 12} + x21 * \text{variable 12} * \text{variable 12} * \text{variable 12} + x22 * \\
& \text{variable 13} + x23 * \text{variable 13} * \text{variable 13} + x24 * \text{variable 14} + x25 * \text{variable 14} * \\
& \text{variable 14} + x26 * \text{variable 15} + x27 * \text{variable 15} * \text{variable 15} + x28 * \text{variable 16} + x29 \\
& * \text{variable 16} * \text{variable 16} + x30 * \text{variable 17} + x31 \text{ variable 17} * \text{variable 17} + x32 * \\
& \text{variable 17} * \text{variable 17} * \text{variable 17} + x33 * \text{variable 18} + x34 * \text{variable 18} * \text{variable 18} \\
& + x35 * \text{variable 19} + x36 * \text{variable 19} * \text{variable 19} + x37 * \text{variable 20} + x38 * \\
& \text{variable 20} * \text{variable 20} + x39 * \text{variable 21} + x40 * \text{variable 22} + x41 * \text{variable 22} * \\
& \text{variable 22} + x42 * \text{variable 22} * \text{variable 22} * \text{variable 22} + x43 * \text{variable 23} + x44 * \\
& \text{variable 23} * \text{variable 23},
\end{aligned}
$$

wherein

variable 1 is defined by the patient's age in years,

variable 2 is defined as equal to 1 if the patient's gender is female and as equal to 0 if the patient's gender is male,

variable 3 is defined as equal to 2 if the patient uses a statin and as equal to 1 if the patient does not use a statin,

variable 4 as the patient's body mass index in kg/m$^2$

variable 5 is defined by the value of the patient's serum or plasma concentration of low density lipoprotein (LDL) cholesterol in mg/dl,

variable 6 is defined by the value of the patient's blood concentration of haemoglobin in g/dl,

variable 7 is defined by the value of the patient's blood serum or plasma concentration of glycated haemoglobin in percent (%),

variable 8 is defined by the value of the patient's blood serum or plasma concentration of phosphate in mg/dl,

variable 9 is defined by the value of the patient's serum or plasma activity of alkaline phosphatase in U/L,

variable 10 is defined by the value of the patient's serum or plasma activity of creatine kinase in U/L,

variable 11 is defined by the value of the patient's blood concentration of white blood cells (leucocytes) in 1000/ μL,

variable 12 is defined by the value of patient's serum or plasma activity of aspartate aminotransferase (ASAT) in U/L,

variable 13 is defined by the value of patient's serum or plasma activity of alanine aminotransferase (ALAT) in U/L,

variable 14 is defined by the value of the patient's blood, plasma or serum concentration of glucose in mg/dl,

variable 15 is defined by the value of the patient' plasma or serum potassium in mmol/L,

variable 16 is defined by the value of the patient's plasma or serum concentration of N-terminal pro B-type natriuretic peptide in pg/mL,

variable 17 is defined by the value of the patient's plasma or serum concentration of lipoprotein associated phospholipase A2 in U/L,

variable 18 is defined by the value of the patient's plasma or serum concentration of C-reactive protein in mg/L,

variable 19 is defined by the value of the patient's plasma or serum concentration of troponin T in ng/mL,

variable 20 is defined by the value of the patient's plasma or serum concentration of homoarginine in micromol/L,

variable 21 is defined by the value of the patient's plasma or serum concentration of fetuin in ng/mL,

variable 22 is defined by the value of the patient's plasma or serum concentration of vitamin D in ng/ml,

variable 23 is defined by the value of the patient's plasma or serum concentration of transthyretin in micromol/L,

wherein all numeric values are calculated per standard deviation, preferably wherein the numeric values are defined with a deviation of 50 %, preferably of 20 %, more preferably of 10 %, and most preferably of 5 %, and

wherein the coefficients of the risk equation are defined as

$x1$= 0,050270753;
$x2$= -0,378570298;
$x3$= -0,135151028;
$x4$= -0,210697601;
$x5$= 0,003507553;
$x6$= 0,038348252;
$x7$= -0,000141365;
$x8$= -0,108313119;
$x9$= 0,317793404;
$x10$= -0,014460052;
$x11$=0,103944446;
$x12$= -0,002678584;
$x13$= 2,94663E-05;
$x14$= -2,38424E-08;
$x15$= -0,004871771;
$x16$= 2,12393E-06;

x17= -0,11460716;
x18= 0,008205668;
x19= -0,12075659;
x20= 0,004877597;
x21= -4,04063E-05;
x22= 0,022114372;
x23= -0,000646295;
x24= 0,00857338;
x25= -2,28866E-05;
x26= -0,761087083;
x27= 0,072310134;
x28= 7,40384E-06;
x26= 1,84863E-11;
x30= -0,004237787;
x31= 4,82593E-06;
x32= -5,32771E-10;
x33= 0,017343072;
x34= -0,000185105;
x35= 1,387790954;
x36= -0,128579487;
x37= -0,09201455;
x38= 0,026258136;
x39= -0,011000311
x40= -0,088242445;
x41= 0,002549627;
x42= -1,84227E-05;
x43= -0,242739688;
x44= 0,015768939.

The patient's risk of all-cause mortality is provided by setting the following subset of biological parameters into correlation: age, gender, alkaline phosphatase, creatine kinase, homoarginine, carbamylated albumin, transthyretin, fetuin, aldosterone, troponin T, natriuretic peptide, and c-reactive protein (CRP).

The patient's risk of all-cause mortality is provided by setting the following subset of biological parameters into correlation: age, gender, alkaline phosphatase, creatine kinase, homoarginine, carbamylated albumin, transthyretin, troponin T, NT-proBNP, c-reactive protein (CRP), galectin 3, and sST2.

The patient's risk of all-cause mortality is provided by setting the following subset of biological parameters into correlation: age, gender, body mass index, LDL cholesterol, hemoglobin, glycated haemoglobin 1c, phosphate, alkaline phosphatase, creatin kinase concentration or activity, white blood cells, alanine aminotransferase (ALAT), aspartate aminotransferase (ASAT), serum, plasma or blood glucose, potassium, natriuretic peptide, preferably NT-pro-BNP, lipoprotein associated phospholipase activity or concentration, c-reactive protein (CRP), troponin T or tropinin I, homoarginine, osteoprotegerin, fetuin, vitamin D, transthyretin, aldosterone, cortisol, soluble Suppression of Tumorigenicity 2 (sST2), copeptin (carboxy-terminales-pro arginin casopressin), and carbamylated albumin.

TThe patient's risk of all-cause mortality is provided by setting the following subset of biological parameters into correlation: age, gender, body mass index, LDL cholesterol, hemoglobin, glycated haemoglobin 1c, phosphate, alkaline phosphatase, creatin kinase concentration or activity, white blood cells, alanine aminotransferase (ALAT), aspartate aminotransferase (ASAT), serum, plasma or blood glucose, potassium, natriuretic peptide, preferably NT-pro-BNP, lipoprotein associated phospholipase activity or concentration, c-reactive protein, troponin T or tropinin I, homoarginine, fetuin, vitamin D, transthyretin, Soluble Suppression of Tumorigenicity 2 (sST2), and carbamylated albumin.

Equally preferred is that the patient's risk of all-cause mortality is provided by setting the following subset of biological parameters into correlation: age, gender, body mass index, LDL cholesterol, hemoglobin, glycated haemoglobin 1c, phosphate, alkaline phosphatase, creatin kinase concentration or activity, white blood cells, alanine aminotransferase (ALAT), aspartate aminotransferase (ASAT), serum, plasma or blood glucose, potassium, natriuretic peptide, preferably NT-pro-BNP, lipoprotein associated phospholipase activity or concentration, c-reactive protein, troponin T or tropinin I, homoarginine, fetuin, vitamin D, and transthyretin.

With respect to all of the preferred embodiments described above, it is to be understood that the patient's age is to be defined by the log value of the patient's age in years, that the gender is to be defined as equal to 1 if the patient's gender is female and as equal to 0 if the patient's gender is male, that the body mass index is to be defined by the log value of the patient's body mass index in $kg/m^2$. It is further to be understood that any of the other listed biological parameters are to be defined by the log value of either their concentration or activity in the patient's blood or plasma serum. The concentration and/or the activity of all these biological parameter can be determined by standard methods which are laboratory routine practice and known to the person skilled in the art.

According to a preferred embodiment, the risk score is indicative for the patient's mortality due to cardiovascular events or due to combined fatal and non-fatal cardiovascular events, preferably wherein the risk score is indicative for cardiac mortality, cardiovascular mortality, sudden heart death, death due to acute myocardial infarction, death due to cardiac insufficiency, death due to stroke, death due to bypass operation, death due to coronary angioplasty, death due to cancer, death due to infection, death to non-fatal myocardial infarction, death to hospitalization due to any cause, death to necessity of coronary angioplasty, death due to necessity of coronary bypass surgery, death due to other interventions to treat coronary heart disease, transitory cerebral ischemic attacks, prolonged reversible ischemic neurologic deficits, or any composites of these endpoints.

Preferably, the patient's risk of cardiovascular mortality is caused by a disease of the arterial tree. Preferably, the cardiovascular disease is due to atherosclerosis and/or to thrombosis.

The risk score of the present invention may be indicative for all-cause mortality within the next year, the next 3 years, preferably within the next 5 years, more preferably within the next 10 years.

Even more preferably, the risk score is indicative for cardiovascular mortality within the next year, the next 3 years, preferably within the next 5 years, more preferably within the next 10 years.

It is further envisaged that both the simplified risk scores and the weighted risk scores have excellent discriminatory power for prediction and determining the risk of all-cause mortality, preferably the risk of cardiovascular mortality.

The risk score determined by the methods of the present invention allows an appropriate medical treatment of the patient to reduce the patient's risk of all-cause mortality, preferably of cardiovascular mortality. This medical treatment is generally in form of administering drugs.

According to the present disclosure the medical treatment of a patient includes the administration of a therapeutically effective dose or pharmaceutically active compound in form of a pharmaceutical composition, e.g. a drug, which prevents, ameliorates or treats the symptoms accompanying a disease or condition referred to in this specification. Therapeutic efficacy and toxicity of the compound can be determined by standard pharmaceutical procedures in cell cultures or experimental animals, e.g., ED50 (the dose therapeutically effective in 50 % of the population) and LD50 (the dose lethal to 50 % of the population). The dose ratio between therapeutic and toxic effects is the therapeutic index, and it can be expressed as the ratio, LD50/ED50.

The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment.

The term "drug" generally means any composition formulated in solid, liquid (or gaseous) form which can be administered to a patient. Said composition generally comprises a therapeutically active compound optionally together with suitable auxiliary compounds such as diluents or carriers or further ingredients. In this context, it is distinguished between auxiliary compounds, i.e. compounds which do not contribute to the effects elicited by the compound upon application of the composition for its desired purpose, and further ingredients, i.e. compounds which contribute a further effect or modulate the effect of the therapeutic effective ingredient. Suitable diluents and/or carriers depend on the purpose for which the composition is to be used and the other ingredients. The person skilled in the art can determine such suitable diluents and/or carriers without further ado. Examples of suitable carriers and/or diluents are disclosed elsewhere herein. Drugs suitable for oral administration may be presented as discrete units such as capsules, cachets, tablets, or lozenges. Other compositions include suspensions in aqueous liquors or non-aqueous liquids such as an syrup, an elixir, or an emulsion.

As used herein, an effective amount of the administered drug is a dosage large enough to produce the desired therapeutic effect to reduce the risk of all-cause mortality and/or cardiovascular mortality. An effective amount is not, however, a dosage so large as to cause adverse side effects. Generally, an effective amount may vary with the patient's age, condition, weight and sex, as well as the extent of the condition being treated, and can be determined by one of skill in the art. The dosage may be adjusted by the individual practitioner in the event of any complication.

Preferably, in the context of the present invention, the medical treatment to reduce the patient's risk score is in form of administering drugs, preferably wherein the drugs are for treating hyperlipidaemia, regulating blood pressure, modifying the heart rate, and/or for providing glycaemic control, and/or for inhibiting blood coagulation in diabetic patients.

The term "hyperlipidaemia" (also referred to as "hyperlipoproteinemia") generally means a disease which involves abnormally elevated levels of any or all lipids and/or lipoproteins in the blood. Hyperlipidaemias are divided in primary and secondary subtypes. Primary hyperlipidaemia is usually due to genetic causes (such as a mutation in a receptor protein), while secondary hyperlipidaemia arises due to other underlying causes such as diabetes. Lipid and lipoprotein abnormalities are common in the general population, and are regarded as a modifiable risk factor for cardiovascular disease due to their influence on atherosclerosis.

Preferably, treating hyperlipidaemia according to the present disclosure means a treatment which results in a reduction of the patient's blood lipids and/or lipoproteins, preferably in reducing the concentration of cholesterol.

The term "glycaemic control" generally refers to any medical treatment that aims at controlling the levels of blood glucose (blood sugar) in a person with diabetes mellitus. Blood glucose levels can be measured by means of a glucose meter, with the result either indicated in mg/dL (milligrams per deciliter in the USA) or mmol/L (millimoles per litre in Canada and Europe) of blood. The average glucose level of a healthy person is around 4.5 to 7.0 mmol/L (80 to 125 mg/dL).

More preferably, the medical treatment to reduce the patient's risk of mortality is in form of changing modalities of hemodialysis, preferably by extending the time of dialysis, and/or by improving the patient's nutritional status, preferably by increasing protein intake.

Advantageously, the present invention provides reliable methods for determining the risk of mortality in patients receiving hemodialysis. The identification of high risk patients allows for a closer monitoring of this group so that preventive treatments can be administered to those patients with the greatest need. This suggests specific preventive measures of medial treatment: patients with a determined intermediate (or moderate) or high risk of mortality should receive therapies that aim, for example, at reducing the concentration of cholesterol in the blood, at reducing the patient's blood glucose concentration, or at reducing the patient's blood pressure. Thus, the present invention contributes to the development of individualized treatment regimens.

In a preferred embodiment of the disclosure the medical treatment is in form of administering drugs, wherein the drugs are for reducing the patient's blood concentration of cholesterol, for reducing the patient's blood pressure, and/or for reducing the patient's blood glucose concentration.

"Medical treatment" according to the present disclosure is to be understood as any treatment of secondary prevention which improves the patient's life span and/or health. The medical treatment of the disclosure is preferably applied to patients for whom a high risk of mortality has been determined by the methods described herein. This risk of mortality can be a risk of all-cause mortality or, preferably, a risk of cardiovascular mortality.

Accordingly, the risk score determined by the methods of the present invention is indicative for a medical treatment of patients with a high risk of mortality.

Patients identified with a high risk of mortality participate in particular from this indication since this group of patients has so far, in most cases, not been subjected to optimal medical treatments for reducing all-cause mortality or, preferably, mortality due to cardiovascular events. Hence, the methods of the present invention are particularly useful for dialysis patients which are categorized with a high risk of mortality.

The medical treatment and/or the medical treatment of secondary prevention is applied more intensively in patients with a high risk of mortality than in patients with a moderate or low risk of mortality. Consequently, the medical treatment and/or the treatment of secondary prevention may be the more intense, the higher the patient's risk of mortality has been determined by the methods of the present invention.

The present disclosure describes a computer program product directly loadable into the internal memory of a digital computer, comprising software code portions for implementing the calculation of the mortality risk score according to the present invention when said product is run on a computer.

A computer program product is provided for performing one of the previously explained methods, when the product is run on a computer. The computer program product is preferred to be directly loadable into the internal memory of a digital computer and comprises software code portions for implementing the calculation of the risk score.

The computer program product may be a computer program preferably stored on a machine readable storage medium like RAM, ROM, or on a removable CD-ROM, flash memory, DVD or USB-stick. The computer program may be provided on a server to be downloaded via for example a data network such as the internet or another transfer system such as a phone line or a wireless transfer connection. Additionally, or alternatively, the computer program product may be a network of computer implemented computer programs such as a client/server system or a cloud computing system, an embedded system with a computer program or an electronic device like a smart phone or a personal computer on which a computer program is stored, loaded, running, exercised, or developed.

In one implementation, the information on the set of biological parameters obtained from the patient is entered into an input device of a computer, an embedded system, an electronic device or a smart phone via a keyboard, a touch screen or a voice interface. In another implementation, the information on the set of biological parameters is measured by adequate measuring devices and transmitted via the internet or another transfer system such as a phone line or a wireless transfer connection to a remote station such as a computer.

In one implementation, the information on the calculated risk score is provided on an output medium, stored in a memory means or transferred to a remote station. In one embodiment, the information on the risk score is displayed on a screen or display as output medium.

Preferably, the computer program product comprises software code portions for implementing the calculation of any of the simplified risk score and/or any of the weighted risk scores as defined in the context of the present invention.

The computer program product comprises software code portions for implementing the calculation of the weighted risk scores as defined in the context of the present invention.

It is to be understood that the definitions and explanations of the methods, measurements, and terms made above apply mutatis mutandis for all aspects described in this specification in the following except as otherwise indicated.

The following Figures and Examples are intended to illustrate various embodiments of the present invention. As such, the specific modifications discussed therein are not to be understood as limitations of the scope of the invention. It will be apparent to the person skilled in the art that various equivalents, and modifications may be made without departing from the invention, and it is thus to be understood that such equivalent embodiments are to be included herein.

## FIGURES

**Figure 1: HRs for 1 year mortality according to specific biomarkers.** HRs for the biomarkers indicate the increase in risk per increment SD, adjusted for variables of the basic model 3, which included age, sex, BMI, PVD, phosphate, alkaline phosphatase and creatin-kinase.

**Figure 2: HRs for 5 years mortality according to specific biomarkers.** HRs for the biomarkers indicate the increase in risk per increment SD, adjusted for variables of the basic model 3, which included age, sex, CAD, CHF, PVD, Hb, HbA1c, alkaline phosphatase and creatin kinase.

**Figure 3: Kaplan-Meier estimates of mortality.** Kaplan-Meier survival curves for A) 1 year mortality and B) 5 years mortality by quartiles of the combination of predictors from the full model (model 5).

**Figure 4: Calibration models.** Calibration of the prediction models for a) 1 year mortality and b) 5 years mortality. Observed (blue columns) and predicted (red columns) mortality risk according to risk strata derived from the final prediction model.

## EXAMPLES

**Background:** Predicting mortality in dialysis patients is important for clinical decision making. Predictors of short- and longer term mortality may differ substantially due to time-differentiating effects of many risk factors. Importantly, the incremental value of multiple biomarkers from different disease pathways in risk prediction in dialysis patients is unknown.

**Methods:** Data from 1255 hemodialysis patients (mean age 66 years, 54% male), who participated in the German Diabetes and Dialysis study (4D study) and were followed prospectively for a median of 4 years, were analyzed. The data set was divided into sets for model development (training set, n = 734) and validation (n = 521) using random selection. Prediction models for 1-year and 5-year mortality were established, respectively. First, the predictive value of patient history was evaluated, and then investigated whether the addition of routine laboratory markers, ECG data and selective biomarkers further improved risk stratification for mortality. Prognostic indices of the models were assessed, and discriminative abilities were calculated with Harrells C-statistics and integrated discrimination improvement (IDI).

**Results:** In total, 347 patients of the 734 patients in the training set died during follow-up of 5 years, 87 patients within the first year. In Cox proportional hazards analyses, age, peripheral vascular disease, the absence of an AV-fistula and a low BMI were strong and independent predictors of short-term death within 1 year of observation (model 1, C-statistic 0.66). Routine laboratory markers such as phosphate, alkaline phosphatase and creatin-kinase and ECG data improved the C-statistic to 0.72. Among the non-routine biomarkers, homoarginine, transthyretin, fetuin, aldosterone and carbamylated albumin had the strongest predictive value and further increased the C statistic to 0.83. Similarly, the prediction of 5-year mortality risk was significantly improved by the addition of biomarkers including Troponin T, NT-pro-BNP, tranythyretin, CRP and carbamylated albumin in the final model. The final models could discriminate accurately among patients with low, intermediate and high mortality risks.

**Conclusion:** In patients undergoing hemodialysis, the addition of multiple biomarkers substantially improved risk stratification for death beyond the models including patient history and routine clinical parameters alone. Future studies should assess the potential of these novel markers for clinical application.

Despite advances in renal replacement therapy, mortality of dialysis patients remains excessive. It is highest in patients on dialysis as reflected by a five year survival of only 35. Efforts have been undertaken to decrease risk and many interventions were designed to improve survival and CV outcomes in dialysis patients. Few of these interventions have been effective in the overall patient groups. However, partial effects in subgroups and depending on baseline risks have been suggested, pointing to the need and importance of patients risk stratification.

In the general population, risk stratification is mainly done by the use of classic, Framingham-type risk factors such as hypertension, diabetes mellitus, hypercholesterolemia, family history of cardiovascular disease and smoking. These predictors allow for a good stratification of individuals regarding their risk of cardiovascular disease and death. Risk prediction in dialysis patients, however, may not be based on the classic risk factors alone, as different and uremia-specific risk factors seem to play a more important role.

Separate instruments have therefore been developed to predict outcome in dialysis patients and support clinical decision making. Importantly, most studies did not address the time-differentiating effects of risk markers. Especially in dialysis patients, short-term risk may be predicted from parameters largely different from those indicating an increased risk in the longer term. According to registry reports, one and five year survival rates are commonly used and would be appropriate to make risk assessment comparable and widely applicable.

Importantly, in recent years a variety of biomarkers have emerged that reflect different pathophysiologies and may serve as indicators of risk in dialysis patients. Novel inflammatory and cardiac biomarkers, markers of endothelial dysfunction, protein-energy wasting, bone and mineral disorders, volume status and fibrosis have been found associated with mortality in dialysis patients. However, few studies have assessed and compared their additional value to a defined set of easily obtainable parameters of patient history and routine laboratory markers. Furthermore, the utility of combining multiple biomarkers for risk prediction in dialysis patients remains largely unclear.

The aim of our study was to assess the incremental value of biomarkers and their combination in the prediction of mortality in dialysis patients. We developed one-year and five-year prediction models using clinical and routine laboratory information and evaluated whether the simultaneous use of multiple biomarkers would contribute substantially to risk stratification.

## METHODS

**Study Population.** This present study was conducted within a cohort of 1255 patients with type 2 diabetes mellitus undergoing hemodialysis. They participated in the 4D study, which was a prospective randomized controlled trial (RCT) investigating the effect of cholesterol lowering treatment on adverse cardiovascular events. Between March 1998 and October 2002, the patients were recruited in 178 dialysis centres in Germany, aged 18 - 80 years and on hemodialysis for less than 2 years. After randomization at baseline, patients were followed until the date of death, censoring, or end of the study in March 2004. The study complies with the Declaration of Helsinki, was approved by the medical ethical committee, and all patients gave their written informed consent before inclusion.

## Covariates

**Clinical definitions and laboratory measurements.** Baseline demographic data were collected through patient interviews. Smoking status was classified as never, former or current. Comorbidities, including the presence of coronary artery disease (CAD) and CHF, as well as the duration of diabetes mellitus and dialysis treatment were reported by the patients' nephrologists. CAD was defined by the history of MI, coronary artery bypass grafting surgery; percutaneous coronary intervention; and the presence of coronary heart disease, as documented by coronary angiography. Clinical data were obtained using standardized protocols. Blood pressure was measured in sitting position. Body mass index (BMI) was calculated as weight (kg) divided by height (m) squared.

Laboratory investigations in the initial trial were performed centrally at the Department of Clinical Chemistry, University of Freiburg, Germany. All measurements were performed in blood samples taken at baseline and stored without repeated freeze thaw cycles at -80°C. The blood samples were taken before the start of dialysis sessions and administration of drugs. Biomarkers were mainly measured by commercially available assay systems or antibodies. Further details on the tests used are provided in supplementary material online.

**Candidate predictors.** The list of candidate predictors considered for the prediction model included the following: (i) demographic factors (age, sex), (ii) clinical characteristics including cardiovascular risk factors (smoking status, duration of diabetes, systolic blood pressure, diastolic blood pressure, blood pressure amplitude), comorbidities (coronary artery disease, congestive heart failure, peripheral vascular disease) and dialysis specific factors (dialysis vintage, ultrafiltration volume, type of vascular access, erythropoietin dose). Furthermore, candidate predictors were considered from (iii) laboratory data (total-, HDL- and LDL-cholesterol, triglycerides, hemoglobin, albumin, calcium, phosphate, alkaline phosphatase, creatin kinase, creatinine, leukocyte count, platelet count, bilirubin, ALT, AST, glucose, HbA1c, potassium, iron, ferritin, transferrin) and (iv) 28 biomarkers (adiponectin, beta-crosslaps, NT-pro-BNP, free triiodothyronine, antibodies to platelet factor 4-heparin complex, osteocalcin, lipoprotein-associated phospholipase A2, PTH, CRP, Troponin T, ADMA, SDMA, homoarginine, osteoprotegerin, fetuin, 25(OH)VitaminD, galectin 3, copeptin, aldosterone, cortisol, Tyroxine, TSH, retinol, retinol-binding protein, transthyretin, alpha-tocopherol, carbamylated albumin, testosterone).

**Outcome assessment.** For the present analysis, all-cause mortality was chosen as the main outcome measure. To account for time-differentiating effects, short and longer term mortality were investigated. Short term mortality represented all-cause mortality within 1 year after study enrolment. Longer term mortality represented all-cause mortality during 5 years follow-up after study start. No patient was lost to follow-up and the endpoints were centrally adjudicated by a specialized committee.

**Statistical analysis.** Descriptive analyses were used to summarize baseline characteristics of the study participants. Continuous variables are expressed as mean $\pm$ SD or median and interquartile range as appropriate. Categorical variables are presented as proportions. The data set was split into a training data set (60% of the original cohort, n = 734) and a validation data set (40% of the original cohort, n = 521). Continuous variables underwent log transformation before analysis. Associations of the candidate predictors were assessed by Spearman correlation coefficients. In case of close correlation only one predictor of a cluster was used for further analysis.

The associations between baseline covariates and 1-year all-cause mortality were assessed using Cox proportional hazards models. Covariates were selected for the analyses according to their biologically plausible potential and avoiding collinearity. Using a forward-selection process, we developed multivariable models in which all covariates with a P value of 0.1 or less in univariate analyses for all-cause death were entered in the model selection process. We first developed a model for patient history (model 1) and secondly, added routine laboratory data (model 2). Finally, the prognostic abilities of all biomarkers were investigated through multivariable proportional hazards regression adjusting for all covariates from the basic model (model 2). Hazard ratios per one unit of the biomarker and for one standard deviation (SD) increase in log-transformed biomarker concentration along with the 95% confidence intervals (CIs) are provided. Those biomarkers with a P value of 0.1 or less in multivariate modelling were added in the further model selection process to achieve the final model 3. All analyses were repeated for 5 year all-cause mortality accordingly.

The performance of the models was assessed using the Harrell global C-statistic. Similar to the area under the receiver operator curve, it describes the probability that the respective model will assign the higher mortality risk to the patient who died compared to the patient who remained alive or was censored. Models resulting from the training data set were applied to the validation data set using fixed hazard ratios. That means, variables and their exact ß coefficients as developed for the prediction model in the training data set were applied to the validation data set. For model calibration, mortality risk was split into quartiles and observed versus predicted risk was investigated for all groups. All analyses were performed using SPSS version 20.0 and STATA version 12.

### Results

**Characteristics of the study participants.** The baseline characteristics, including demographic and clinical characteristics and laboratory data for study participants in the training and validation data sets are provided in Table 1. The mean age of patients was 66 $\pm$ 8 years and about half of the patients were male. Approximately one third of patients had coronary artery disease, one third had congestive heart failure and almost half of the patients had peripheral vascular disease. The duration of diabetes was 18 $\pm$ 9 years and dialysis vintage 8 months. The biomarker concentrations of the study participants in both data sets are provided in Table 1b.

**Predictors for 1-year mortality.** Age and sex were first evaluated separately (model 1 of Table 2) and in subsequent steps forced into the models. Analyses of patient history and clinical characteristics revealed an univariate positive association with mortality for higher age and duration of diabetes, lower BMI, and the presence of coronary artery disease, congestive heart failure and peripheral vascular disease as well as the type of dialysis access (other than an arteriovenous fistula); p<0.1, respectively. These parameters were entered into the forward-selection process, resulting in a multivariate model that finally included BMI, peripheral vascular disease and dialysis access (model 2 of Table 2). The laboratory parameters serum albumin, phosphate, alkaline phosphatase, creatin-kinase, iron, bilirubin and leukocyte count showed an univariate association to all-cause mortality during 1 year follow-up. In a second step, when these were added to the forward-selection modelling, phosphate, alkaline phosphatase and creatin-kinase remained independent predictors of mortality (model 3 of Table 2). Addition of ECG variables revealed a significant impact of heart rhythm (model 4).

The strongest univariate associations for biomarkers with 1-year mortality were observed for transthyretin, fetuin, homoarginine, retinol, free triiodothyronine, troponin T, aldosterone, NT-pro-BNP and carbamylated albumin. These parameters showed significant (p<0.1) associations with outcome after adjustment for the clinical and routine laboratory parameters from model 3. Adding those to the forward selection process resulted in a final model 5 including age, sex, BMI, alkaline phosphatase, creatin-kinase, homoarginine, fetuin, carbamylated albumin, aldosterone and transthyretin (model 5 of Table 2). In several occasions of daily activities, the patient may not accurately remember comorbidities and thereby increasing the measurement error and/or clinical data may not be readily available. For such situations a possibility of risk assessment based on one blood draw may be useful and we investigated a separate extra model based on laboratory data alone. This model (model 6) provided a similar predictive ability as the main model 5.

**Predictors for 5-year mortality.** When demographic and clinical patient characteristics were included in the multivariate model, older age, lower BMI, and the presence of coronary artery disease, congestive heart failure and peripheral vascular disease independently predicted all-cause mortality within 5 years follow-up after a forward selection process (model 2 of Table 3). Secondly, the routine laboratory parameters were added and hemoglobin, HbA1c, alkaline phosphatase and creatin-kinase also were independently associated with poor outcome (model 3). Addition of ECG variables revealed a significant impact of heart rhythm and heart rate (model 4).

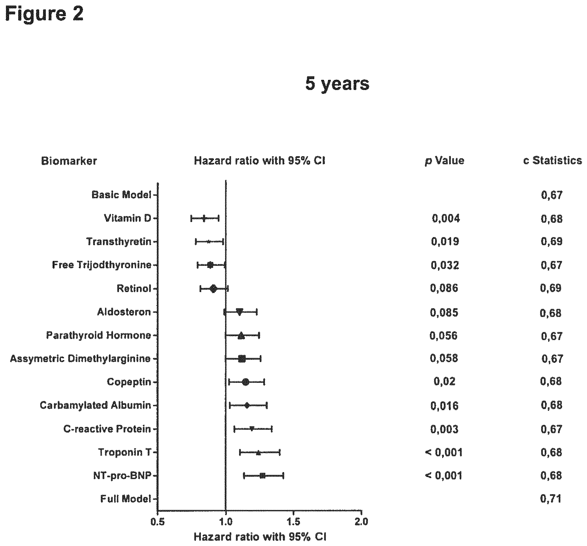

Biomarkers that were independently associated with mortality after adjustment for the variables from the basic model 3 are shown in Figure 2. Finally, these were entered into the forward selection process leading to model 5. The final model 5 consisted of age, sex, PVD, alkaline phosphatase, creatin-kinase, troponin T, transthyretin and carbamylated albumin to independently predict 5 years mortality (Table 3).

Again, we investigated a separate extra model based on laboratory data alone (model 6), which is provided in Table 3.

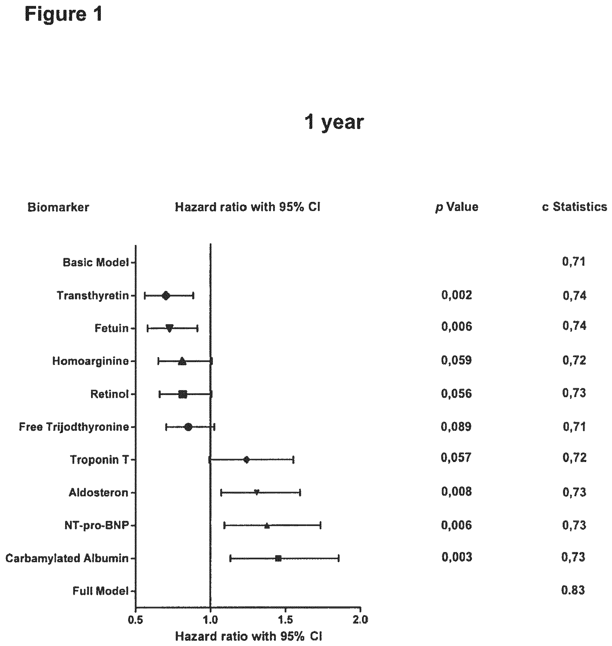

**Model performances.** In order to test the accuracy of the prediction models, we computed the C-index based on 1 year and 5 years event-free survival, respectively. Regarding 1 year survival, the model with basic demographic and clinical characteristics achieved a considerable accuracy as indicated by a C-index of 0.66. By adding routine laboratory data (model 3), the C-index meaningfully increased to 0.71 and the best accuracy was reached by adding multiple biomarkers, resulting in a C-statistic of 0.83 (model 5). The information obtained through the individual biomarkers alone was assessed on top of the basic model 3 and is presented in Figure 1.

Regarding 5 years survival, the model with basic demographic and clinical characteristics achieved a C-index of 0.66, which was slightly increased to 0.68 by adding routine laboratory data (model 3). Again, the best accuracy was reached by adding multiple biomarkers, resulting in a C-statistic of 0.71 (model 5). The information obtained through the individual biomarkers alone was assessed on top of the basic model 2 and is presented in Figure 2.

The IDI for the respective models was also calculated. A positive and significant IDI ($P < 0.05$) for the final models was observed when we compared them to the models including patient history only or combinations of patient history, routine laboratory and ECG data.

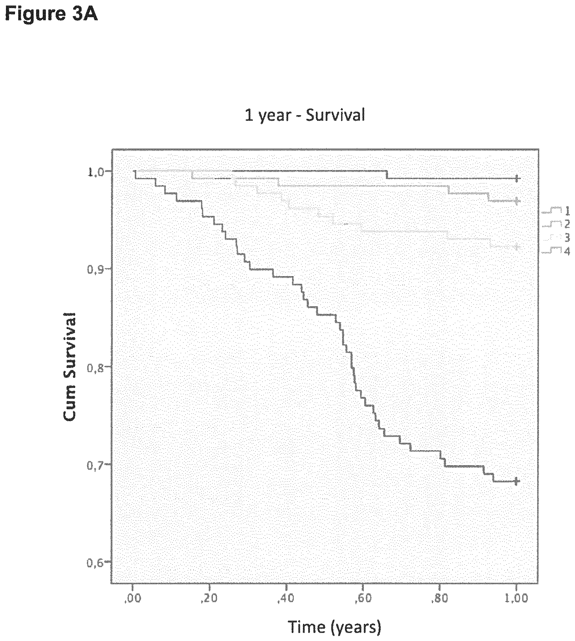

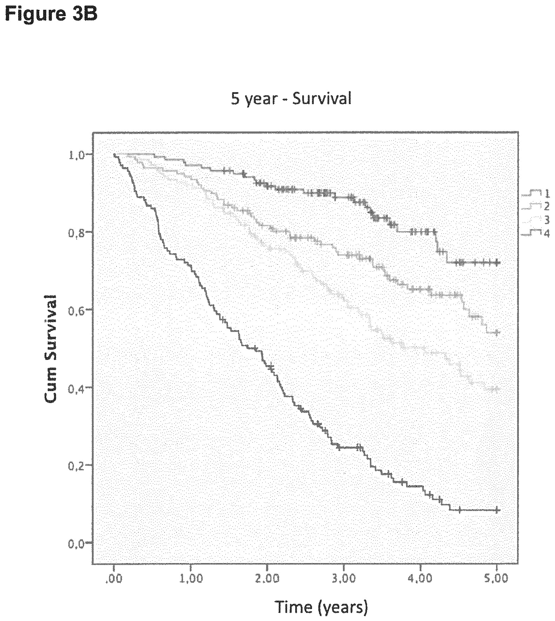

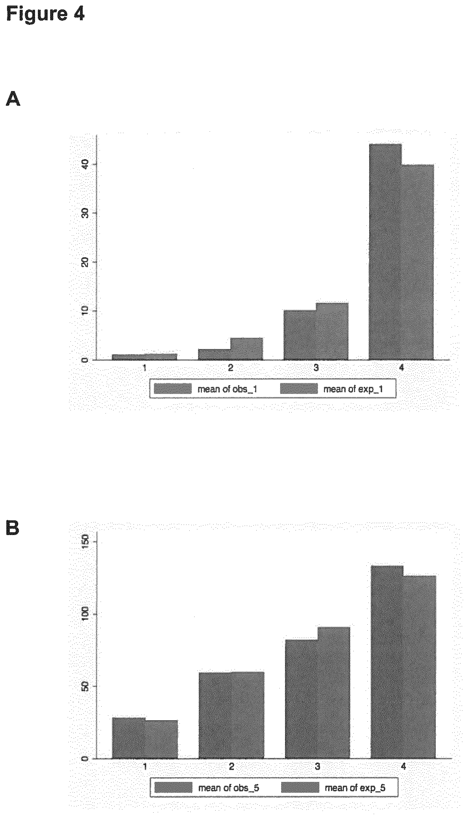

**Calibration and validation of the models.** Patients in the training data set were categorized into quartiles a) quartiles according to their 1 year mortality risk and b) quartiles according to their 5 years mortality risk. Kaplan-Meier curves for mortality by quartiles of the combination of predictors from the full model are shown in Figure 3. The final model including biomarkers could discriminate accurately among patients with very low, low, intermediate and high risk of death. Observed and predicted mortality was compared and no significant differences were found (Figure 4).

**Validation of the models.** The prediction models as developed in the training data set were investigated in the validation data set. Therefore, we applied the respective models with fixed hazard ratios to the validation data set. The final model for 1 year mortality reached a C-statistic of 0.75 in the validation set and the final model for 5 years mortality reached a C-statistic of 0.71 in the validation set.

## TABLES

**Table 1.** Patient characteristics, routine laboratory data and serum biomarker concentrations in 1255 patients undergoing maintenance hemodialysis treatment.

|  | Training Set (N=734) | Validation Set (N=521) |
|---|---|---|
| **Demographic and clinical characteristics** |  |  |
| Age (y) | 66 ± 8 | 65 ± 8 |
| Sex % female | 45 | 48 |
| Duration of diabetes (y) | 18 ± 9 | 18 ± 9 |
| Time on dialysis (m) | 8 ± 7 | 9 ± 7 |
| Body mass index | 27.6 ± 4.8 | 27.5 ± 4.8 |
| **Systolic blood pressure** | **146 ± 21** | **145 ± 23** |
| Diastolic blood pressure | 76 ± 11 | 76 ± 11 |
| Atorvastatin | 51 | 47 |
| Smoker/Ex-smoker | 41 | 40 |
| CAD | 30 | 28 |
| CHF | 35 | 37 |
| PVD | 44 | 46 |

(continued)

| Routine laboratory | | |
| --- | --- | --- |
| Total cholesterol (mg/dl) | 218 ± 42 | 220 ± 43 |
| LDL-cholesterol (mg/dl) | 126 ± 30 | 125 ± 30 |
| HDL-cholesterol (mg/dl) | 36 ± 13 | 37 ± 14 |
| Triglycerides (mg/dl) | 263 ± 161 | 266 ± 175 |
| Hemoglobin (g/dl) | 11.0 ± 1.4 | 10.8 ± 1.3 |
| HbA1c (%) | 6.7 ± 1,3 | 6.7 ± 1.3 |
| Albumin (g/dl) | 3.8 ± 0,3 | 3.8 ± 0.3 |
| Calcium (mmol/l) | 2.3 ± 0,2 | 2.3 ± 0.2 |
| Phosphate (mg/dl) | 6.0 ± 1,6 | 6.1 ± 1.6 |
| Ultrafiltration volume (kg) | 2.2 ± 1,2 | 2.3 ± 1.2 |
| Alkaline phosphatase (U/L) | 126 ± 64 | 124 ± 57 |
| Creatine-kinase (U/L) | 69 ± 62 | 69 ± 53 |
| Leukocytes (1000/μL) | 8.2 ± 2.5 | 8.0 ± 2.4 |
| AST (U/L) | 14 ± 6 | 14 ± 14 |
| ALT (U/L) | 16 ± 9 | 17 ± 13 |
| Total bilirubin (mg/dL) | 0.4 ± 0.2 | 0.4 ± 0.2 |
| Platelets (1000/μL) | 259 ± 82 | 254 ± 79 |
| Glucose (mg/dL) | 158 ± 64 | 155 ± 65 |
| Potassium (mmol/L) | 5.2 ± 0.8 | 5.2 ± 0.9 |
| Ferritin (μg/l) | 493 ± 419 | 477 ± 436 |
| Iron (μmol/l) | 10.4 ± 4.6 | 10.3 ± 4.6 |
| | **Training Set (N=734)** | **Validation Set (N=521)** |
| **Biomarker** | | |
| Adiponectin (mg/L) | 16 ± 10 | 17 ± 10 |
| Beta-CrossLaps (ng/mL) | 1.2 ± 0,7 | 1.2 ± 0,6 |
| NT-pro-BNP (pg/mL) | 7841 ± 14107 | 8554 ± 12781 |
| Copeptin (pmol/L) | 103 ± 362 | 94 ± 58 |
| Free triiodothyronine (pmol/L) | 3.8 ± 0.9 | 3.8 ± 1.1 |
| Heparin complex antibodies (%) | 0.3 ± 0.3 | 0,3 ± 0.2 |
| Osteocalcin (μg/L) | 106 ± 104 | 109 ± 98 |
| Lp-PLA2 (U/L) | 545 ± 144 | 541 ± 156 |
| Parathyroid hormone (pg/mL) | 103 ± 133 | 101 ± 97 |
| CRP (mg/L) | 11.1 ± 17.7 | 10.8 ± 21.0 |
| Troponin T (ng/mL) | 0.1 ± 0.1 | 0.1 ± 0.1 |
| ADMA (μmol/L) | 0.9 ± 0.2 | 0.9 ± 0.2 |
| Homoarginine (μmol/L) | 1.2 ± 0.5 | 1.1 ± 0.5 |
| SDMA (μmol/L) | 2.5 ± 0.8 | 2.6 ± 0.8 |
| Osteoprotegerin (pmol/L) | 11.1 ± 4.6 | 11.7 ± 4.9 |
| Fetuin (ng/mL) | 35 ± 13 | 34 ± 11 |
| 25(OH)Vitamin D (μg/L) | 18 ± 10 | 18 ± 10 |
| Carbamylated albumin (%) | 0.6 ± 0.3 | 0.6 ± 0.3 |
| Thyroxine (pmol/L) | 15 ± 4 | 15 ± 3 |
| Thyroid stimulating hormone (TSH, mIU/L) | 1.2 ± 1.5 | 1.2 ± 1.0 |
| Retinol (μmol/L) | 3.3 ± 1.0 | 3.3 ± 1.0 |
| Transthyretin (μmol/L) | 3.7 ± 1.5 | 3.7 ± 1.6 |
| Retinol-binding protein 4 (μmol/L) | 4.0 ± 1.4 | 4.0 ± 1.4 |
| a-Tocopherol (μmol/L) | 24 ± 9 | 25 ± 10 |
| Galectin 3 (ng/uL) | 54 ± 20 | 54 ± 19 |

(continued)

| Biomarker | Training Set (N=734) | Validation Set (N=521) |
|---|---|---|
| Aldosterone (pg/mL) | 103 ± 232 | 88 ± 166 |
| Cortisol (mg/dL) | 17 ± 6 | 18 ± 6 |
| Testosterone (µg/l) | 3.3 ± 2.6 | 3.3 ± 2.7 |

**Table 2.** Prediction of mortality within 1 year follow-up

| Predictors | Model 1 age and sex | | Model 2 patient history | | Model 3 plus routine lab | | Model 4 plus ECG | | Model 5 plus biomarkers | | Model 6 Laboratory only | |
|---|---|---|---|---|---|---|---|---|---|---|---|---|
| Age | 1.48 (1.15-1.91) | 0.003 | 1.26 (0.96-1.64) | 0.09 | 1.42 (1.08-1.85) | 0.011 | 1.35 (1.03-1.78) | 0.032 | 1.66 (1.17-2.35) | 0.005 | 1.81 (1.26-2.60) | 0.001 |
| female sex | 0.75 (0.48-1.16) | 0.20 | 0.77 (0.48-1.24) | 0.29 | 0.74 (0.47-1.17) | 0.20 | 0.73 (0.45-1.18) | 0.20 | 0.61 (0.34-1.10) | 0.10 | 0.54 (0.30-0.96) | 0.034 |
| BMI | | | 0.75 (0.59-0.96) | 0.02 | 0.77 (0.60-0.97) | 0.028 | 0.74 (0.58-0.94) | 0.013 | 0.73 (0.53-1.00) | 0.05 | | |
| CAD | | | | | | | | | | | | |
| CHF | | | | | | | | | | | | |
| PVD | | | 2.01 (1.27-3.18) | 0.003 | 1.70 (1.09-2.63) | 0.019 | 1.53 (0.98-2.40) | 0.063 | | | | |
| AV-fistula | | | 0.59 (0.35-1.00) | 0.05 | | | | | | | | |
| Phosphate | | | | | 1.47 (1.20-1.81) | <0.001 | 1.59 (1.28-1.96) | <0.001 | | | | |
| Alkaline Phosphatase | | | | | 1.48 (1.23-1.78) | <0.001 | 1.43 (1.18-1.72) | <0.001 | 1.56 (1.26-1.93) | <0.001 | 1.62 (1.30-2.03) | <0.001 |
| Creatinkinase | | | | | 0.74 (0.59-0.94) | 0.013 | 0.74 (0.57-0.95) | 0.019 | 0.73 (0.53-0.99) | 0.045 | 0.70 (0.51-0.94) | 0.019 |
| Sinus rhythm | | | | | | | 0.47 (0.26-0.86) | 0.014 | | | | |
| Heart rate | | | | | | | | | | | | |
| Homoarginine | | | | | | | | | 0.75 (0.57-0.99) | 0.04 | 0.71 (0.55-0.93) | 0.014 |
| carbamylated Albumin | | | | | | | | | 1.41 (1.05-1.90) | 0.023 | 1.45 (1.08-1.94) | 0.014 |
| Transthyretin | | | | | | | | | 0.67 (0.52-0.88) | 0.004 | 0.68 (0.52-0.90) | 0.006 |
| Fetuin | | | | | | | | | 0.72 (0.57-0.92) | 0.007 | 0.73 (0.57-0.93) | 0.010 |
| Aldosterone | | | | | | | | | 1.37 (1.07-1.74) | 0.011 | 1.36 (1.06-1.75) | 0.015 |
| C-statistic | 0.62 | | 0.66 | | 0.71 | | 0.72 | | 0.83 | | 0.82 | |

**Table 3.** Prediction of mortality within 5 years follow-up

| Predictors | Model 1 age and sex | | Model 2 patient history | | Model 3 plus routine lab | | Model 4 plus ECG | | Model 5 plus biomarkers | | Model 6 Laboratory only | |
|---|---|---|---|---|---|---|---|---|---|---|---|---|
| Age | 1.45 (1.30-1.68) | <0.001 | 1.36 (1.19-1.55) | <0.001 | 1.40 (1.22-1.60) | <0.001 | 1.37 (1.19-1.57) | <0.001 | 1.54 (1.30-1.81) | <0.001 | 1.57 (1.34-1.84) | <0.001 |
| female sex | 0.87 (0.70-1.09) | 0.22 | 0.98 (0.78-1.22) | 0.85 | 0.81 (0.64-1.02) | 0.07 | 0.80 (0.63-1.01) | 0.058 | 0.85 (0.65-1.12) | 0.25 | | |
| BMI | | | 0.87 (0.77-0.97) | 0.015 | | | | | | | | |
| CAD | | | 1.39 (1.11-1.73) | 0.004 | 1.34 (1.07-1.68) | 0.01 | 1.33 (1.06-1.68) | 0.014 | | | | |
| CHF | | | 1.36 (1.09-1.70) | 0.007 | 1.31 (1.05-1.64) | 0.017 | 1.27 (1.01-1.60) | 0.046 | | | | |
| PVD | | | 1.79 (1.44-2.23) | <0.001 | 1.70 (1.36-2.12) | <0.001 | 1.67 (1.33-2.09) | <0.001 | 1.69 (1.30-2.20) | <0.001 | | |
| Hemoglobin | | | | | 0.88 (0.79-0.97) | 0.014 | 0.88 (0.79-0.98) | 0.02 | | | | |
| HbA1c | | | | | 1.19 (1.07-1.32) | 0.001 | 1.17 (1.05-1.30) | 0.005 | | | | |
| Alkaline Phosphatase | | | | | 1.22 (1.10-1.36) | <0.001 | 1.18 (1.05-1.32) | 0.005 | 1.29 (1.13-1.46) | <0.001 | 1.31 (1.15-1.49) | <0.001 |
| Creatinkinase | | | | | 0.77 (0.68-0.86) | <0.001 | 0.76 (0.68-0.88) | <0.001 | 0.71 (0.62-0.83) | <0.001 | 0.69 (0.60-0.79) | <0.001 |
| Sinus rhythm | | | | | | | 0.67 (0.47-0.94) | 0.021 | 0.62 (0.42-0.90) | 0.013 | | |
| Heart rate | | | | | | | 1.01 (1.00-1.02) | 0.008 | | | | |
| Troponin T | | | | | | | | | 1.35 (1.18-1.54) | <0.001 | 1.31 (1.13-1.52) | <0.001 |
| carbamylated Albumin | | | | | | | | | 1.34 (1.17-1.53) | <0.001 | 1.24 (1.09-1.41) | 0.001 |
| Transthyretin | | | | | | | | | 0.83 (0.73-0.93) | 0.002 | | |
| NT-pro-BNP | | | | | | | | | | | 1.13 (0.98-1.31) | 0.09 |
| CRP | | | | | | | | | | | 1.19 (1.05-1.36) | 0.007 |
| C-statistic | 0.60 | | 0.66 | | 0.68 | | 0.69 | | 0.71 | | 0.70 | |

**Conclusions.** We developed two prediction models for patients undergoing hemodialysis that predicted mortality within 1 year and 5 years, respectively. In addition to patient history and laboratory data that are routinely collected in clinical practice, we evaluated the prognostic value of 28 biomarkers. Our data indicate that specific biomarkers significantly contributed to risk prediction by providing incremental predictive ability over routinely collected data. The most informative biomarkers were homoarginine, transthyretin, fetuin, aldosterone and carbamylated albumin in the prediction of 1 year mortality. Concerning the prediction of 5 years mortality, troponin T, NT-pro-BNP, CRP, transthyretin and carbamylated albumin significantly added incremental predictive information.

In the general population, the use of multiple biomarkers has been suggested to improve risk stratification for cardiovascular death. In a Swedish cohort of elderly men, the simultaneous addition of several biomarkers of cardiovascular and renal abnormalities substantially improved the risk stratification beyond that of a model based on classical risk factors. The biomarkers included troponin T, NT-pro-BNP, cystatin C and CRP, which in their combination improved the C-statistic from 0.66 to 0.75. Another study in participants from the Framingham Heart Study, however, could not demonstrate a meaningful incremental value of multiple biomarkers. In the 3209 participants, 10 contemporary biomarkers added only moderately to the overall prediction of risk based on conventional cardiovascular risk factors.

Results from the general population however can not be applied to dialysis patients and a different approach is needed. The rate of death of dialysis patients is excessive, with an 18% first year mortality rate reported by the ERA-EDTA Registry. Importantly, time-differentiating effects have been noted with different etiologies underlying short-term as compared to longer term mortality risks in dialysis patients (refs). Previous research indicated that risk factors for short-term death may be largely different from risk factors affecting longer-term mortality in dialysis patients. In order to identify patients at highest versus lowest risk, we therefore developed prediction models taking time differentiating effects into account with a short-term approach of 1 year and a longer term model investigating predictors for 5 years survival. Such approach may enable for unique comparisons across databases in line with registry data usually providing 1 and 5 years survival data.

Interestingly, in our study the specific biomarkers significantly contributed to risk prediction and provided incremental predictive ability over routinely collected data. This is in line with the observation from previous research that - in contrast to the general population - traditional cardiovascular risk factors fail to explain the excess mortality in dialysis patients. Non-traditional risk-factors seem to play a more important role, but association studies provided so far limited additional information, and pathways are not fully understood. In our study, we found phosphate and alkaline phosphatase to be important predictors of short term mortality beyond clinical characteristics including age, BMI and patient history in dialysis patients. This finding is supported by previous studies, suggesting phosphate and alkaline phosphatase as indicators of CKD-MBD which may lead to calcification and subsequent cardiovascular events as well as inflammation, trauma and subsequent non-cardiovascular events. We furthermore found creatin-kinase, fetuin and transthyretin to be of incremental predictive value. These markers may be representative of the protein energy wasting (PEW), being highly prevalent in dialysis patients. The PEW syndrome is characterized by depletion of body energy and protein stores, inflammation and the development of co-morbidities and is highly associated with mortality and cardiovascular disease (CVD) in dialysis patients. Fetuin, an acute phase protein and calcification inhibitor, was found negatively correlated with mortality in a study of 226 dialysis patients. Transthyretin (TTR), formerly referred to as pre-albumin, is known as a sensitive indicator of inflammation and malnutrition and has also been described as an adequate marker of body composition. Even within normal ranges of BMI and albumin, transthyretin was able to depict patients at risk in whom the wasting syndrome may not yet be clinically apparent.

Interestingly, another novel pathway of risk may be represented by homoarginine, an amino acid recently discovered to strongly affect adverse outcome in renal patients. Homoarginine is derived from lysine and suggested to increase the availability of nitric oxide (NO), a lack of which is associated with endothelial and myocardial dysfunction. Recent studies showed that low blood concentrations of homoarginine were associated with heart failure and markedly increased mortality risks in patients referred for coronary angiography and in patients undergoing hemodialysis. Finally, our results identifying aldosterone as an important predictor supports the notion of potential novel pathways being mediated by the hormone. Recent investigations have documented that - apart from the classical effect on sodium reabsorption - aldosterone exerts a variety of other effects, which are potentially important for renal and cardiovascular injury. Aldosterone increases oxidative stress through induction of NADPH oxidase and promotes vascular inflammation. In addition aldosterone impairs endothelial function by reducing the bioavailability of nitric oxide. Finally, high aldosterone concentrations in the presence of salt overload cause cardiac hypertrophy and fibrosis, which can contribute to the excess morbidity and mortality of dialysis patients. Clinical application and decision making is based on the availability of prognostic indices. As such, a simple tool may also be the subjective assessment of health status by the treating physician. Of note, the "surprise question" asked to the physician had a strong predictive information. When the treating nephrologist answered "no" to the question "Would I be surprised if this patient died within the next 6 months?" the respective patients had a 2.7-fold increased risk of death. Even more impressive, the single question of a diminished appetite revealed a strong likelihood to mortality - patients with diminished appetite had a 4.7 fold increased mortality risk as compared to those with normal or good appetite.

However, due to the subjective nature of the evaluation tools, comparison across studies and populations remains difficult. Our study therefore provides a valuable alternative tool to make risk assessment comparable and widely applicable. Using laboratory data and biomarkers that are uniformly assessable across centres with a high standardization may considerably contribute to an improved risk prediction for patients undergoing dialysis.

In conclusion, this study suggests that novel biomarkers including homoarginine, transthyretin, fetuin, aldosterone, carbamylated albumin, troponin T, NT-pro-BNP and CRP improve risk prediction in dialysis patients by providing incremental prognostic value over routinely available parameters. Improved risk prediction may thus help to identify patients likely to benefit from specific treatments and support clinical decision making. Future studies should assess the potential of these novel markers for clinical application.

**Claims**

1. A method for determining the risk of mortality in dialysis patients, comprising the steps of

   a) analysing a set of biological parameters obtained from the patient or from the patient's file history, wherein the biological parameters are selected from the group consisting of:

   the patient's age,
   the patient's gender,
   the patient's body mass index,
   the patient's use of statin,
   at least one indicator of oxygen transport in the blood,
   at least one indicator of calcium-phosphate homeostasis,
   at least one indicator of bone metabolism,
   at least one indicator of immune function,
   at least one indicator of skeletal muscle metabolism,
   at least one indicator of lipid metabolism,
   at least one indicator of carbohydrate metabolism,
   at least one indicator of hormonal blood pressure regulation,
   at least one indicator of vascular inflammation,
   at least one indicator of myocardial performance,
   at least one indicator of myocardial integrity,
   at least one indicator of sudden cardiac death,
   at least one indicator of renal function,
   at least one indicator of hepatic function,
   or a subset thereof;

   b) setting the biological parameters or a subset thereof into correlation, wherein the correlation of a subset of biological parameters provides a risk score indicative for the patient's mortality within the next year, wherein the biological parameters are selected from the group consisting of
   the patient's age,
   the patient's gender,
   the patient's body mass index,
   at least one indicator of skeletal muscle metabolism,
   at least one indicator of sudden cardiac death,
   at least one indicator of renal function,
   at least one indicator of hormonal blood pressure regulation,
   at least one indicator of bone metabolism,
   at least two indicators of hepatic function,
   wherein
   the at least one indicator of skeletal muscle metabolism is defined by the patient's blood concentration or activity of creatine kinase,
   the at least one indicator of sudden cardiac death is defined by the patient's blood concentration of homoarginine,
   the at least one indicator of renal function is defined by the patient's blood concentration of carbamylated albumin,
   the at least one indicator of hormonal blood pressure regulation is defined by the patient's blood concentration of aldosterone or copeptin (carboxy-terminal-pro arginin vasopressin),
   the at least one indicator of bone metabolism is defined by the patient's blood concentration or activity of alkaline phosphatase, and

the at least two indicators of hepatic function are defined by the patient's blood concentration of transthyretin and fetuin,

wherein the risk score is a weighted risk score $Y_1$ indicative for the patient's risk of mortality within the next year, and wherein the biological parameters are set into correlation by using the following formula, thereby providing the weighted risk score $Y_1$:

$$Y_1 = 0.51 * \text{variable } 1 + (-0.49 * \text{variable } 2) + (-0.32 * \text{variable } 3) + 0.44 * \text{variable } 4$$
$$+ (-0.32 * \text{variable } 5) + (-0.29 * \text{variable } 6) + (-0.33 * \text{variable } 7) + 0.35 * \text{variable } 8$$
$$+ (-0.4 * \text{variable } 9) + 0.31 * \text{variable } 10$$

wherein

variable 1 is defined by the log value of the patient's age in years,
variable 2 is defined as equal to 1 if the patient's gender is female and as equal to 0 if the patient's gender is male,
variable 3 is defined by the log value of the patient's body mass index in $kg/m^2$,
variable 4 is defined by the log value of the patient's blood activity of alkaline phosphatase in U/L,
variable 5 is defined by the log value of the patient's blood activity of creatine kinase in U/L,
variable 6 is defined by the log value of the patient's blood concentration of homoarginine in micromol/L,
variable 7 is defined by the log value of the patient's blood concentration of fetuin in ng/mL,
variable 8 is defined by the log value of the patient's blood concentration of carbamylated albumin in percent (%),
variable 9 is defined by the log value of the patient's blood concentration of transthyretin in micromol/L, and
variable 10 is defined by the log value of the patient's blood concentration of aldosterone in pg/mL,
wherein all log values are calculated to the basis e, and wherein all numeric values in the formula providing the weighted risk score $Y_1$ are calculated per standard deviation, preferably wherein the numeric values are defined with a deviation of 50 %, preferably of 20 %, more preferably of 10 %, and most preferably of 5 %, and wherein the risk score is indicative for a medical treatment to reduce the patient's risk of mortality.

2. The method of claim 1, wherein the correlation of a subset of biological parameters provides a risk score indicative for the patient's risk of mortality within the next five year, wherein the biological parameters are selected from the group consisting of
the patient's age,
the patient's gender,
at least one indicator of bone metabolism,
at least one indicator of skeletal muscle metabolism,
at least one indicator of myocardial performance,
at least one indicator of myocardial integrity,
at least one indicator of renal function, and
at least one indicator of inflammation,
wherein
the at least one indicator of bone metabolism is defined by the patient's blood concentration or activity of alkaline phosphatase,
the at least one indicator of skeletal muscle metabolism is defined by the patient's blood concentration or activity of creatine kinase,
the at least one indicator of myocardial performance is defined by the patient's blood concentration of NT-proBNP,
the at least one indicator of myocardial integrity is defined by the patient's blood concentration of troponin T or troponin I,
the at least one indicator of renal function is defined by the patient's blood concentration of carbamylated albumin,
the at least one indicator of inflammation is defined by the patient's blood concentration of c-reactive protein (CRP),
wherein the risk score is a weighted risk score $Y_2$ indicative for the patient's risk of mortality within the next five years, and wherein the biological parameters are set into correlation by using the following formula, thereby providing the weighted risk score $Y_2$:

$$Y_2 = 0.38 * \text{variable 1} + (-0.1 * \text{variable 2}) + 0.21 * \text{variable 3} + (-0.34 * \text{variable 4})$$
$$+ 0.18 * \text{variable 5} + 0.16 * \text{variable 6} + 0.26 * \text{variable 7} + 0.16 * \text{variable 8}$$

wherein

variable 1 is defined by the log value of the patient's age in years,
variable 2 is defined as equal to 1 if the patient's gender is female and as equal to 0 if the patient's gender is male,
variable 3 is defined by the log value of the patient's blood activity of alkaline phosphatase in U/L,
variable 4 is defined by the log value of the patient's blood activity of creatine kinase in U/L,
variable 5 is defined by the log value of the patient's blood concentration of NT-proBNP in pg/mL,
variable 6 is defined by the log value of the patient's blood concentration of c-reactive protein (CRP) in mg/L,
variable 7 is defined by the log value of the patient's blood concentration of troponin T in ng/mL, and
variable 8 is defined by the log value of the patient's blood concentration of carbamylated albumin in percent (%),
wherein all log values are calculated to the basis e, and wherein all numeric values in the formula providing the weighted risk score $Y_2$ are calculated per standard deviation, preferably wherein the numeric values are defined with a deviation of 50 %, preferably of 20 %, more preferably of 10 %, and most preferably of 5 %.

3. The method of claim 1, wherein the correlation of a subset of biological parameters provides a risk score indicative for the patient's risk of mortality within the next year, wherein the biological parameters are selected from the group consisting of

the patient's age,
the patient's gender,
the patient's body mass index,
the patient's use of a statin,
at least one indicator of oxygen transport in the blood,
at least one indicator of calcium-phosphate homeostasis,
at least one indicator of electrolyte homoestasis,
at least one indicator of bone metabolism,
at least one indicator of immune function,
at least one indicator of skeletal muscle metabolism,
at least one indicator of lipid metabolism,
at least one indicator of carbohydrate metabolism,
at least one indicator of hormone blood pressure regulation,
at least one indicator of vascular inflammation,
at least one indicator of myocardial performance,
at least one indicator of myocardial integrity,
at least one indicator of sudden cardiac death,
at least one indicator of renal function, and
at least one indicator of hepatic function,

wherein
the at least one indicator of lipid metabolism is defined by the patient's blood concentration of LDL cholesterol,
the at least one indicator of oxygen transport is defined by the patient's blood concentration of hemoglobin,
the at least one indicator of carbohydrate metabolism is defined by the patient's blood concentration of glycated haemoglobin or blood, serum or plasma glucose,
the at least one indicator of calcium-phosphate homeostasis is defined by the patient's blood concentration of phosphate,
the at least one indicator of bone metabolism is defined by the patient's blood concentration of alkaline phosphatase and/or by the patient's blood concentration of osteoprotegerin and/or by the patient's blood concentration of vitamin D,
the at least one indicator of skeletal muscle metabolism is defined by the patient's blood concentration or activity of creatine kinase,
the at least one indicator of immune function is defined by the patient's blood concentration of white blood cells and/or cortisol and/or soluble suppression of Tumorigenicity 2 (sST2),
the at least one indicator of hepatic function is defined by the patient's blood concentrations or activities of alanine aminotransferase (ALAT) and/or aspartate aminotransferase (ASAT) and/or fetuin and/or transthyretin,

the at least one indicator of electrolyte homeostasis is defined by the patient's blood concentration of potassium,
the at least one indicator of myocardial performance is defined by the patient's blood concentration of a natriuretic peptide,
the at least one indicator of vascular inflammation is defined by the patient's blood concentration or activity of lipoprotein associated phospholipase A2,
the at least one indicator of sudden cardiac death is defined by the patient's blood concentration of homoarginine,
the at least one indicator of myocardial integrity is defined by the patient's blood concentration of troponin T or troponin 1,
the at feast one indicator of hormonal blood pressure regulation is defined by the patient's blood concentration of aldosterone and or of copeptin,
the at least one indicator of renal function is defined by the patient's blood concentration of carbamylated albumin,
wherein the risk score is a weighted risk score $Y_3$ indicative for the patient's risk of mortality within the next year, and wherein the biological parameters are set into correlation by using the following formula, thereby providing the weighted risk score $Y_3$:

$$Y_3 = 0,057410212 * \text{variable } 1 - 0,543915506 * \text{variable } 2 - 0,143998352 * \text{variable } 3 - 0,102420371 * \text{variable } 4 + 0,002126855 * \text{variable } 4 * \text{variable } 4 + 0,028997043 * \text{variable } 5 - 0,000102574 * \text{variable } 5 * \text{variable } 5 - 0,144446014 * \text{variable } 6 + 0,500608722 * \text{variable } 7 - 0,032782705 * \text{variable } 7 * \text{variable } 7 + 0,141166863 * \text{variable } 8 - 0,004400439 * \text{variable } 9 + 4,87592E\text{-}05 * \text{variable } 9 * \text{variable } 9 + -4,08374E\text{-}08 * \text{variable } 9 * \text{variable } 9 * \text{variable } 9 - 0,007492671 * \text{variable } 10 + 8,65369E\text{-}06 * \text{variable } 10 * \text{variable } 10 + 0,23222716 * \text{variable } 11 - 0,006951655 * \text{variable } 11 * \text{variable } 11 - 0,073850191 * \text{variable } 12 + 0,001124082 * \text{variable } 12 * \text{variable } 12 + 2,88445E\text{-}05 * \text{variable } 12 * \text{variable } 12 * \text{variable } 12 + 0,020974682 * \text{variable } 13 - 0,001555684 * \text{variable } 13 * \text{variable } 13 + 0,064258442 * \text{variable } 14 - 0,000208666 * \text{variable } 14 * \text{variable } 14 - 0,439032703 * \text{variable } 15 + 0,064866606 * \text{variable } 15 * \text{variable } 15 + 5,67869E\text{-}05 * \text{variable } 16 - 1,15087E\text{-}09 * \text{variable } 16 * \text{variable } 16 - 0,043785154 * \text{variable } 17 + 6,01674E\text{-}05 \text{ variable } 17 * \text{variable } 17 - 2,2989E\text{-}08 * \text{variable } 17 * \text{variable } 17 * \text{variable } 17 - 0,035837307 * \text{variable } 18 + 0,000195508 * \text{variable } 18 * \text{variable } 18 + 1,747437912 * \text{variable } 19 - 0,30324001 * \text{variable } 19 * \text{variable } 19 + 3,652306243 * \text{variable } 20 - 1,722577314 * \text{variable } 20 * \text{variable } 20 - 0,069675 * \text{variable } 21 + 0,003134 * \text{variable } 21 * \text{variable } 21 - 0,054227857 * \text{variable } 22 + 0,026799499 * \text{variable } 23 - 0,000456787 * \text{variable } 23 * \text{variable } 23 - 1,12341E\text{-}05 * \text{variable } 23 * \text{variable } 23 * \text{variable } 23 - 0,373490667 * \text{variable } 24 - 0,006961823 * \text{variable } 24 * \text{variable } 24 + 0,000774153 * \text{variable } 25 + 0,238619449 * \text{variable } 26 - 0,006139105 * \text{variable } 26 * \text{variable } 26 + 8,53488E\text{-}05 * \text{variable } 27 + 0,016262 * \text{variable } 28 + 1,579488608 * \text{variable } 29,$$

wherein

variable 1 is defined by the patient's age in years,

variable 2 is defined as equal to 1 if the patient's gender is female and as equal to 0 if the patient's gender is male,

variable 3 is defined as equal to 2 if the patient uses a statin and as equal to 1 if the patient does not use a statin,

variable 4 as the patient's body mass index in kg/m$^2$

variable 5 is defined by the value of the patient's serum or plasma concentration of low density lipoprotein (LDL) cholesterol in mg/dl,

variable 6 is defined by the value of the patient's blood concentration of haemoglobin in g/dl,

variable 7 is defined by the value of the patient's blood serum or plasma concentration of glycated haemoglobin in percent (%),

variable 8 is defined by the value of the patient's blood serum or plasma concentration of phosphate in mg/dl,

variable 9 is defined by the value of the patient's serum or plasma activity of alkaline phosphatase in U/L,

variable 10 is defined by the value of the patient's serum or plasma activity of creatine kinase in U/L,

variable 11 is defined by the value of the patient's blood concentration of white blood cells (leucocytes) in 1000/μL,

variable 12 is defined by the value of patient's serum or plasma activity of aspartate aminotransferase (ASAT) in U/L,

variable 13 is defined by the value of patient's serum or plasma activity of alanine aminotransferase (ALAT) in U/L,

variable 14 is defined by the value of the patient's blood, plasma or serum concentration of glucose in mg/dl,

variable 15 is defined by the value of the patient's plasma or serum potassium in mmol/L,

variable 16 is defined by the value of the patient's plasma or serum concentration of N-terminal pro B-type natriuretic peptide in pg/ml,

variable 17 is defined by the value of the patient's plasma or serum concentration of lipoprotein associated phospholipase A2 in U/L,

variable 18 is defined by the value of the patient's plasma or serum concentration of C-reactive protein in mg/L,

variable 19 is defined by the value of the patient's plasma or serum concentration of troponin T in ng/mL,

variable 20 is defined by the value of the patient's plasma or serum concentration of homoarginine in micromol/L,

variable 21 is defined by the value of the patient's plasma or serum concentration of osteoprotegerin in picomol/L,

variable 22 is defined by the value of the patient's plasma or serum concentration of fetuin in ng/mL,

variable 23 is defined by the value of the patient's plasma or serum concentration of vitamin D in ng/ml,

variable 24 is defined by the value of the patient's plasma or serum concentration of transthyretin in micromol/L,

variable 25 is defined by the value of the patient's plasma or serum concentration of aldosterone in pg/mL,

variable 26 is defined by the value of the patient's plasma or serum concentration of cortisol microgram/dL,

variable 27 is defined by the value of the patient's plasma or serum concentration of copeptin (carboxy-terminal-pro arginin vasopressin) in pmol/L,

variable 28 is defined by the value of the patient's plasma or serum concentration of soluble suppression of tumorigenicity 2 (sST2) in ng/mL,

variable 29 is defined by the value of the patient's plasma or serum concentration of carbamylated albumin in percent (%),

wherein all numeric values are calculated per standard deviation, preferably wherein the numeric values are defined with a deviation of 50 %, preferably of 20 %, more preferably of 10 %, and most preferably of 5 %.

4. The method of claim 1, wherein the correlation of a subset of biological parameters provides a risk score indicative for the patient's risk of mortality within the next five years, wherein the biological parameters are selected from the group consisting of

the patient's age,
the patient's gender,
the patient's body mass index,
the patient's use of a statin,
at least one indicator of oxygen transport in the blood,
at least one indicator of calcium-phosphate homeostasis,
at least one indicator of electrolyte homoestasis,
at least one indicator of bone metabolism,
at least one indicator of immune function,
at least one indicator of skeletal muscle metabolism,
at least one indicator of lipid metabolism,
at least one indicator of carbohydrate metabolism,
at least one indicator of hormonal blood pressure regulation,
at least one indicator of vascular inflammation,

at least one indicator of myocardial performance,
at least one indicator of myocardial integrity,
at least one indicator of sudden cardiac death,
at least one indicator of renal function, and
at least one indicator of hepatic function,

wherein

the at least one indicator of lipid metabolism is defined by the patient's blood concentration of LDL cholesterol,

the at least one indicator of oxygen transport is defined by the patient's blood concentration of hemoglobin,

the at least one indicator of carbohydrate metabolism is defined by the patient's blood concentration of glycated haemoglobin or blood, serum or plasma glucose,

the at least one indicator of calcium-phosphate homeostasis is defined by the patient's blood concentration of phosphate,

the at least one indicator of bone metabolism is defined by the patient's blood concentration of alkaline phosphatase and/or by the patient's blood concentration of osteoprotegerin and/or by the patient's blood concentration of vitamin D,

the at least one indicator of skeletal muscle metabolism is defined by the patient's blood concentration or activity of creatine kinase,

the at least one indicator of immune function is defined by the patents blood concentration of white blood cells and/or cortisol and/or soluble suppression of Tumorigenicity 2 (sST2),

the at least one indicator of hepatic function is defined by the patient's blood concentrations or activities of alanine aminotransferase (ALAT) and/or aspartate aminotransferase (ASAT) and/or fetuin and/or transthyretin,

the at least one indicator of electrolyte homeostasis is defined by the patient's blood concentration of potassium,

the at least one indicator of myocardial performance is defined by the patient's blood concentration of a natriuretic peptide,

the at least one indicator of vascular inflammation is defined by the patient's blood concentration or activity of lipoprotein associated phospholipase A2,

the at least one indicator of sudden cardiac death is defined by the patient's blood concentration of homoarginine,

the at least one indicator of myocardial integrity is defined by the patient's blood concentration of troponin T or troponin I,

the at least one indicator of hormonal blood pressure regulation is defined by the patient's blood concentration of aldosterone and or of copeptin,

the at least one indicator of renal function is defined by the patient's blood concentration of carbamylated albumin, wherein the risk score is a weighted risk score $Y_4$ indicative for the patient's risk of mortality within the next five years, and wherein the biological parameters are set into correlation by using the following formula, thereby providing the weighted risk score $Y_4$:

$$Y_4 = x1 * \text{variable } 1 + x2 * \text{variable } 2 + x3 * \text{variable } 3 + x4 * \text{variable } 4 + x5 * \text{variable } 4 * \text{variable } 4 + x6 * \text{variable } 5 + x7 * \text{variable } 5 * \text{variable } 5 + x8 * \text{variable}$$

6 + x9 * variable 7 + x10 * variable 7 * variable 7 + x11 * variable 8 + x12 * variable 9 + x13 * variable 9 * variable 9 + x14 * variable 9 * variable 9 * variable 9 + x15 * variable 10 + x16 * variable 10 * variable 10 + x17 * variable 11 + x18 * variable 11 * variable 11 + x19 * variable 12 + x20 * variable 12 * variable 12 + x21 * variable 12 * variable 12 * variable 12 + x22 * variable 13 + x23 * variable 13 * variable 13 + x24 * variable 14 + x25 * variable 14 * variable 14 + x26 * variable 15 + x27 * variable 15 * variable 15 + x28 * variable 16 + x29 * variable 16 * variable 16 + x30 * variable 17 + x31 variable 17 * variable 17 + x32 * variable 17 * variable 17 * variable 17 + x33 * variable 18 + x34 * variable 18 * variable 18 + x35 * variable 19 + x36 * variable 19 * variable 19 + x37 * variable 20 + x38 * variable 20 * variable 20 + x39 * variable 21 + x40 * variable 21 * variable 21 + x41 * variable 22 + x42 * variable 23 + x43 * variable 23 * variable 23 + x44 * variable 23 * variable 23 * variable 23 + x45 * variable 24 + x46 * variable 24 * variable 24 + x47 * variable 25 + x48 * variable 26 + x49 * variable 26 * variable 26 + x50 * variable 27 + x51 * variable 28 + x52 * variable 29,

wherein

variable 1 is defined by the patient's age in years,
variable 2 is defined as equal to 1 if the patient's gender is female and as equal to 0 if the patient's gender is male,
variable 3 is defined as equal to 2 if the patient uses a statin and as equal to 1 if the patient does not use a statin,
variable 4 as the patient's body mass index in kg/m$^2$
variable 5 is defined by the value of the patient's serum or plasma concentration of low density lipoprotein (LDL) cholesterol in mg/dl,
variable 6 is defined by the value of the patient's blood concentration of haemoglobin in g/dl,
variable 7 is defined by the value of the patient's blood serum or plasma concentration of glycated haemoglobin in percent (%),
variable 8 is defined by the value of the patient's blood serum or plasma concentration of phosphate mg/dl,
variable 9 is defined by the value of the patient's serum or plasma activity of alkaline phosphatase in U/L,
variable 10 is defined by the value of the patient's serum or plasma activity of creatine kinase in U/L,
variable 11 is defined by the value of the patient's blood concentration of white blood cells (leucocytes) in 1000/$\mu$L,
variable 12 is defined by the value of patient's serum or plasma activity of aspartate aminotransferase (ASAT) in U/L,
variable 13 is defined by the value of patient's serum or plasma activity of alanine aminotransferase (ALAT) in U/L,
variable 14 is defined by the value of the patient's blood, plasma or serum concentration of glucose in mg/dl,
variable 15 is defined by the value of the patient' plasma or serum potassium in mmol/L,
variable 16 is defined by the value of the patient's plasma or serum concentration of N-terminal pro B-type natriuretic peptide in pg/mL,
variable 17 is defined by the value of the patient's plasma or serum concentration of lipoprotein associated phospholipase A2 in U/L,
variable 18 is defined by the value of the patient's plasma or serum concentration of C-reactive protein in mg/L,
variable 19 is defined by the value of the patient's plasma or serum concentration of troponin T in ng/mL,
variable 20 is defined by the value of the patient's plasma or serum concentration of homoarginine in micromol/L,
variable 21 is defined by the value of the patient's plasma or serum concentration of osteoprotegerin pmol/L,
variable 22 is defined by the value of the patient's plasma or serum concentration of fetuin in ng/mL,
variable 23 is defined by the value of the patient's plasma or serum concentration of vitamin D in ng/ml,
variable 24 is defined by the value of the patient's plasma or serum concentration of transthyretin in micromol/L,
variable 25 is defined by the value of the patient's plasma or serum concentration of aldosterone in pg/mL,
variable 26 is defined by the value of the patient's plasma or serum concentration of cortisol microgram/dL,

variable 27 is defined by the value of the patient's plasma or serum concentration of copeptin (carboxy-terminal-pro arginin vasopressin) in pmol/L,

variable 28 is defined by the value of the patient's plasma or serum concentration of soluble suppression of Tumorigenicity 2 (sST2) in ng/mL,

variable 29 is defined by the value of the patient's plasma or serum concentration of carbamylated albumin in percent (%),

wherein all numeric values are calculated per standard deviation, preferably wherein the numeric values are defined with a deviation of 50 %, preferably of 20 %, more preferably of 10 %, and most preferably of 5 %, and wherein the coefficients of the risk equation are defined as

$X1 = 0{,}047702681;$
$x2 = -0{,}385079479;$
$x3 = -0{,}170679763;$
$x4 = -0{,}19424517;$
$x5 = 0{,}003364687;$
$x6 = 0{,}038838794;$
$x7 = -0{,}000144754;$
$x8 = -0{,}106157347;$
$x9 = 0{,}286153578;$
$x10 = -0{,}012747406;$
$x11 = 0{,}085284785;$
$x12 = -0{,}001889941;$
$x13 = 2{,}72969E{-}05;$
$x14 = -2{,}22659E{-}08;$
$x15 = -0{,}004498075;$
$x16 = 1{,}69671E{-}06;$
$x17 = -0{,}099378831;$
$x18 = 0{,}006740262;$
$x19 = -0{,}096168983;$
$x20 = 0{,}003300182;$
$x21 = -1{,}37846E{-}05;$
$x22 = 0{,}021377942;$
$x23 = -0{,}000652653;$
$x24 = 0{,}008605366;$
$x25 = -2{,}35966E{-}05;$
$x26 = -0{,}965745662;$
$x27 = 0{,}089391895;$
$x28 = 2{,}73536E{-}06;$
$x29 = 3{,}81425E{-}11;$
$x30 = -0{,}005449139;$
$x31 = 6{,}77085E{-}06;$
$x32 = -1{,}35998E{-}09;$
$x33 = 0{,}013156362;$
$x34 = -0{,}000156612;$
$x35 = 1{,}073159193;$
$x36 = 0{,}080792566;$
$x37 = 0{,}018852403;$
$x38 = -0{,}003042769;$
$x39 = -0{,}068736703;$
$x40 = 0{,}003368983;$
$x41 = -0{,}011378031;$
$x42 = -0{,}073762152;$
$x43 = 0{,}00204079;$
$x44 = -1{,}29568E{-}05;$
$x45 = -0{,}272897733;$
$x46 = 0{,}019087226;$
$x47 = 0{,}00048282;$
$x48 = 0{,}039697765;$

x49 = -0,000875904;
x50 = 0,000956853;
x51 = 0,010142565;
x52 = 0,101680363.

**5.** The method of claim 1, wherein the correlation of a subset of biological parameters provides a risk score indicative for the patient's risk of mortality within the next year, wherein the biological parameters are selected from the group consisting of

the patient's age,
the patient's gender,
the patient's body mass index,
the patient's use of a statin,
at least one indicator of oxygen transport in the blood,
at least one indicator of calcium-phosphate homeostasis,
at least one indicator of electrolyte homoestasis,
at least one indicator of bone metabolism,
at least one indicator of immune function,
at least one indicator of skeletal muscle metabolism,
at least one indicator of lipid metabolism,
at least one indicator of carbohydrate metabolism,
at least one indicator of vascular inflammation,
at least one indicator of myocardial performance,
at least one indicator of myocardial integrity,
at least one indicator of sudden cardiac death,
at least one indicator of renal function,
at least one indicator of hepatic function,

wherein
the at least one indicator of lipid metabolism is defined by the patient's blood concentration of LDL cholesterol,
the at least one indicator of oxygen transport is defined by the patient's blood concentration of hemoglobin,
the at least one indicator of carbohydrate metabolism is defined by the patient's blood concentration of glycated haemoglobin or blood, serum or plasma glucose,
the at least one indicator of calcium-phosphate homeostasis is defined by the patient's blood concentration of phosphate,
the at least one indicator of bone metabolism is defined by the patient's blood concentration of alkaline phosphatase and/or by the patient's blood concentration of vitamin D,
the at least one indicator of skeletal muscle metabolism is defined by the patient's blood concentration or activity of creatine kinase,
the at least one indicator of immune function is defined by the patient's blood concentration of white blood cells and/or soluble suppression of tumorigenicity 2 (sST2),
the at least one indicator of hepatic function is defined by the patient's blood concentrations or activities of alanine aminotransferase (ALAT) and/or aspartate aminotransferase (ASAT) and/or fetuin and/or transthyretin,
the at least one indicator of electrolyte homeostasis is defined by the patient's blood concentration of potassium,
the at least one indicator of myocardial performance is defined by the patient's blood concentration of a natriuretic peptide,
the at least one indicator of vascular inflammation is defined by the patient's blood concentration or activity of lipoprotein associated phospholipase A2,
the at least one indicator of sudden cardiac death is defined by the patient's blood concentration of homoarginine,
the at least one indicator of myocardial integrity is defined by the patient's blood concentration of troponin T or troponin I,
the at least one indicator of renal function is defined by the patient's blood concentration of carbamylated albumin,
wherein the risk score is a weighted risk score $Y_5$ indicative for the patient's risk of mortality within the next year, and wherein the biological parameters are set into correlation by using the following formula, thereby providing the weighted risk score $Y_5$:

$Y_5$ = x1 * variable 1 + x2 * variable 2 + x3 * variable 3 + x4 * variable 4 + x5 * variable 4 * variable 4 + x6 * variable 5 + x7 * variable 5 * variable 5 + x8 * variable 6 + x9 * variable 7 + x10 * variable 7 * variable 7 + x11 * variable 8 + x12 * variable 9 + x13 * variable 9 * variable 9 + x14 * variable 9 * variable 9 * variable 9 + x15 * variable 10 + x16 * variable 10 * variable 10 + x17 * variable 11 + x18 * variable 11 * variable 11 + x19 * variable 12 + x20 * variable 12 * variable 12 + x21 * variable 12 * variable 12 * variable 12 + x22 * variable 13 + x23 * variable 13 * variable 13 + x24 * variable 14 + x25 * variable 14 * variable 14 + x26 * variable 15 + x27 * variable 15 * variable 15 + x28 * variable 16 + x29 * variable 16 * variable 16 + x30 * variable 17 + x31 variable 17 * variable 17 + x32 * variable 17 * variable 17 * variable 17 + x33 * variable 18 + x34 * variable 18 * variable 18 + x35 * variable 19 + x36 * variable 19 * variable 19 + x37 * variable 20 + x38 * variable 20 * variable 20 + x39 * variable 21 + x40 * variable 22 + x41 * variable 22 * variable 22 + x42 * variable 22 * variable 22 * variable 22 + x43 * variable 23 + x44 * variable 23 * variable 23 + x45 * variable 24 + x46 * variable 25.

wherein

variable 1 is defined by the patient's age in years,
variable 2 is defined as equal to 1 if the patient's gender is female and as equal to 0 if the patient's gender is male,
variable 3 is defined as equal to 2 if the patient uses a statin and as equal to 1 if the patient does not use a statin,
variable 4 as the patient's body mass index in kg/m$^2$
variable 5 is defined by the value of the patient's serum or plasma concentration of low density lipoprotein (LDL) cholesterol in mg/dl,
variable 6 is defined by the value of the patient's blood concentration of haemoglobin in g/dl,
variable 7 is defined by the value of the patient's blood serum or plasma concentration of glycated haemoglobin in percent (%),
variable 8 is defined by the value of the patient's blood serum or plasma concentration of phosphate in mg/dl,
variable 9 is defined by the value of the patient's serum or plasma activity of alkaline phosphatase in U/L,
variable 10 is defined by the value of the patient's serum or plasma activity of creatine kinase in U/L,
variable 11 is defined by the value of the patient's blood concentration of white blood cells (leucocytes) in 1000/μL
variable 12 is defined by the value of patient's serum or plasma activity of aspartate aminotransferase (ASAT) in U/L,
variable 13 is defined by the value of patient's serum or plasma activity of alanine aminotransferase (ALAT) in U/L,
variable 14 is defined by the value of the patient's blood, plasma or serum concentration of glucose in mg/dl,
variable 15 is defined by the value of the patient' plasma or serum potassium in mmol/L,
variable 16 is defined by the value of the patient's plasma or serum concentration of N-terminal pro B-type natriuretic peptide in pg/mL,
variable 17 is defined by the value of the patient's plasma or serum concentration of lipoprotein associated phospholipase A2 in U/L,
variable 18 is defined by the value of the patient's plasma or serum concentration of C-reactive protein in mg/L,
variable 19 is defined by the value of the patient's plasma or serum concentration of troponin T in ng/mL,
variable 20 is defined by the value of the patient's plasma or serum concentration of homoarginine in micromol/L,
variable 21 is defined by the value of the patient's plasma or serum concentration of fetuin in ng/mL,
variable 22 is defined by the value of the patient's plasma or serum concentration of vitamin D in ng/ml,
variable 23 is defined by the value of the patient's plasma or serum concentration of transthyretin in micromol/L,
variable 24 is defined by the value of the patient's plasma or serum concentration of soluble suppression of Tumorigenicity 2 (sST2) in ng/mL,
variable 25 is defined by the value of the patient's plasma or serum concentration of carbamylated albumin in percent (%),
wherein all numeric values are calculated per standard deviation, preferably wherein the numeric values are defined with a deviation of 50 %, preferably of 20 %, more preferably of 10 %, and most preferably of 5 %, wherein the coefficients of the risk equation are defined as

$x1 = 0,053386018;$
$x2 = -0,507359962;$
$x3 = -0,159672995;$
$x4 = -0,077278203;$
$x5 = 0,001675282;$
$x5 = 0,035553017;$
$x7 = -0,000122901;$
$x8 = -0,139558759;$
$x9 = 0,63289541;$
$x10 = -0,040388869;$
$x11 = 0,161839595;$
$x12 = -0,002408882;$
$x13 = 4,3439E-05;$
$x14 = -3,73529E-08;$
$x15 = -0,009348889;$
$x16 = 1,00102E-05:$
$x17 = 0,259087904;$
$x18 = -0,010106474;$
$x19 = -0,090758507;$
$x20 = 0,002770596;$
$x21 = -3,26291E-06;$
$x22 = 0,026311865;$
$x23 = -0,00170523;$
$x24 = 0,057829917;$
$x25 = -0,000190269;$
$x26 = -0,278450291;$
$x27 = 0,050511166;$
$x28 = 5,86621E-05;$
$x29 = -1,08488E-09;$
$x30 = -0,041792948;$
$x31 = 5,7281E-05;$
$x32 = -2,18132E-08;$
$x33 = -0,033933381;$
$x34 = 0,000184845;$
$x35 = 1,971444416;$
$x36 = -0,401763801;$
$x37 = 3,417807829;$
$x38 = -1,60701075;$
$x39 = -0,048572426;$
$x40 = -0,001714864;$
$x41 = 0,000628046;$
$x42 = 1,71234E-07;$
$x43 = -0,422328754;$
$x44 = -0,002316355;$
$x45 = 0,016454073.$
$x46 = 1,597285182.$

6. The method of any of the preceding claims, wherein a calculated risk score of
   equal to 1 or less indicates a very low risk of mortality,
   equal to 2 indicates a low risk of mortality,
   equal to 3 indicates an intermediate risk of mortality, and
   equal to 4 or more indicates a high risk of mortality,
   wherein a low risk of mortality means a risk of mortality of equal to or below 20 %, and intermediate risk of mortality means a risk of mortality of equal to or below 40 %, while a high risk of mortality means a risk of mortality of at least 50 %.

7. The method of any of the preceding claims, wherein the risk score is indicative for the patient's mortality due to cardiovascular events or due to the combined fatal and non-fatal cardiovascular events, preferably wherein the risk score is indicative for cardiac mortality, cardiovascular mortality, sudden heart death, death due to acute myocardial infarction, death due to cardiac insufficiency, death due to stroke, death due to bypass operation, death due to coronary angioplasty, death due to cancer, death due to infection, non-fatal myocardial infarction, hospitalization due to any cause, necessity of coronary angioplasty, necessity of coronary bypass surgery, other interventions to treat coronary heart disease, transitory cerebral ischemic attacks, prolonged reversible ischemic neurologic deficits or any composites of these endpoints.

8. The method of any of the preceding claims, wherein the medical treatment to reduce the patient's risk of mortality is in form of administering drugs for treating hyperlipidaemia, for regulating blood pressure, for modifying the patient's heart rate, for providing glycaemic control, for inhibiting blood coagulation, and/or in form of changing the modalities of hemodialysis, preferably by extending the time of dialysis, and/ or by improving the patient's nutritional status, preferably by increasing protein intake.

**Patentansprüche**

1. Verfahren zum Bestimmen des Sterblichkeitsrisikos in Dialysepatienten, umfassend die Schritte von

a) Analysieren eines Satzes von biologischen Parametern, die von dem Patienten oder von der Aktengeschichte des Patienten erhalten wurden, wobei die biologischen Parameter ausgewählt sind aus der Gruppe bestehend aus:

dem Alter des Patienten,
dem Geschlecht des Patienten,
dem Body-Mass-Index des Patienten,
der Verwendung von Statin durch den Patienten,
mindestens einem Indikator von Sauerstofftransport in dem Blut,
mindestens einem Indikator von Calciumphosphathomöostase,
mindestens einem Indikator von Knochenmetabolismus,
mindestens einem Indikator von Immunfunktion,
mindestens einem Indikator von Skelettmuskelmetabolismus,
mindestens einem Indikator von Lipidmetabolismus,
mindestens einem Indikator von Kohlenhydratmetabolismus,
mindestens einem Indikator von hormonaler Regulierung des Blutdrucks,
mindestens einem Indikator von vaskulärer Entzündung,
mindestens einem Indikator von myokardialer Leistung,
mindestens einem Indikator von myokardialer Integrität,
mindestens einem Indikator von plötzlichem Herztod,
mindestens einem Indikator von Nierenfunktion,
mindestens einem Indikator von Leberfunktion oder
einer Untergruppe davon;

b) Setzen der biologischen Parameter oder einer Untergruppe davon in Korrelation, wobei die Korrelation einer Untergruppe von biologischen Parametern einen Risikoscore liefert, der für die Sterblichkeit des Patienten innerhalb des nächsten Jahres indikativ ist, wobei die biologischen Parameter ausgewählt sind aus der Gruppe bestehend aus
dem Alter des Patienten,
dem Geschlecht des Patienten,
dem Body-Mass-Index des Patienten,
mindestens einem Indikator von Skelettmuskelmetabolismus, mindestens einem Indikator von plötzlichem Herztod,
mindestens einem Indikator von Nierenfunktion,
mindestens einem Indikator von hormonaler Regulierung des Blutdrucks,
mindestens einem Indikator von Knochenmetabolismus,

mindestens zwei Indikatoren von Leberfunktion,
wobei der mindestens eine Indikator von Skelettmuskelmetabolismus durch die Blutkonzentration oder -aktivität von Kreatinkinase des Patienten definiert ist,
der mindestens eine Indikator von plötzlichem Herztod durch die Blutkonzentration von Homoarginin des Patienten definiert ist,
der mindestens eine Indikator von Nierenfunktion durch die Blutkonzentration von carbamyliertem Albumin des Patienten definiert ist,
der mindestens eine Indikator von hormoneller Regulierung des Blutdrucks durch die Blutkonzentration von Aldosteron oder Copeptin (carboxyterminales pro-Arginin-Vasopressin) des Patienten definiert ist,
der mindestens eine Indikator von Knochenmetabolismus durch die Blutkonzentration oder Aktivität von alkalischer Phosphatase des Patienten definiert ist, und
die mindestens zwei Indikatoren von Leberfunktion durch die Blutkonzentration von Transthyretin und Fetuin des Patienten definiert sind,
wobei der Risikoscore ein gewichteter Risikoscore $Y_1$ ist, der für das Sterblichkeitsrisiko des Patienten innerhalb des nächsten Jahres indikativ ist, und wobei die biologischen Parameter in Korrelation unter Verwendung der folgenden Formel gesetzt werden, wodurch der gewichtete Risikoscore $Y_1$ bereitgestellt wird:

$$Y_1 = 0{,}51 * \text{Variable 1} + (-0{,}49 * \text{Variable 2}) + (-0{,}32 * \text{Variable 3}) + 0{,}44 * \text{Variable 4} + (-0{,}32 * \text{Variable 5}) + (-0{,}29 * \text{Variable 6}) + (-0{,}33 * \text{Variable 7}) + 0{,}35 * \text{Variable 8} + (-0{,}4 * \text{Variable 9}) + 0{,}31 * \text{Variable 10}$$

wobei

Variable 1 durch den log-Wert des Alters des Patienten in Jahren definiert ist,
Variable 2 als gleich mit 1 definiert ist, wenn das Geschlecht des Patienten weiblich ist, und als gleich mit 0 definiert ist, wenn das Geschlecht des Patienten männlich ist,
Variable 3 durch den log-Wert des Body-Mass-Index des Patienten in kg/m$^2$ definiert ist,
Variable 4 durch den log-Wert der Blutaktivität von alkalischer Phosphatase des Patienten in U/L definiert ist,
Variable 5 durch den log-Wert der Blutaktivität von Kreatinkinase des Patienten in U/L definiert ist,
Variable 6 durch den log-Wert der Blutkonzentration von Homoarginin des Patienten in Mikromol/L definiert ist,
Variable 7 durch den log-Wert der Blutkonzentration von Fetuin des Patienten in ng/ml definiert ist,
Variable 8 durch den log-Wert der Blutkonzentration von carbamyliertem Albumin des Patienten in Prozent (%) definiert ist,
Variable 9 durch den log-Wert der Blutkonzentration von Transthyretin des Patienten in Mikromol/L definiert ist und
Variable 10 durch den log-Wert der Blutkonzentration von Aldosteron des Patienten in pg/mL definiert ist, wobei alle log-Werte auf der Basis e berechnet werden und wobei alle numerischen Werte in der Formel, die den gewichteten Risikoscore $Y_1$ bereitstellen, mit Standardabweichung berechnet werden, bevorzugt wobei die numerischen Werte mit einer Abweichung von 50 %, bevorzugt von 20 %, stärker bevorzugt von 10 % und am meisten bevorzugt von 5 % definiert werden,
und wobei der Risikoscore für eine medizinische Behandlung, um das Sterblichkeitsrisiko des Patienten zu reduzieren, indikativ ist.

**2.** Verfahren von Anspruch 1, wobei die Korrelation einer Untergruppe von biologischen Parametern einen Risikoscore bereitstellt, der für das Sterblichkeitsrisiko des Patienten innerhalb der nächsten fünf Jahre indikativ ist, wobei die biologischen Parameter ausgewählt sind aus der Gruppe bestehend aus
dem Alter des Patienten,
dem Geschlecht des Patienten,
mindestens einem Indikator von Knochenmetabolismus,
mindestens einem Indikator von Skelettmuskelmetabolismus,
mindestens einem Indikator von myokardialer Leistung,
mindestens einem Indikator von myokardialer Integrität,
mindestens einem Indikator von Nierenfunktion und
mindestens einem Indikator von Entzündung,

wobei

der mindestens eine Indikator von Knochenmetabolismus durch die Blutkonzentration oder Aktivität von alkalischer Phosphatase des Patienten definiert ist,

der mindestens eine Indikator von Skelettmuskelmetabolismus durch die Blutkonzentration oder -aktivität von Kreatinkinase des Patienten definiert ist,

der mindestens eine Indikator von myokardialer Leistung durch die Blutkonzentration von NT-proBNP des Patienten definiert ist,

der mindestens eine Indikator der myokardialen Integrität durch die Blutkonzentration von Troponin T oder Troponin I des Patienten definiert ist,

der mindestens eine Indikator von Nierenfunktion durch die Blutkonzentration von carbamyliertem Albumin des Patienten definiert ist,

der mindestens eine Indikator von Entzündung durch die Blutkonzentration von C-reaktivem Protein (CRP) des Patienten definiert ist,

wobei der Risikoscore ein gewichteter Risikoscore $Y_2$ ist, der für das Sterblichkeitsrisiko des Patienten innerhalb der nächsten fünf Jahre indikativ ist, und wobei die biologischen Parameter in Korrelation unter Verwendung der folgenden Formel gesetzt werden, wodurch der gewichtete Risikoscore $Y_2$ bereitgestellt wird:

$$Y_2 = 0{,}38 * \text{Variable 1} + (-0{,}1 * \text{Variable 2}) + 0{,}21 * \text{Variable 3} + (-0{,}34 * \text{Variable 4}) + 0{,}18 * \text{Variable 5} + 0{,}16 * \text{Variable 6} + 0{,}26 * \text{Variable 7} + 0{,}16 * \text{Variable 8,}$$

wobei

Variable 1 durch den log-Wert des Alters des Patienten in Jahren definiert ist,

Variable 2 als gleich mit 1 definiert ist, wenn das Geschlecht des Patienten weiblich ist, und als gleich mit 0 definiert ist, wenn das Geschlecht des Patienten männlich ist,

Variable 3 durch den log-Wert der Blutaktivität von alkalischer Phosphatase des Patienten in U/L definiert ist,

Variable 4 durch den log-Wert der Blutaktivität von Kreatinkinase des Patienten in U/L definiert ist,

Variable 5 durch den log-Wert der Blutkonzentration von NT-proBNP des Patienten in pg/mL definiert ist,

Variable 6 durch den log-Wert der Blutkonzentration von C-reaktivem Protein (CRP) des Patienten in mg/L definiert ist,

Variable 7 durch den log-Wert der Blutkonzentration von Troponin T des Patienten in ng/ml definiert ist und

Variable 8 durch den log-Wert der Blutkonzentration von carbamyliertem Albumin des Patienten in Prozent (%) definiert ist,

wobei alle log-Werte auf der Basis e berechnet werden und wobei alle numerischen Werte in der Formel, die den gewichteten Risikoscore $Y_2$ bereitstellen, mit Standardabweichung berechnet werden, bevorzugt wobei die numerischen Werte mit einer Abweichung von 50 %, bevorzugt von 20 %, stärker bevorzugt von 10 % und am meisten bevorzugt von 5 % definiert werden.

3. Verfahren von Anspruch 1, wobei die Korrelation einer Untergruppe von biologischen Parametern einen Risikoscore bereitstellt, der für das Sterblichkeitsrisiko des Patienten innerhalb des nächsten Jahres indikativ ist, wobei die biologischen Parameter ausgewählt sind aus der Gruppe bestehend aus

dem Alter des Patienten,

dem Geschlecht des Patienten,

dem Body-Mass-Index des Patienten,

der Verwendung von Statin durch den Patienten,

mindestens einem Indikator von Sauerstofftransport in dem Blut,

mindestens einem Indikator von Calciumphosphathomöostase,

mindestens einem Indikator von Elektrolythomöostase,

mindestens einem Indikator von Knochenmetabolismus,

mindestens einem Indikator von Immunfunktion,

mindestens einem Indikator von Skelettmuskelmetabolismus,

mindestens einem Indikator von Lipidmetabolismus,

mindestens einem Indikator von Kohlenhydratmetabolismus,

mindestens einem Indikator von hormonaler Regulierung des Blutdrucks,

mindestens einem Indikator von vaskulärer Entzündung,

mindestens einem Indikator von myokardialer Leistung,
mindestens einem Indikator von myokardialer Integrität,
mindestens einem Indikator von plötzlichem Herztod,
mindestens einem Indikator von Nierenfunktion und
mindestens einem Indikator von Leberfunktion,
wobei
der mindestens eine Indikator von Lipidmetabolismus durch die Blutkonzentration von LDL-Cholesterin des Patienten definiert ist,
der mindestens eine Indikator von Sauerstofftransport durch die Blutkonzentration von Hämoglobin des Patienten definiert ist,
der mindestens eine Indikator von Kohlenhydratmetabolismus durch die Blutkonzentration von glykiertem Hämoglobin oder Blut, Serum oder Plasmaglucose definiert ist,
der mindestens eine Indikator von Calciumphosphathomöostase durch die Blutkonzentration von Phosphat des Patienten definiert ist,
der mindestens eine Indikator von Knochenmetabolismus durch die Blutkonzentration von alkalischer Phosphatase des Patienten und / oder durch die Blutkonzentration von Osteoprotegerin des Patienten und / oder durch die Blutkonzentration von Vitamin D des Patienten definiert ist,
der mindestens eine Indikator von Skelettmuskelmetabolismus durch die Blutkonzentration oder -aktivität von Kreatinkinase des Patienten definiert ist,
der mindestens eine Indikator von Immunfunktion durch die Blutkonzentration von weißen Blutzellen und / oder Cortison und / oder löslicher Suppression von Tumorigenität 2 (sST2) ("soluble suppression of Tumorigenicity 2") definiert ist,
der mindestens eine Indikator von Leberfunktion durch die Blutkonzentrationen oder Aktivitäten von Alaninaminotransferase (ALAT) und / oder Aspartataminotransferase (ASAT) und / oder Fetuin und / oder Transthyretin des Patienten definiert ist,
der mindestens eine Indikator von Elektrolythomöostase durch die Blutkonzentration von Kalium des Patienten definiert ist,
der mindestens eine Indikator von myokardialer Leistung durch die Blutkonzentration eines natriuretischen Peptids definiert ist,
der mindestens eine Indikator von vaskulärer Entzündung durch die Blutkonzentration oder Aktivität von mit Lipoprotein assoziierte Phospholipase A2 des Patienten definiert ist,
der mindestens eine Indikator von plötzlichem Herztod durch die Blutkonzentration von Homoarginin des Patienten definiert ist,
der mindestens eine Indikator von myokardialer Integrität durch die Blutkonzentration von Troponin T oder Troponin I des Patienten definiert ist,
der mindestens eine Indikator von hormoneller Regulierung des Blutdrucks durch die Blutkonzentration von Aldosteron oder von Copeptin des Patienten definiert ist,
der mindestens eine Indikator von Nierenfunktion durch die Blutkonzentration von carbamyliertem Albumin des Patienten definiert ist,
wobei der Risikoscore ein gewichteter Risikoscore $Y_3$ ist, der für das Sterblichkeitsrisiko des Patienten innerhalb des nächsten Jahres indikativ ist, und wobei die biologischen Parameter in Korrelation unter Verwendung der folgenden Formel gesetzt werden, wobei der gewichtete Risikoscore $Y_3$ bereitgestellt wird:

$$Y_3 = 0{,}057410212 * \text{Variable } 1 - 0{,}543915506 * \text{Variable } 2 - 0{,}143998352 * \text{Variable } 3 - 0{,}102420371 * \text{Variable } 4 + 0{,}002126855 * \text{Variable } 4 * \text{Variable } 4 + \quad 0{,}028997043 * \text{Variable } 5 - 0{,}000102574 * \text{Variable } 5 *$$

Variable 5 - 0,144446014* Variable 6 + 0,500608722 * Variable 7 - 0,032782705 * Variable 7 * Variable 7 + 0,141166863 * Variable 8 - 0,004400439 * Variable 9 + 4,87592E-05 * Variable 9 * Variable 9 + - 4,08374E-08 * Variable 9 * Variable 9 * Variable 9 - 0,007492671* Variable 10 + 8,65369E-06 * Variable 10 * Variable 10 + 0,23222716 * Variable 11 - 0,006951655 * Variable 11 * Variable 11 - 0,073850191 * Variable 12 + 0,001124082 * Variable 12 * Variable 12 + 2,88445E-05 * Variable 12 * Variable 12 * Variable 12 + 0,020974682 * Variable 13 - 0,001555684 * Variable 13 * Variable 13 + 0,064258442 * Variable 14 - 0,000208666 * Variable 14 * Variable 14 - 0,439032703 * Variable 15 + 0,064866606 * Variable 15 * Variable 15 + 5,67869E-05 * Variable 16 - 1,15087E-09 * Variable 16 * Variable 16 - 0,043785154 * Variable 17 + 6,01674E-05 Variable 17 * Variable 17 - 2,2989E-08 * Variable 17 * Variable 17 * Variable 17 - 0,035837307 * Variable 18 + 0,000195508 * Variable 18 * Variable 18 + 1,747437912 * Variable 19 - 0,30324001 * Variable 19 * Variable 19 + 3,652306243 * Variable 20 - 1,722577314 * Variable 20 * Variable 20 - 0,069675 * Variable 21 + 0,003134 * Variable 21 * Variable 21 - 0,054227857 * Variable 22 + 0,026799499 * Variable 23 - 0,000456787 * Variable 23 * Variable 23 - 1,12341E-05 * Variable 23 * Variable 23 * Variable 23 - 0,373490667 * Variable 24 - 0,006961823 * Variable 24 * Variable 24 + 0,000774153 * Variable 25 + 0,238619449 * Variable 26 - 0,006139105 * Variable 26 * Variable 26 + 8,53488E-05 * Variable 27 + 0,016262 * Variable 28 + 1,579488608 * Variable 29,

wobei

Variable 1 durch das Alter des Patienten in Jahren definiert ist,
Variable 2 als gleich mit 1 definiert ist, wenn das Geschlecht des Patienten weiblich ist, und als gleich mit 0 definiert ist, wenn das Geschlecht des Patienten männlich ist,
Variable 3 als gleich mit 2 definiert ist, wenn der Patient ein Statin verwendet, und als gleich mit 1 definiert ist, wenn der Patient kein Statin verwendet,
Variable 4 als der Body-Mass-Index des Patienten in kg/m$^2$,
Variable 5 als der Wert der Serum- oder Plasmakonzentration von Cholesterin der Lipoproteine geringer Dichte (LDL) ("low density lipoprotein (LDL) cholesterol") des Patienten in mg/dl definiert ist,
Variable 6 durch den Wert der Blutkonzentration von Hämoglobin des Patienten in g/dl definiert ist,
Variable 7 durch den Wert der Blutserum- oder Plasmakonzentration von glykiertem Hämoglobin des Patienten in Prozent (%) definiert ist,
Variable 8 durch den Wert der Blutserum- oder Plasmakonzentration von Phosphat des Patienten in mg/dl definiert ist,
Variable 9 durch den Wert der Serum- oder Plasma-Aktivität von alkalischer Phosphatase des Patienten in U/L definiert ist,
Variable 10 durch den Wert der Serum- oder Plasma-Aktivität von Kreatinkinase des Patienten in U/L definiert ist,
Variable 11 durch den Wert der Blutkonzentration von weißen Blutzellen (Leukozyten) des Patienten in 1000/$\mu$L definiert ist,
Variable 12 durch den Wert der Serum- oder Plasmaaktivität von Aspartataminotransferase (ASAT) des Patienten in U/L definiert ist,

Variable 13 durch den Wert der Serum- oder Plasmaaktivität von Alaninaminotransferase (ALAT) des Patienten in U/L definiert ist,

Variable 14 durch den Wert der Blut-, Plasma- oder Serumkonzentration von Glucose des Patienten in mg/dl definiert ist,

Variable 15 durch den Wert von Plasma- oder Serumkalium des Patienten in mmol/L definiert ist,

Variable 16 durch den Wert der Plasma- oder Serumkonzentration von N-terminalem pro-B-Typ-natriuretischem Peptid des Patienten in pg/ml definiert ist,

Variable 17 durch den Wert der Plasma- oder Serumkonzentration von mit Lipoprotein assoziierte Phospholipase A2 des Patienten in U/L definiert ist,

Variable 18 durch den Wert der Plasma- oder Serumkonzentration von C-reaktivem Protein des Patienten in mg/L definiert ist,

Variable 19 durch den Wert der Plasma- oder Serumkonzentration von Troponin T des Patienten in ng/ml definiert ist,

Variable 20 durch den Wert der Plasma- oder Serumkonzentration von Homoarginin des Patienten in Mikromol/L definiert ist,

Variable 21 durch den Wert der Plasma- oder Serumkonzentration von Osteoprotegerin des Patienten in Picomol/L definiert ist,

Variable 22 durch den Wert der Plasma- oder Serumkonzentration von Fetuin des Patienten in ng/mL definiert ist,

Variable 23 durch den Wert der Plasma- oder Serumkonzentration von Vitamin D des Patienten in ng/ml definiert ist,

Variable 24 durch den Wert der Plasma- oder Serumkonzentration von Transthyretin des Patienten in Mikromol/L definiert ist,

Variable 25 durch den Wert der Plasma- oder Serumkonzentration von Aldosteron des Patienten in pg/mL definiert ist,

Variable 26 durch den Wert der Plasma- oder Serumkonzentration von Cortison des Patienten in Mikrogramm/dL definiert ist,

Variable 27 durch den Wert der Plasma- oder Serumkonzentration von Copeptin (carboxyterminales pro-Arginin-Vasopressin) des Patienten in pmol/L definiert ist,

Variable 28 durch den Wert der Plasma- oder Serumkonzentration von löslicher Suppression von Tumorigenität 2 (sST2) des Patienten in ng/mL definiert ist,

Variable 29 durch den Wert der Plasma- oder Serumkonzentration von carbamyliertem Albumin des Patienten in Prozent (%) definiert ist,

wobei alle numerischen Werte mit Standardabweichung berechnet werden, bevorzugt wobei die numerischen Werte mit einer Abweichung von 50 %, bevorzugt von 20 %, stärker bevorzugt von 10 % und am meisten bevorzugt von 5 % definiert sind.

4. Verfahren von Anspruch 1, wobei die Korrelation einer Untergruppe von biologischen Parametern einen Risikoscore bereitstellt, der für das Sterblichkeitsrisiko innerhalb der nächsten fünf Jahre indikativ ist, wobei die biologischen Parameter ausgewählt sind aus der Gruppe bestehend aus

dem Alter des Patienten,

dem Geschlecht des Patienten,

dem Body-Mass-Index des Patienten,

der Verwendung von Statin durch den Patienten,

mindestens einem Indikator von Sauerstofftransport in dem Blut,

mindestens einem Indikator von Calciumphosphathomöostase,

mindestens einem Indikator von Elektrolythomöostase,

mindestens einem Indikator von Knochenmetabolismus,

mindestens einem Indikator von Immunfunktion,

mindestens einem Indikator von Skelettmuskelmetabolismus,

mindestens einem Indikator von Lipidmetabolismus,

mindestens einem Indikator von Kohlenhydratmetabolismus,

mindestens einem Indikator von hormonaler Regulierung des Blutdrucks,

mindestens einem Indikator von vaskulärer Entzündung,

mindestens einem Indikator von myokardialer Leistung,

mindestens einem Indikator von myokardialer Integrität,

mindestens einem Indikator von plötzlichem Herztod,

mindestens einem Indikator von Nierenfunktion und

mindestens einem Indikator von Leberfunktion,

wobei

der mindestens eine Indikator von Lipidmetabolismus durch die Blutkonzentration von LDL-Cholesterin des Patienten definiert ist,

der mindestens eine Indikator von Sauerstofftransport durch die Blutkonzentration von Hämoglobin des Patienten definiert ist,

der mindestens eine Indikator von Kohlenhydratmetabolismus durch die Blutkonzentration von glykiertem Hämoglobin oder Blut-, Serum- oder Plasmaglucose des Patienten definiert ist,

der mindestens eine Indikator von Calciumphosphathomöostase durch die Blutkonzentration von Phosphat des Patienten definiert ist,

der mindestens eine Indikator von Knochenmetabolismus durch die Blutkonzentration von alkalischer Phosphatase des Patienten und / oder durch die Blutkonzentration von Osteoprotegerin des Patienten oder durch die Blutkonzentration von Vitamin D des Patienten definiert ist,

der mindestens eine Indikator von Skelettmuskelmetabolismus durch die Blutkonzentration oder -aktivität von Kreatinkinase des Patienten definiert ist,

der mindestens eine Indikator von Immunfunktion durch die Blutkonzentration von weißen Blutzellen und / oder Cortisol und / oder löslicher Suppression von Tumorigenität 2 (sST2) des Patienten definiert ist,

der mindestens eine Indikator von Leberfunktion durch die Blutkonzentrationen oder -aktivitäten von Alaninaminotransferase (ALAT) und / oder Asparatataminotransferase (ASAT) und / oder Fetuin und / oder Transthyretin des Patienten definiert ist,

der mindestens eine Indikator von Elektrolythomöostase durch die Blutkonzentration von Kalium des Patienten definiert ist,

der mindestens eine Indikator von myokardialer Leistung durch die Blutkonzentration eines natriuretischen Peptids des Patienten definiert ist,

der mindestens eine Indikator von vaskulärer Entzündung durch die Blutkonzentration oder -aktivität von mit Lipoprotein assoziierter Phospholipase A2 des Patienten definiert ist,

der mindestens eine Indikator von plötzlichem Herztod durch die Blutkonzentration von Homoarginin des Patienten definiert ist,

der mindestens eine Indikator von myokardialer Integrität durch die Blutkonzentration von Troponin T oder Troponin I des Patienten definiert ist,

der mindestens eine Indikator von hormoneller Regulierung des Blutdrucks durch die Blutkonzentration von Aldosterin und / oder von Copeptin definiert ist,

der mindestens eine Indikator von Nierenfunktion durch die Blutkonzentration von carbamyliertem Albumin des Patienten definiert ist,

wobei der Risikoscore ein gewichteter Risikoscore $Y_4$ ist, der für das Sterblichkeitsrisiko des Patienten innerhalb der nächsten fünf Jahre indikativ ist, und wobei die biologischen Parameter in Beziehung unter Verwendung der folgenden Formel gesetzt werden, wodurch der gewichtete Risikoscore $Y_4$ bereitgestellt wird:

$$Y_4 = x1 * \text{Variable 1} + x2 * \text{Variable 2} + x3 * \text{Variable 3} + x4 * \text{Variable 4} +$$
x5 * Variable 4 * Variable 4 +x6 * Variable 5 + x7 * Variable 5 * Variable 5 + x8 * Variable 6 + x9 * Variable 7 + x10 * Variable 7 * Variable 7 + x11 * Variable 8 + x12 * Variable 9 + x13 * Variable 9 * Variable 9 + x14 * Variable 9 * Variable 9 * Variable 9 + x15 * Variable 10 + x16 * Variable 10 * Variable 10 + x17 * Variable 11 + x18 * Variable 11 * Variable 11 + x19 * Variable 12 + x20 * Variable 12 * Variable 12 + x21 * Variable 12 * Variable 12 * Variable 12 + x22 * Variable 13 + x23 * Variable 13 * Variable 13 + x24 * Variable 14 + x25 * Variable 14 * Variable 14 + x26 * Variable 15 + x27 * Variable 15 * Variable 15 + x28 * Variable 16 + x29 * Variable 16 * Variable 16 + x30 * Variable 17 + x31 Variable 17 * Variable 17 + x32 * Variable 17 * Variable 17 * Variable 17 + x33 * Variable 18 + x34 * Variable 18 * Variable 18 + x35 * Variable 19 + x36 * Variable 19 * Variable 19 + x37 * Variable 20 + x38 * Variable 20 * Variable 20 + x39 * Variable 21 + x40 * Variable 21 * Variable 21 + x41 * Variable 22 + x42 * Variable 23 + x43 * Variable 23 * Variable 23 + x44 * Variable 23 * Variable 23 * Variable 23 + x45 * Variable 24 + x46 * Variable 24 * Variable 24 + x47 * Variable 25

+ x48 * Variable 26 + x49 * Variable 26 * Variable 26 + x50 * Variable 27 + x51 * Variable 28 + x52 * Variable 29,

wobei

Variable 1 durch das Alter des Patienten in Jahren definiert ist,
Variable 2 als gleich mit 1 definiert ist, wenn das Geschlecht des Patienten weiblich ist, und als gleich mit 0 definiert ist, wenn das Geschlecht des Patienten männlich ist,
Variable 3 als gleich mit 2 definiert ist, wenn der Patient ein Statin verwendet, und als gleich mit 1 definiert ist, wenn der Patient kein Statin verwendet,
Variable 4 als der Body-Mass-Index des Patienten in kg/m$^2$,
Variable 5 als der Wert der Serum- oder Plasmakonzentration von Cholesterin der Lipoproteine geringer Dichte (LDL) des Patienten in mg/dl definiert ist,
Variable 6 durch den Wert der Blutkonzentration von Hämoglobin des Patienten in g/dl definiert ist,
Variable 7 durch den Wert der Blutserum- oder Plasmakonzentration von glykiertem Hämoglobin des Patienten in Prozent (%) definiert ist,
Variable 8 durch den Wert der Blutserum- oder Plasmakonzentration von Phosphat des Patienten in mg/dl definiert ist,
Variable 9 durch den Wert der Serum- oder Plasma-Aktivität von alkalischer Phosphatase des Patienten in U/L definiert ist,
Variable 10 durch den Wert der Serum- oder Plasma-Aktivität von Kreatinkinase des Patienten in U/L definiert ist,
Variable 11 durch den Wert der Blutkonzentration von weißen Blutzellen (Leukozyten) des Patienten in 1000/µL definiert ist,
Variable 12 durch den Wert der Serum- oder Plasmaaktivität von Aspartataminotransferase (ASAT) des Patienten in U/L definiert ist,
Variable 13 durch den Wert der Serum- oder Plasmaaktivität von Alaninaminotransferase (ALAT) des Patienten in U/L definiert ist,
Variable 14 durch den Wert der Blut-, Plasma- oder Serumkonzentration von Glucose des Patienten in mg/dl definiert ist,

Variable 15 durch den Wert von Plasma- oder Serumkalium des Patienten in mmol/L definiert ist,

Variable 16 durch den Wert der Plasma- oder Serumkonzentration von N-terminalem pro-B-Typ-natriuretischem Peptid des Patienten in pg/ml definiert ist,

Variable 17 durch den Wert der Plasma- oder Serumkonzentration von mit Lipoprotein assoziierter Phospholipase A2 des Patienten in U/L definiert ist,

Variable 18 durch den Wert der Plasma- oder Serumkonzentration von C-reaktivem Protein des Patienten in mg/L definiert ist,

Variable 19 durch den Wert der Plasma- oder Serumkonzentration von Troponin T des Patienten in ng/ml definiert ist,

Variable 20 durch den Wert der Plasma- oder Serumkonzentration von Homoarginin des Patienten in Mikromol/L definiert ist,

Variable 21 durch den Wert der Plasma- oder Serumkonzentration von Osteoprotegerin des Patienten in Picomol/L definiert ist,

Variable 22 durch den Wert der Plasma- oder Serumkonzentration von Fetuin des Patienten in ng/mL definiert ist,

Variable 23 durch den Wert der Plasma- oder Serumkonzentration von Vitamin D des Patienten in ng/ml definiert ist,

Variable 24 durch den Wert der Plasma- oder Serumkonzentration von Transthyretin des Patienten in Mikromol/L definiert ist,

Variable 25 durch den Wert der Plasma- oder Serumkonzentration von Aldosteron des Patienten in pg/mL definiert ist,

Variable 26 durch den Wert der Plasma- oder Serumkonzentration von Cortison des Patienten in Mikrogramm/dL definiert ist,

Variable 27 durch den Wert der Plasma- oder Serumkonzentration von Copeptin (carboxyterminales pro-Arginin-Vasopressin) des Patienten in pmol/L definiert ist,

Variable 28 durch den Wert der Plasma- oder Serumkonzentration von löslicher Suppression von Tumorigenität 2 (sST2) des Patienten in ng/mL definiert ist,

Variable 29 durch den Wert der Plasma- oder Serumkonzentration von carbamyliertem Albumin des Patienten in Prozent (%) definiert ist,

wobei alle numerischen Werte mit Standardabweichung berechnet werden, bevorzugt wobei die numerischen Werte mit einer Abweichung von 50 %, bevorzugt von 20 %, stärker bevorzugt von 10 % und am meisten bevorzugt von 5 % definiert sind, und

wobei die Koeffizienten der Risikogleichung definiert sind als

$$X1 = 0{,}047702681;$$
$$x2 = -0{,}385079479;$$
$$x3 = -0{,}170679763;$$
$$x4 = -0{,}19424517;$$
$$x5 = 0{,}003364687;$$
$$x6 = 0{,}038836794;$$
$$x7 = -0{,}000144754;$$
$$x8 = -0{,}106157347;$$
$$x9 = 0{,}286153578;$$
$$x10 = -0{,}012747406;$$
$$x11 = 0{,}085284785;$$
$$x12 = -0{,}001889941;$$
$$x13 = 2{,}72969E\text{-}05;$$
$$x14 = -2{,}22659E\text{-}08;$$
$$x15 = -0{,}004498075;$$
$$x16 = 1{,}69671E\text{-}06;$$
$$x17 = -0{,}099378831;$$
$$x18 = 0{,}006740262;$$
$$x19 = -0{,}096168983;$$
$$x20 = 0{,}003300182;$$
$$x21 = -1{,}37846E\text{-}05;$$
$$x22 = 0{,}021377942;$$
$$x23 = -0{,}000652653;$$
$$x24 = 0{,}008605366;$$

x25 = -2,35966E-05;
x26 = -0,965745662;
x27 = 0,089391895;
x28 = 2,73536E-06;
x29 = 3,81425E-11;
x30 = -0,005449139;
x31 = 6,77085E-06;
x32 = -1,35998E-09;
x33 = 0,013156362;
x34 = -0,000156612;
x35 = 1,073159193;
x36 = 0,080792566;
x37 = 0,018852403;
x38 = -0,003042769;
x39 = -0,068736703;
x40 = 0,003368983;
x41 = -0,011378031;
x42 = -0,073762152;
x43 = 0,00204079;
x44 = -1,29568E-05;
x45 = -0,272897733;
x46 = 0,019087226;
x47 = 0,00048282;
x48 = 0,039697765;
x49 = -0,000875904;
x50 = 0,000956853;
x51 = 0,010142565;
x52 = 0,101680363.

5. Verfahren von Anspruch 1, wobei die Korrelation einer Untergruppe von biologischen Parametern einen Risikoscore bereitstellt, der für das Sterblichkeitsrisiko des Patienten innerhalb des nächsten Jahres indikativ ist, wobei die biologischen Parameter ausgewählt sind aus der Gruppe bestehend aus
dem Alter des Patienten,
dem Geschlecht des Patienten,
dem Body-Mass-Index des Patienten,
der Verwendung von Statin durch den Patienten,
mindestens einem Indikator von Sauerstofftransport in dem Blut,
mindestens einem Indikator von Calciumphosphathomöostase,
mindestens einem Indikator von Elektrolythomöostase,
mindestens einem Indikator von Knochenmetabolismus,
mindestens einem Indikator von Immunfunktion,
mindestens einem Indikator von Skelettmuskelmetabolismus,
mindestens einem Indikator von Lipidmetabolismus,
mindestens einem Indikator von Kohlenhydratmetabolismus,
mindestens einem Indikator von vaskulärer Entzündung,
mindestens einem Indikator von myokardialer Leistung,
mindestens einem Indikator von myokardialer Integrität,
mindestens einem Indikator von plötzlichem Herztod,
mindestens einem Indikator von Nierenfunktion,
mindestens einem Indikator von Leberfunktion,
wobei
der mindestens eine Indikator von Lipidmetabolismus durch die Blutkonzentration von LDL-Cholesterin des Patienten definiert ist,
der mindestens eine Indikator von Sauerstofftransport durch die Blutkonzentration von Hämoglobin des Patienten definiert ist,
der mindestens eine Indikator von Kohlenhydratmetabolismus durch die Blutkonzentration von glykiertem Hämoglobin oder Blut-, Serum- oder Plasmaglucose des Patienten definiert ist,
der mindestens eine Indikator von Calciumphosphathomöostase durch die Blutkonzentration von Phosphat des Patienten definiert ist,

der mindestens eine Indikator von Knochenmetabolismus durch die Blutkonzentration von alkalischer Phosphatase des Patienten und / oder durch die Blutkonzentration von Vitamin D des Patienten definiert ist,

der mindestens eine Indikator von Skelettmuskelmetabolismus durch die Blutkonzentration oder -aktivität von Kreatinkinase des Patienten definiert ist,

der mindestens eine Indikator von Immunfunktion durch die Blutkonzentration von weißen Blutzellen und / oder Cortisol und / oder löslicher Suppression von Tumorigenität 2 (sST2) des Patienten definiert ist,

der mindestens eine Indikator von Leberfunktion durch die Blutkonzentrationen oder -aktivitäten von Alaninaminotransferase (ALAT) und / oder Asparatataminotransferase (ASAT) und / oder Fetuin und / oder Transthyretin des Patienten definiert ist,

der mindestens eine Indikator von Elektrolythomöostase durch die Blutkonzentration von Kalium des Patienten definiert ist,

der mindestens eine Indikator von myokardialer Leistung durch die Blutkonzentration eines natriuretischen Peptids des Patienten definiert ist,

der mindestens eine Indikator von vaskulärer Entzündung durch die Blutkonzentration oder -aktivität von mit Lipoprotein assoziierter Phospholipase A2 des Patienten definiert ist,

der mindestens eine Indikator von plötzlichem Herztod durch die Blutkonzentration von Homoarginin des Patienten definiert ist,

der mindestens eine Indikator von myokardialer Integrität durch die Blutkonzentration von Troponin T oder Troponin I des Patienten definiert ist,

der mindestens eine Indikator von Nierenfunktion durch die Blutkonzentration von carbamyliertem Albumin des Patienten definiert ist,

wobei der Risikoscore ein gewichteter Risikoscore $Y_5$ ist, der für das Sterblichkeitsrisiko des Patienten innerhalb des nächsten Jahres indikativ ist, und wobei die biologischen Parameter in Korrelation unter Verwendung der folgenden Formeln gesetzt werden, wodurch der gewichtete Risikoscore $Y_5$ bereitgestellt wird:

$$Y_5 = x1 * \text{Variable } 1 + x2 * \text{Variable } 2 + x3 * \text{Variable } 3 + x4 * \text{Variable } 4 + x5 * \text{Variable } 4 * \text{Variable } 4 + x6 * \text{Variable } 5 + x7 * \text{Variable } 5 * \text{Variable } 5 + x8 * \text{Variable } 6 + x9 * \text{Variable } 7 + x10 * \text{Variable } 7 * \text{Variable } 7 + x11 * \text{Variable } 8 + x12 * \text{Variable } 9 + x13 * \text{Variable } 9 * \text{Variable } 9 + x14 * \text{Variable } 9 * \text{Variable } 9 * \text{Variable } 9 + x15 * \text{Variable } 10 + x16 * \text{Variable } 10 * \text{Variable } 10 + x17 * \text{Variable } 11 + x18 * \text{Variable } 11 * \text{Variable } 11 + x19 * \text{Variable } 12 + x20 * \text{Variable } 12 * \text{Variable } 12 + x21 * \text{Variable } 12 * \text{Variable } 12 * \text{Variable } 12 + x22 * \text{Variable } 13 + x23 * \text{Variable } 13 * \text{Variable } 13 + x24 * \text{Variable } 14 + x25 * \text{Variable } 14 * \text{Variable } 14 + x26 * \text{Variable } 15 + x27 * \text{Variable } 15 * \text{Variable } 15 + x28 * \text{Variable } 16 + x29 * \text{Variable } 16 * \text{Variable } 16 + x30 * \text{Variable } 17 + x31 \text{ Variable } 17 * \text{Variable } 17 + x32 * \text{Variable } 17 * \text{Variable } 17 * \text{Variable } 17 + x33 * \text{Variable } 18 + x34 * \text{Variable } 18 * \text{Variable } 18 + x35 * \text{Variable } 19 + x36 * \text{Variable } 19 * \text{Variable } 19 + x37 * \text{Variable } 20 + x38 * \text{Variable } 20 * \text{Variable } 20 + x39 * \text{Variable } 21 + x40 * \text{Variable } 22 + x41 * \text{Variable } 22 * \text{Variable } 22 + x42 * \text{Variable } 22 * \text{Variable } 22 * \text{Variable } 22 + x43 * \text{Variable } 23 + x44 * \text{Variable } 23 * \text{Variable } 23 + x45 * \text{Variable } 24 + x46 * \text{Variable } 25.$$

wobei

Variable 1 durch das Alter des Patienten in Jahren definiert ist,

Variable 2 als gleich mit 1 definiert ist, wenn das Geschlecht des Patienten weiblich ist, und als gleich mit 0 definiert ist, wenn das Geschlecht des Patienten männlich ist,

Variable 3 als gleich mit 2 definiert ist, wenn der Patient ein Statin verwendet, und als gleich mit 1 definiert ist, wenn der Patient kein Statin verwendet,

Variable 4 als der Body-Mass-Index des Patienten in kg/m$^2$,

Variable 5 als der Wert der Serum- oder Plasmakonzentration von Cholesterin der Lipoproteine geringer Dichte (LDL) des Patienten in mg/dl definiert ist,

Variable 6 durch den Wert der Blutkonzentration von Hämoglobin des Patienten in g/dl definiert ist,

Variable 7 durch den Wert der Blutserum- oder Plasmakonzentration von glykiertem Hämoglobin des Patienten in Prozent (%) definiert ist,

Variable 8 durch den Wert der Blutserum- oder Plasmakonzentration von Phosphat des Patienten in mg/dl definiert ist,

Variable 9 durch den Wert der Serum- oder Plasma-Aktivität von alkalischer Phosphatase des Patienten in U/L definiert ist,

Variable 10 durch den Wert der Serum- oder Plasma-Aktivität von Kreatinkinase des Patienten in U/L definiert ist,

Variable 11 durch den Wert der Blutkonzentration von weißen Blutzellen (Leukozyten) des Patienten in 1000/μL definiert ist,

Variable 12 durch den Wert der Serum- oder Plasmaaktivität von Aspartataminotransferase (ASAT) des Patienten in U/L definiert ist,

Variable 13 durch den Wert der Serum- oder Plasmaaktivität von Alaninaminotransferase (ALAT) des Patienten in U/L definiert ist,

Variable 14 durch den Wert der Blut-, Plasma- oder Serumkonzentration von Glucose des Patienten in mg/dl definiert ist,

Variable 15 durch den Wert von Plasma- oder Serumkalium des Patienten in mmol/L definiert ist,

Variable 16 durch den Wert der Plasma- oder Serumkonzentration von N-terminalem pro-B-Typ-natriuretischem Peptid des Patienten in pg/ml definiert ist,

Variable 17 durch den Wert der Plasma- oder Serumkonzentration von mit Lipoprotein assoziierte Phospholipase A2 des Patienten in U/L definiert ist,

Variable 18 durch den Wert der Plasma- oder Serumkonzentration von C-reaktivem Protein des Patienten in mg/L definiert ist,

Variable 19 durch den Wert der Plasma- oder Serumkonzentration von Troponin T des Patienten in ng/ml definiert ist,

Variable 20 durch den Wert der Plasma- oder Serumkonzentration von Homoarginin des Patienten in Mikromol/L definiert ist,

Variable 21 durch den Wert der Plasma- oder Serumkonzentration von Fetuin des Patienten in ng/mL definiert ist,

Variable 22 durch den Wert der Plasma- oder Serumkonzentration von Vitamin D des Patienten in ng/ml definiert ist,

Variable 23 durch den Wert der Plasma- oder Serumkonzentration von Transthyretin des Patienten in Mikromol/L definiert ist,

Variable 24 durch den Wert der Plasma- oder Serumkonzentration von löslicher Suppression von Tumorigenität 2 (sST2) des Patienten in ng/mL definiert ist,

Variable 25 durch den Wert der Plasma- oder Serumkonzentration von carbamyliertem Albumin des Patienten in Prozent (%) definiert ist,

wobei alle numerischen Werte mit Standardabweichung berechnet werden, bevorzugt wobei die numerischen Werte mit einer Abweichung von 50 %, bevorzugt von 20 %, stärker bevorzugt von 10 % und am meisten bevorzugt von 5 % definiert sind,

wobei die Koeffizienten der Risikogleichung definiert sind als

$x1 = 0,053386018;$
$x2 = -0,507359962;$
$x3 = -0,159672995;$
$x4 = -0,077278203;$
$x5 = 0,001675282;$
$x6 = 0,035553017;$
$x7 = -0,000122901;$
$x8 = -0,139558759;$
$x9 = 0,63289541;$
$x10 = -0,040388869;$
$x11 = 0,161839595;$
$x12 = -0,002408882;$

x13 = 4,3439E-05;
x14 = -3,73529E-08;
x15 = -0,009348889;
x16 = 1,00102E-05;
x17 = 0,259087904;
x18 = -0,010106474;
x19 = -0,090758507;
x20 = 0,002770596;
x21 =-3,26291E-06;
x22 = 0,026311865;
x23 = -0,00170523;
x24 = 0,057829917;
x25 = -0,000190269;
x26 = -0,278450291;
x27 = 0,050511166;
x28 = 5,86621E-05;
x29 = -1,08488E-09;
x30 = -0,041792948;
x31 = 5,7281E-05;
x32 = -2,18132E-08;
x33 = -0,033933381;
x34 = 0,000184845;
x35 = 1,971444416;
x36 = -0,401763801;
x37 = 3,417807829;
x38 = -1,60701075;
x39 = -0,048572426;
x40 = -0,001714864;
x41 = 0,000628046;
x42 = 1,71234E-07;
x43 = -0,422328754;
x44 = -0,002316355;
x45 =0,016454073.
x46 =1,597285182.

6. Verfahren von einem der vorhergehenden Ansprüche, wobei der kalkulierte Risikoscore von
gleich 1 oder weniger ein sehr geringes Sterblichkeitsrisiko anzeigt,
gleich 2 ein geringes Sterblichkeitsrisiko anzeigt,
gleich 3 ein dazwischen liegendes Sterblichkeitsrisiko anzeigt und
gleich 4 ein hohes Sterblichkeitsrisiko anzeigt,
wobei ein geringes Sterblichkeitsrisiko ein Sterblichkeitsrisiko von gleich oder unter 20 %, ein dazwischen liegendes Sterblichkeitsrisiko von gleich oder unter 40 % bedeuten, während ein hohes Sterblichkeitsrisiko ein Sterblichkeitsrisiko von mindestens 50 % bedeutet.

7. Verfahren von einem der vorhergehenden Ansprüche, wobei der Risikoscore indikativ ist für die Sterblichkeit des Patienten aufgrund von kardiovaskulären Ereignissen oder aufgrund von kombinierten tödlichen und nicht-tödlichen kardiovaskulären Ereignissen, bevorzugt wobei der Risikoscore indikativ ist für kardiale Sterblichkeit, kardiovaskuläre Sterblichkeit, plötzlichen Herztod, Tod aufgrund von akutem myokardialem Infarkt, Tod aufgrund von kardialer Insuffizienz, Tod aufgrund von Schlaganfall, Tod aufgrund von Bypassoperation, Tod aufgrund von koronarer Angioplastie, Tod aufgrund von Krebs, Tod aufgrund von Infektion, nicht-tödlichen myokardialen Infarkt, Krankenhausaufenthalt aufgrund einer beliebigen Ursache, Notwendigkeit von koronarer Angioplastie, Notwendigkeit von koronarer Bypassoperation, andere Interventionen, um koronare Herzerkrankung, transitorische zerebrale ischämische Attacken, verlängerte reversible ischämische neurologische Mängel oder irgendwelche Gemische dieser Endpunkte zu behandeln.

8. Verfahren von einem beliebigen der vorhergehenden Ansprüche, wobei die medizinische Behandlung zur Reduzierung des Sterblichkeitsrisikos des Patienten in der Form des Verabreichens von Wirkstoffen zum Behandeln von Hyperlipidämie, zum Regulieren des Blutdrucks, zum Modifizieren der Herzfrequenz des Patienten, zum Bereitstellen einer glykämischen Kontrolle, zum Hemmen von Blutkoagulation und / oder in der Form des Veränderns der Modalitäten von Hämodialyse, bevorzugt durch Verlängern der Dialysezeit und / oder durch Verbessern des Ernährungszustandes des Patienten, bevorzugt durch Erhöhen der Proteinaufnahme, ist.

**Revendications**

1. Procédé de détermination du risque de mortalité chez des patients dialysés, comprenant les étapes de :

    a) analyse d'un ensemble de paramètres biologiques obtenus à partir du patient ou du fichier d'antécédents médicaux du patient, les paramètres biologiques étant choisis dans le groupe constitué de :

    l'âge du patient,
    le sexe du patient,
    l'indice de masse corporelle du patient,
    l'utilisation de statine par le patient,
    au moins un indicateur du transport d'oxygène dans le sang,
    au moins un indicateur de l'homéostase phospho-calcique,
    au moins un indicateur du métabolisme osseux,
    au moins un indicateur de la fonction immunitaire,
    au moins un indicateur du métabolisme des muscles squelettiques,
    au moins un indicateur du métabolisme des lipides,
    au moins un indicateur du métabolisme des glucides,
    au moins un indicateur de la régulation hormonale de la pression sanguine,
    au moins un indicateur d'une inflammation vasculaire,
    au moins un indicateur des performances myocardiques,
    au moins un indicateur de l'intégrité myocardique,
    au moins un indicateur de mort subite d'origine cardiaque,
    au moins un indicateur de la fonction rénale,
    au moins un indicateur de la fonction hépatique,
    ou l'un de leurs sous-ensembles ;

    b) mise en corrélation des paramètres biologiques ou de l'un de leurs sous-ensembles, la corrélation d'un sous-ensemble des paramètres biologiques fournissant un score de risque indicatif de la mortalité du patient au cours de l'année suivante, les paramètres biologiques étant choisis dans le groupe constitué de :

    l'âge du patient,
    le sexe du patient,
    l'indice de masse corporelle du patient,
    au moins un indicateur du métabolisme des muscles squelettiques,
    au moins un indicateur de mort subite d'origine cardiaque,
    au moins un indicateur de la fonction rénale,
    au moins un indicateur de la régulation hormonale de la pression sanguine,
    au moins un indicateur du métabolisme osseux,
    au moins deux indicateurs de la fonction hépatique,

    où :

    l'au moins un indicateur du métabolisme des muscles squelettiques est défini par la concentration ou l'activité sanguine de la créatine kinase du patient,
    l'au moins un indicateur de mort subite d'origine cardiaque est défini par la concentration sanguine d'homoarginine du patient,
    l'au moins un indicateur de la fonction rénale est défini par la concentration sanguine de l'albumine carbamylée du patient,
    l'au moins un indicateur de la régulation hormonale de la pression sanguine est défini par la concentration sanguine de l'aldostérone ou de la copeptine (pro-arginine-vasopressine carboxy-terminale) du patient,
    l'au moins un indicateur du métabolisme osseux est défini par la concentration ou l'activité sanguine de la phosphatase alcaline du patient, et

les au moins deux indicateurs de la fonction hépatique sont définis par la concentration sanguine de la transthyrétine et de la fétuine du patient,

dans lequel le score de risque est un score de risque pondéré $Y_1$ indicatif du risque de mortalité du patient au cours de l'année suivante, et dans lequel les paramètres biologiques sont mis en corrélation en utilisant la formule suivante, fournissant de cette façon le score de risque pondéré $Y_1$ :

$$Y_1 = 0{,}51 * \text{variable 1} + (-0{,}49 * \text{variable 2}) + (-0{,}32 * \text{variable 3}) + 0{,}44 * \text{variable 4} + (-0{,}32 * \text{variable 5}) + (-0{,}29 * \text{variable 6}) + (-0{,}33 * \text{variable 7}) + 0{,}35 * \text{variable 8} + (-0{,}4 * \text{variable 9}) + 0{,}31 * \text{variable 10}$$

dans laquelle :

la variable 1 est définie par la valeur log de l'âge du patient en années,
la variable 2 est définie comme étant égale à 1 si le sexe du patient est féminin et comme étant égale à 0 si le sexe du patient est masculin,
la variable 3 est définie par la valeur log de l'indice de masse corporelle du patient en kg/m$^2$,
la variable 4 est définie par la valeur log de l'activité sanguine de la phosphatase alcaline du patient en U/l,
la variable 5 est définie par la valeur log de l'activité sanguine de la créatine kinase du patient en U/l,
la variable 6 est définie par la valeur log de la concentration sanguine de l'homoarginine du patient en micromol/l,
la variable 7 est définie par la valeur log de la concentration sanguine de la fétuine du patient en ng/ml,
la variable 8 est définie par la valeur log de la concentration sanguine de l'albumine carbamylée du patient en pourcentage (%),
la variable 9 est définie par la valeur log de la concentration sanguine de la transthyrétine du patient en micromol/l, et
la variable 10 est définie par la valeur log de la concentration sanguine de l'aldostérone du patient en pg/ml,

dans lequel toutes les valeurs log sont calculées par rapport à la base e, et dans lequel toutes les valeurs numériques dans la formule fournissant le score de risque pondéré $Y_1$ sont calculées par écart-type, de préférence dans lequel les valeurs numériques sont définies avec un écart de 50 %, de préférence de 20 %, de façon davantage préférée de 10 %, et de façon préférée entre toutes de 5 %,
et dans lequel le score de risque est indicatif pour un traitement médical afin de réduire le risque de mortalité du patient.

2. Procédé selon la revendication 1, dans lequel la corrélation d'un sous-ensemble des paramètres biologiques fournit un score de risque indicatif du risque de mortalité du patient au cours des cinq années suivantes, dans lequel les paramètres biologiques sont choisis dans le groupe constitué de :

l'âge du patient,
le sexe du patient,
au moins un indicateur du métabolisme osseux,
au moins un indicateur du métabolisme des muscles squelettiques,
au moins un indicateur des performances myocardiques,
au moins un indicateur de l'intégrité myocardique,
au moins un indicateur de la fonction rénale, et
au moins un indicateur d'une inflammation,

où :

l'au moins un indicateur du métabolisme osseux est défini par la concentration ou l'activité sanguine de la phosphatase alcaline du patient,
l'au moins un indicateur du métabolisme des muscles squelettiques est défini par la concentration ou l'activité sanguine de la créatine kinase du patient,

l'au moins un indicateur des performances myocardiques est défini par la concentration sanguine du NT-proBNP du patient,

l'au moins un indicateur de l'intégrité myocardique est défini par la concentration sanguine de la troponine T ou de la troponine I du patient,

l'au moins un indicateur de la fonction rénale est défini par la concentration sanguine de l'albumine carbamylée du patient,

l'au moins un indicateur d'une inflammation est défini par la concentration sanguine de la protéine C réactive (CRP) du patient,

dans lequel le score de risque est un score de risque pondéré $Y_2$ indicatif du risque de mortalité du patient au cours des cinq années suivantes, et dans lequel les paramètres biologiques sont mis en corrélation en utilisant la formule suivante, fournissant de cette façon le score de risque pondéré $Y_2$ :

$$Y_2 = 0{,}38 * \text{variable 1} + (-0{,}1 * \text{variable 2}) + 0{,}21 * \text{variable 3} + (-0{,}34 * \text{variable 4}) + 0{,}18 * \text{variable 5} + 0{,}16 * \text{variable 6} + 0{,}26 * \text{variable 7} + 0{,}16 * \text{variable 8}$$

dans laquelle :

la variable 1 est définie par la valeur log de l'âge du patient en années,
la variable 2 est définie comme étant égale à 1 si le sexe du patient est féminin et comme étant égale à 0 si le sexe du patient est masculin,
la variable 3 est définie par la valeur log de l'activité sanguine de la phosphatase alcaline du patient en U/l,
la variable 4 est définie par la valeur log de l'activité sanguine de la créatine kinase du patient en U/l,
la variable 5 est définie par la valeur log de la concentration sanguine du NT-proBNP du patient en pg/ml,
la variable 6 est définie par la valeur log de la concentration sanguine de la protéine C réactive (CRP) du patient en mg/l,
la variable 7 est définie par la valeur log de la concentration sanguine de la troponine T du patient en ng/ml, et
la variable 8 est définie par la valeur log de la concentration sanguine de l'albumine carbamylée du patient en pourcentage (%),

dans lequel toutes les valeurs log sont calculées par rapport à la base e, et dans lequel toutes les valeurs numériques dans la formule fournissant le score de risque pondéré $Y_2$ sont calculées par écart-type, de préférence dans lequel les valeurs numériques sont définies avec un écart de 50 %, de préférence de 20 %, de façon davantage préférée de 10%, et de façon préférée entre toutes de 5 %.

3. Procédé selon la revendication 1, dans lequel la corrélation d'un sous-ensemble des paramètres biologiques fournit un score de risque indicatif du risque de mortalité du patient au cours de l'année suivante, dans lequel les paramètres biologiques sont choisis dans le groupe constitué de :

l'âge du patient,
le sexe du patient,
l'indice de masse corporelle du patient,
l'utilisation d'une statine par le patient,
au moins un indicateur du transport d'oxygène dans le sang,
au moins un indicateur de l'homéostase phospho-calcique,
au moins un indicateur de l'homéostase électrolytique,
au moins un indicateur du métabolisme osseux,
au moins un indicateur de la fonction immunitaire,
au moins un indicateur du métabolisme des muscles squelettiques,
au moins un indicateur du métabolisme des lipides,
au moins un indicateur du métabolisme des glucides,
au moins un indicateur de la régulation hormonale de la pression sanguine,
au moins un indicateur d'une inflammation vasculaire,
au moins un indicateur des performances myocardiques,
au moins un indicateur de l'intégrité myocardique,
au moins un indicateur de mort subite d'origine cardiaque,

au moins un indicateur de la fonction rénale, et
au moins un indicateur de la fonction hépatique,

où :

l'au moins un indicateur du métabolisme des lipides est défini par la concentration sanguine du cholestérol LDL du patient,

l'au moins un indicateur du transport d'oxygène est défini par la concentration sanguine de l'hémoglobine du patient,

l'au moins un indicateur du métabolisme des glucides est défini par la concentration sanguine de l'hémoglobine glyquée ou du glucose sanguin, sérique ou plasmatique du patient,

l'au moins un indicateur de l'homéostase phospho-calcique est défini par la concentration sanguine en phosphate du patient,

l'au moins un indicateur du métabolisme osseux est défini par la concentration sanguine de la phosphatase alcaline du patient et/ou par la concentration sanguine de l'ostéoprotégérine du patient et/ou par la concentration sanguine de la vitamine D du patient,

l'au moins un indicateur du métabolisme des muscles squelettiques est défini par la concentration ou l'activité sanguine de la créatine kinase du patient,

l'au moins un indicateur de la fonction immunitaire est défini par la concentration sanguine des leucocytes et/ou du cortisol et/ou du récepteur soluble de la suppression de la tumorigénicité 2 (sST2) du patient,

l'au moins un indicateur de la fonction hépatique est défini par les concentrations ou activités sanguines de l'alanine-aminotransférase (ALAT) et/ou de l'aspartate-aminotransférase (ASAT) et/ou de la fétuine et/ou de la transthyrétine du patient,

l'au moins un indicateur de l'homéostase électrolytique est défini par la concentration sanguine du potassium du patient,

l'au moins un indicateur des performances myocardiques est défini par la concentration sanguine d'un peptide natriurétique du patient,

l'au moins un indicateur d'une inflammation vasculaire est défini par la concentration ou l'activité sanguine de la phospholipase A2 associée aux lipoprotéines du patient,

l'au moins un indicateur de mort subite d'origine cardiaque est défini par la concentration sanguine de l'homoarginine du patient,

l'au moins un indicateur de l'intégrité myocardique est défini par la concentration sanguine de la troponine T ou de la troponine I du patient,

l'au moins un indicateur de la régulation hormonale de la pression sanguine est défini par la concentration sanguine de l'aldostérone et/ou de la copeptine du patient,

l'au moins un indicateur de la fonction rénale est défini par la concentration sanguine de l'albumine carbamylée du patient,

dans lequel le score de risque est un score de risque pondéré $Y_3$ indicatif du risque de mortalité du patient au cours de l'année suivante, et dans lequel les paramètres biologiques sont mis en corrélation en utilisant la formule suivante, fournissant de cette façon le score de risque pondéré $Y_3$ :

$Y_3 = 0,057410212 *$ variable 1 $- 0,543915506 *$ variable 2 $- 0,143998352 *$ variable 3 $- 0,102420371 *$ variable 4 $+ 0,002126855 *$ variable 4 $*$ variable 4 $+ 0,028997043 *$ variable 5 $- 0,000102574 *$ variable 5 $*$ variable 5 $- 0,144446014 *$ variable 6 $+ 0,500608722 *$ variable 7 $- 0,032782705 *$ variable 7 $*$ variable 7 $+ 0,141166863 *$ variable 8 $- 0,004400439 *$ variable 9 $+ 4,87592E{-}05 *$ variable 9 $*$ variable 9 $+ {-}4,08374E{-}08 *$ variable 9 $*$ variable 9 $*$ variable 9 $- 0,007492671 *$ variable 10 $+ 8,65369E{-}06 *$ variable 10 $*$ variable 10 $+ 0,23222716 *$ variable 11 $- 0,006951655 *$ variable 11 $*$ variable 11 $- 0,073850191 *$ variable 12 $+ 0,001124082 *$ variable 12 $*$

variable 12 + 2,88445E–05 * variable 12 * variable 12 * variable 12 + 0,020974682 * variable 13 – 0,001555684 * variable 13 * variable 13 + 0,064258442 * variable 14 – 0,000208666 * variable 14 * variable 14 – 0,439032703 * variable 15 + 0,064866606 * variable 15 * variable 15 + 5,67869E–05 * variable 16 – 1,15087E–09 * variable 16 * variable 16 – 0,043785154 * variable 17 + 6,01674E–05 variable 17 * variable 17 – 2,2989E–08 * variable 17 * variable 17 * variable 17 – 0,035837307 * variable 18 + 0,000195508 * variable 18 * variable 18 + 1,747437912 * variable 19 – 0,30324001 * variable 19 * variable 19 + 3,652306243 * variable 20 – 1,722577314 * variable 20 * variable 20 – 0,069675 * variable 21 + 0,003134 * variable 21 * variable 21 – 0,054227857 * variable 22 + 0,026799499 * variable 23 – 0,000456787 * variable 23 * variable 23 – 1,12341E–05 * variable 23 * variable 23 * variable 23 – 0,373490667 * variable 24 – 0,006961823 * variable 24 * variable 24 + 0,000774153 * variable 25 + 0,238619449 * variable 26 – 0,006139105 * variable 26 * variable 26 + 8,53488E–05 * variable 27 + 0,016262 * variable 28 + 1,579488608 * variable 29,

dans laquelle :

la variable 1 est définie par l'âge du patient en années,

la variable 2 est définie comme étant égale à 1 si le sexe du patient est féminin et comme étant égale à 0 si le sexe du patient est masculin,

la variable 3 est définie comme étant égale à 2 si le patient utilise une statine et comme étant égale à 1 si le patient n'utilise pas une statine,

la variable 4 est définie comme l'indice de masse corporelle du patient en kg/m$^2$,

la variable 5 est définie par la valeur de la concentration sérique ou plasmatique du cholestérol LDL (lipoprotéines de basse densité) du patient en mg/dl,

la variable 6 est définie par la valeur de la concentration sanguine de l'hémoglobine du patient en g/dl,

la variable 7 est définie par la valeur de la concentration dans le sérum ou le plasma sanguin de l'hémoglobine glyquée du patient en pourcentage (%),

la variable 8 est définie par la valeur de la concentration dans le sérum ou le plasma sanguin en phosphate du patient en mg/dl,

la variable 9 est définie par la valeur de l'activité sérique ou plasmatique de la phosphatase alcaline du patient en U/l,

la variable 10 est définie par la valeur de l'activité sérique ou plasmatique de la créatine kinase du patient en U/l,

la variable 11 est définie par la valeur de la concentration sanguine des leucocytes du patient en 1000/μl,

la variable 12 est définie par la valeur de l'activité sérique ou plasmatique de l'aspartate-aminotransférase (ASAT) du patient en U/l,

la variable 13 est définie par la valeur de l'activité sérique ou plasmatique de l'alanine-aminotransférase (ALAT) du patient en U/l,

la variable 14 est définie par la valeur de la concentration sanguine, plasmatique ou sérique du glucose du patient en mg/dl,

la variable 15 est définie par la valeur de la concentration plasmatique ou sérique du potassium du patient en mmol/l,

la variable 16 est définie par la valeur de la concentration plasmatique ou sérique du peptide natriurétique de type pro-B N-terminal du patient en pg/ml,

la variable 17 est définie par la valeur de la concentration plasmatique ou sérique de la phospholipase A2 associée aux lipoprotéines du patient en U/l,

la variable 18 est définie par la valeur de la concentration plasmatique ou sérique de la protéine C réactive du patient en mg/l,

la variable 19 est définie par la valeur de la concentration plasmatique ou sérique de la troponine T du patient en ng/ml,

la variable 20 est définie par la valeur de la concentration plasmatique ou sérique de l'homoarginine du patient en micromol/l,

la variable 21 est définie par la valeur de la concentration plasmatique ou sérique de l'ostéoprotégérine du patient en picomol/l,

la variable 22 est définie par la valeur de la concentration plasmatique ou sérique de la fétuine du patient en ng/ml,

la variable 23 est définie par la valeur de la concentration plasmatique ou sérique de la vitamine D du patient en ng/ml,

la variable 24 est définie par la valeur de la concentration plasmatique ou sérique de la transthyrétine du patient en micromol/l,

la variable 25 est définie par la valeur de la concentration plasmatique ou sérique de l'aldostérone du patient en pg/ml,

la variable 26 est définie par la valeur de la concentration plasmatique ou sérique du cortisol du patient en microgramme/dl,

la variable 27 est définie par la valeur de la concentration plasmatique ou sérique de la copeptine (pro-arginine-vasopressine carboxy-terminale) du patient en pmol/l,

la variable 28 est définie par la valeur de la concentration plasmatique ou sérique du récepteur soluble de la suppression de la tumorigénicité 2 (sST2) du patient en ng/ml,

la variable 29 est définie par la valeur de la concentration plasmatique ou sérique de l'albumine carbamylée du patient en pourcentage (%),

dans lequel toutes les valeurs numériques sont calculées par écart-type, de préférence dans lequel les valeurs numériques sont définies avec un écart de 50 %, de préférence de 20 %, de façon davantage préférée de 10 %, et de façon préférée entre toutes de 5 %.

**4.** Procédé selon la revendication 1, dans lequel la corrélation d'un sous-ensemble des paramètres biologiques fournit un score de risque indicatif du risque de mortalité du patient au cours des cinq années suivantes, dans lequel les paramètres biologiques sont choisis dans le groupe constitué de :

l'âge du patient,
le sexe du patient,
l'indice de masse corporelle du patient,
l'utilisation d'une statine par le patient,
au moins un indicateur du transport d'oxygène dans le sang,
au moins un indicateur de l'homéostase phospho-calcique,
au moins un indicateur de l'homéostase électrolytique,
au moins un indicateur du métabolisme osseux,
au moins un indicateur de la fonction immunitaire,
au moins un indicateur du métabolisme des muscles squelettiques,
au moins un indicateur du métabolisme des lipides,
au moins un indicateur du métabolisme des glucides,
au moins un indicateur de la régulation hormonale de la pression sanguine,
au moins un indicateur d'une inflammation vasculaire,
au moins un indicateur des performances myocardiques,
au moins un indicateur de l'intégrité myocardique,
au moins un indicateur de mort subite d'origine cardiaque,
au moins un indicateur de la fonction rénale, et
au moins un indicateur de la fonction hépatique,

où :

l'au moins un indicateur du métabolisme des lipides est défini par la concentration sanguine du cholestérol LDL du patient,

l'au moins un indicateur du transport d'oxygène est défini par la concentration sanguine de l'hémoglobine du patient,

l'au moins un indicateur du métabolisme des glucides est défini par la concentration sanguine de l'hémoglobine glyquée ou sanguine, sérique ou plasmatique du glucose du patient,

l'au moins un indicateur de l'homéostase phospho-calcique est défini par la concentration sanguine en phosphate du patient,

l'au moins un indicateur du métabolisme osseux est défini par la concentration sanguine de la phosphatase alcaline du patient et/ou par la concentration sanguine de l'ostéoprotégérine du patient et/ou par la concentration sanguine de la vitamine D du patient,

l'au moins un indicateur du métabolisme des muscles squelettiques est défini par la concentration ou l'activité sanguine de la créatine kinase du patient,

l'au moins un indicateur de la fonction immunitaire est défini par la concentration sanguine des leucocytes et/ou du cortisol et/ou du récepteur soluble de la suppression de la tumorigénicité 2 (sST2) du patient,

l'au moins un indicateur de la fonction hépatique est défini par les concentrations ou activités sanguines de l'alanine-aminotransférase (ALAT) et/ou de l'aspartate-aminotransférase (ASAT) et/ou de la fétuine et/ou de la transthyrétine du patient,

l'au moins un indicateur de l'homéostase électrolytique est défini par la concentration sanguine du potassium du patient,

l'au moins un indicateur des performances myocardiques est défini par la concentration sanguine d'un peptide natriurétique du patient,

l'au moins un indicateur d'une inflammation vasculaire est défini par la concentration ou l'activité sanguine de la phospholipase A2 associée aux lipoprotéines du patient,

l'au moins un indicateur de mort subite d'origine cardiaque est défini par la concentration sanguine de l'homoarginine du patient,

l'au moins un indicateur de l'intégrité myocardique est défini par la concentration sanguine de la troponine T ou de la troponine I du patient,

l'au moins un indicateur de la régulation hormonale de la pression sanguine est défini par la concentration sanguine de l'aldostérone et/ou de la copeptine du patient,

l'au moins un indicateur de la fonction rénale est défini par la concentration sanguine de l'albumine carbamylée du patient,

dans lequel le score de risque est un score de risque pondéré $Y_4$ indicatif du risque de mortalité du patient au cours des cinq années suivantes, et dans lequel les paramètres biologiques sont mis en corrélation en utilisant la formule suivante, fournissant de cette façon le score de risque pondéré $Y_4$ :

$$Y_4 = x1 * \text{variable 1} + x2 * \text{variable 2} + x3 * \text{variable 3} + x4 * \text{variable 4} + x5 * \text{variable 4} * \text{variable 4} + x6 * \text{variable 5} + x7 * \text{variable 5} * \text{variable 5} + x8 *$$

variable 6 + x9 * variable 7 + x10 * variable 7 * variable 7 + x11 * variable 8 + x12 * variable 9 + x13 * variable 9 * variable 9 + x14 * variable 9 * variable 9 * variable 9 + x15 * variable 10 + x16 * variable 10 * variable 10 + x17 * variable 11 + x18 * variable 11 * variable 11 + x19 * variable 12 + x20 * variable 12 * variable 12 + x21 * variable 12 * variable 12 * variable 12 + x22 * variable 13 + x23 * variable 13 * variable 13 + x24 * variable 14 + x25 * variable 14 * variable 14 + x26 * variable 15 + x27 * variable 15 * variable 15 + x28 * variable 16 + x29 * variable 16 * variable 16 + x30 * variable 17 + x31 variable 17 * variable 17 + x32 * variable 17 * variable 17 * variable 17 + x33 * variable 18 + x34 * variable 18 * variable 18 + x35 * variable 19 + x36 * variable 19 * variable 19 + x37 * variable 20 + x38 * variable 20 * variable 20 + x39 * variable 21 + x40 * variable 21 * variable 21 + x41 * variable 22 + x42 * variable 23 + x43 * variable 23 * variable 23 + x44 * variable 23 * variable 23 * variable 23 + x45 * variable 24 + x46 * variable 24 * variable 24 + x47 * variable 25 + x48 * variable 26 + x49 * variable 26 * variable 26 + x50 * variable 27 + x51 * variable 28 + x52 * variable 29,

dans laquelle :

la variable 1 est définie par l'âge du patient en années,

la variable 2 est définie comme étant égale à 1 si le sexe du patient est féminin et comme étant égale à 0 si le sexe du patient est masculin,

la variable 3 est définie comme étant égale à 2 si le patient utilise une statine et comme étant égale à 1 si le patient n'utilise pas une statine,

la variable 4 est définie comme l'indice de masse corporelle du patient en kg/m$^2$,

la variable 5 est définie par la valeur de la concentration sérique ou plasmatique du cholestérol LDL (lipoprotéines de basse densité) du patient en mg/dl,

la variable 6 est définie par la valeur de la concentration sanguine de l'hémoglobine du patient en g/dl,

la variable 7 est définie par la valeur de la concentration dans le sérum ou le plasma sanguin de l'hémoglobine glyquée du patient en pourcentage (%),

la variable 8 est définie par la valeur de la concentration dans le sérum ou le plasma sanguin en phosphate du patient en mg/dl,

la variable 9 est définie par la valeur de l'activité sérique ou plasmatique de la phosphatase alcaline du patient en U/l,

la variable 10 est définie par la valeur de l'activité sérique ou plasmatique de la créatine kinase du patient en U/l,

la variable 11 est définie par la valeur de la concentration sanguine des leucocytes du patient en 1000/μl,

la variable 12 est définie par la valeur de l'activité sérique ou plasmatique de l'aspartate-aminotransférase (ASAT) du patient en U/l,

la variable 13 est définie par la valeur de l'activité sérique ou plasmatique de l'alanine-aminotransférase (ALAT) du patient en U/l,

la variable 14 est définie par la valeur de la concentration sanguine, plasmatique ou sérique du glucose du patient en mg/dl,

la variable 15 est définie par la valeur de la concentration plasmatique ou sérique du potassium du patient en mmol/l,

la variable 16 est définie par la valeur de la concentration plasmatique ou sérique du peptide natriurétique de type pro-B N-terminal du patient en pg/ml,

la variable 17 est définie par la valeur de la concentration plasmatique ou sérique de la phospholipase A2 associée aux lipoprotéines du patient en U/l,

la variable 18 est définie par la valeur de la concentration plasmatique ou sérique de la protéine C réactive du patient en mg/l,

la variable 19 fait définie par la valeur de la concentration plasmatique ou sérique de la troponine T du patient en ng/ml,

la variable 20 est définie par la valeur de la concentration plasmatique ou sérique de l'homoarginine du patient en micromol/l,

la variable 21 est définie par la valeur de la concentration plasmatique ou sérique de l'ostéoprotégérine du patient en pmol/l,

la variable 22 est définie par la valeur de la concentration plasmatique ou sérique de la fétuine du patient en ng/ml,

la variable 23 est définie par la valeur de la concentration plasmatique ou sérique de la vitamine D du patient en ng/ml,

la variable 24 est définie par la valeur de la concentration plasmatique ou sérique de la transthyrétine du patient en micromol/l,

la variable 25 est définie par la valeur de la concentration plasmatique ou sérique de l'aldostérone du patient en pg/ml,

la variable 26 est définie par la valeur de la concentration plasmatique ou sérique du cortisol du patient en microgramme/dl,

la variable 27 est définie par la valeur de la concentration plasmatique ou sérique de la copeptine (pro-arginine-vasopressine carboxy-terminale) du patient en pmol/l,

la variable 28 est définie par la valeur de la concentration plasmatique ou sérique du récepteur soluble de la suppression de la tumorigénicité 2 (sST2) du patient en ng/ml,

la variable 29 est définie par la valeur de la concentration plasmatique ou sérique de l'albumine carbamylée du patient en pourcentage (%),

dans lequel toutes les valeurs numériques sont calculées par écart-type, de préférence dans lequel les valeurs numériques sont définies avec un écart de 50 %, de préférence de 20 %, de façon davantage préférée de 10 %, et de façon préférée entre toutes de 5 %, et

dans lequel les coefficients de l'équation du risque sont définis par :

$x1 = 0,047702681$ ;
$x2 = -0,385079479$ ;
$x3 = -0,170679763$ ;
$x4 = -0,19424517$ ;
$x5 = 0,003364687$ ;
$x6 = 0,038836794$ ;
$x7 = -0,000144754$ ;
$x8 = -0,106157347$ ;
$x9 = 0,286153578$ ;
$x10 = -0,012747406$ ;
$x11 = 0,085284785$ ;
$x12 = -0,001889941$ ;
$x13 = 2,72969\text{E-}05$ ;
$x14 = -2,22659\text{E-}08$ ;
$x15 = -0,004498075$ ;
$x16 = 1,69671\text{E-}06$ ;
$x17 = -0,099378831$ ;
$x18 = 0,006740262$ ;
$x19 = -0,096168983$ ;
$x20 = 0,003300182$ ;
$x21 = -1,37846\text{E-}05$ ;
$x22 = 0,021377942$ ;
$x23 = -0,000652653$ ;
$x24 = 0,008605366$ ;
$x25 = -2,35966\text{E-}05$ ;
$x26 = -0,965745662$ ;
$x27 = 0,089391895$ ;
$x28 = 2,73536\text{E-}06$ ;

x29 = 3,81425E-11 ;
x30 = -0,005449139 ;
x31 = 6,77085E-06 ;
x32 = -1,35998E-09 ;
x33 = 0,013156362 ;
x34 = -0, 000156612 ;
x35 = 1,073159193 ;
x36 = 0,080792566 ;
x37 = 0,018852403 ;
x38 = -0,003042769 ;
x39 = -0,068736703 ;
x40 = 0,003368983 ;
x41 = -0,011378031 ;
x42 = -0,073762152 ;
x43 = 0,00204079 ;
x44 = -1,29568E-05 ;
x45 = -0,272897733 ;
x46 = 0,019087226 ;
x47 = 0,00048282 ;
x48 = 0,039697765 ;
x49 = -0,000875904 ;
x50 = 0,000956853 ;
x51 = 0,010142565 ;
x52 = 0,101680363.

5. Procédé selon la revendication 1, dans lequel la corrélation d'un sous-ensemble des paramètres biologiques fournit un score de risque indicatif du risque de mortalité du patient au cours de l'année suivante, dans lequel les paramètres biologiques sont choisis dans le groupe constitué de :

l'âge du patient,
le sexe du patient,
l'indice de masse corporelle du patient,
l'utilisation d'une statine par le patient,
au moins un indicateur du transport d'oxygène dans le sang,
au moins un indicateur de l'homéostase phospho-calcique,
au moins un indicateur de l'homéostase électrolytique,
au moins un indicateur du métabolisme osseux,
au moins un indicateur de la fonction immunitaire,
au moins un indicateur du métabolisme des muscles squelettiques,
au moins un indicateur du métabolisme des lipides,
au moins un indicateur du métabolisme des glucides,
au moins un indicateur d'une inflammation vasculaire,
au moins un indicateur des performances myocardiques,
au moins un indicateur de l'intégrité myocardique,
au moins un indicateur de mort subite d'origine cardiaque,
au moins un indicateur de la fonction rénale,
au moins un indicateur de la fonction hépatique,

où :

l'au moins un indicateur du métabolisme des lipides est défini par la concentration sanguine du cholestérol LDL du patient,
l'au moins un indicateur du transport d'oxygène est défini par la concentration sanguine de l'hémoglobine du patient,
l'au moins un indicateur du métabolisme des glucides définis par la concentration sanguine de l'hémoglobine glyquée ou sanguine, sérique ou plasmatique du glucose du patient,
l'au moins un indicateur de l'homéostase phospho-calcique définie par la concentration sanguine en phosphate du patient,

l'au moins un indicateur du métabolisme osseux est défini par la concentration sanguine de la phosphatase alcaline du patient et/ou par la concentration sanguine de la vitamine D du patient,

l'au moins un indicateur du métabolisme des muscles squelettiques est défini par la concentration ou l'activité sanguine de la créatine kinase du patient,

l'au moins un indicateur de la fonction immunitaire est défini par la concentration sanguine des leucocytes et/ou du récepteur soluble de la suppression de la tumorigénicité 2 (sST2) du patient,

l'au moins un indicateur de la fonction hépatique est défini par les concentrations ou activités sanguines de l'alanine-aminotransférase (ALAT) et/ou de l'aspartate-aminotransférase (ASAT) et/ou de la fétuine et/ou de la transthyrétine du patient,

l'au moins un indicateur de l'homéostase électrolytique est défini par la concentration sanguine du potassium du patient,

l'au moins un indicateur des performances myocardiques est défini par la concentration sanguine d'un peptide natriurétique du patient,

l'au moins un indicateur d'une inflammation vasculaire est défini par la concentration ou l'activité sanguine de la phospholipase A2 associée aux lipoprotéines du patient,

l'au moins un indicateur de mort subite d'origine cardiaque est défini par la concentration sanguine de l'homoarginine du patient,

l'au moins un indicateur de l'intégrité myocardique est défini par la concentration sanguine de la troponine T ou de la troponine I du patient,

l'au moins un indicateur de la fonction rénale est défini par la concentration sanguine de l'albumine carbamylée du patient,

dans lequel le score de risque est un score de risque pondéré $Y_5$ indicatif du risque de mortalité du patient au cours de l'année suivante, et dans lequel les paramètres biologiques sont mis en corrélation en utilisant la formule suivante, fournissant de cette façon le score de risque pondéré $Y_5$ :

$$Y_5 = x1 * \text{variable } 1 + x2 * \text{variable } 2 + x3 * \text{variable } 3 + x4 * \text{variable } 4 + x5 * \text{variable } 4 * \text{variable } 4 + x6 * \text{variable } 5 + x7 * \text{variable } 5 * \text{variable } 5 + x8 * \text{variable } 6 + x9 * \text{variable } 7 + x10 * \text{variable } 7 * \text{variable } 7 + x11 * \text{variable } 8 + x12 * \text{variable } 9 + x13 * \text{variable } 9 * \text{variable } 9 + x14 * \text{variable } 9 * \text{variable } 9 * \text{variable } 9 + x15 * \text{variable } 10 + x16 * \text{variable } 10 * \text{variable } 10 + x17 * \text{variable } 11 + x18 * \text{variable } 11 * \text{variable } 11 + x19 * \text{variable } 12 + x20 * \text{variable } 12 * \text{variable } 12 + x21 * \text{variable } 12 * \text{variable } 12 * \text{variable } 12 + x22 * \text{variable } 13 + x23 * \text{variable } 13 * \text{variable } 13 + x24 * \text{variable } 14 + x25 * \text{variable } 14 * \text{variable } 14 + x26 * \text{variable } 15 + x27 * \text{variable } 15 * \text{variable } 15 + x28 * \text{variable } 16 + x29 * \text{variable } 16 * \text{variable } 16 + x30 * \text{variable } 17 + x31 * \text{variable } 17 * \text{variable } 17 + x32 * \text{variable } 17 * \text{variable } 17 * \text{variable } 17 + x33 * \text{variable } 18 + x34 * \text{variable } 18 * \text{variable } 18 + x35 * \text{variable } 19 + x36 * \text{variable } 19 * \text{variable } 19 + x37 * \text{variable } 20 + x38 * \text{variable } 20 * \text{variable } 20 + x39 * \text{variable } 21 + x40 * \text{variable } 22 + x41 * \text{variable } 22 * \text{variable } 22 + x42 * \text{variable } 22 * \text{variable } 22 * \text{variable } 22 + x43 * \text{variable } 23 + x44 * \text{variable } 23 * \text{variable } 23 + x45 * \text{variable } 24 + x46 * \text{variable } 25,$$

dans laquelle :

la variable 1 est définie par l'âge du patient en années,
la variable 2 est définie comme étant égale à 1 si le sexe du patient est féminin et comme étant égale à 0 si le sexe du patient est masculin,
la variable 3 est définie comme étant égale à 2 si le patient utilise une statine et comme étant égale à 1 si le patient n'utilise pas une statine,

la variable 4 est définie comme l'indice de masse corporelle du patient en kg/m$^2$,

la variable 5 est définie par la valeur de la concentration sérique ou plasmatique du cholestérol LDL (lipoprotéines de basse densité) du patient en mg/dl,

la variable 6 est définie par la valeur de la concentration sanguine de l'hémoglobine du patient en g/dl,

la variable 7 est définie par la valeur de la concentration dans le sérum ou le plasma sanguin de l'hémoglobine glyquée du patient en pourcentage (%),

la variable 8 est définie par la valeur de la concentration dans le sérum ou le plasma sanguin en phosphate du patient en mg/dl,

la variable 9 est définie par la valeur de l'activité sérique ou plasmatique de la phosphatase alcaline du patient en U/l,

la variable 10 est définie par la valeur de l'activité sérique ou plasmatique de la créatine kinase du patient en U/l,

la variable 11 est définie par la valeur de la concentration sanguine des leucocytes du patient en 1000/µl,

la variable 12 est définie par la valeur de l'activité sérique ou plasmatique de l'aspartate-aminotransférase (ASAT) du patient en U/l,

la variable 13 est définie par la valeur de l'activité sérique ou plasmatique de l'alanine-aminotransférase (ALAT) du patient en U/l,

la variable 14 est définie par la valeur de la concentration sanguine, plasmatique ou sérique du glucose du patient en mg/dl,

la variable 15 est définie par la valeur de la concentration plasmatique ou sérique du potassium du patient en mmol/l,

la variable 16 est définie par la valeur de la concentration plasmatique ou sérique du peptide natriurétique de type pro-B N-terminal du patient en pg/ml,

la variable 17 est définie par la valeur de la concentration plasmatique ou sérique de la phospholipase A2 associée aux lipoprotéines du patient en U/l,

la variable 18 est définie par la valeur de la concentration plasmatique ou sérique de la protéine C réactive du patient en mg/l,

la variable 19 est définie par la valeur de la concentration plasmatique ou sérique de la troponine T du patient en ng/ml,

la variable 20 est définie par la valeur de la concentration plasmatique ou sérique de l'homoarginine du patient en micromol/l,

la variable 21 est définie par la valeur de la concentration plasmatique ou sérique de la fétuine du patient en ng/ml,

la variable 22 est définie par la valeur de la concentration plasmatique ou sérique de la vitamine D du patient en ng/ml,

la variable 23 est définie par la valeur de la concentration plasmatique ou sérique de la transthyrétine du patient en micromol/l,

la variable 24 est définie par la valeur de la concentration plasmatique ou sérique du récepteur soluble de la suppression de la tumorigénicité 2 (sST2) du patient en ng/ml,

la variable 25 est définie par la valeur de la concentration plasmatique ou sérique de l'albumine carbamylée du patient en pourcentage (%),

dans lequel toutes les valeurs numériques sont calculées par écart-type, de préférence dans lequel les valeurs numériques sont définies avec un écart de 50 %, de préférence de 20 %, de façon davantage préférée de 10 %, et de façon préférée entre toutes de 5 %,

dans lequel les coefficients de l'équation du risque sont définis par :

x1 = 0,053386018 ;
x2 = -0,507359962 ;
x3 = -0,159672995 ;
x4 = -0,077278203 ;
x5 = 0,001675282 ;
x6 = 0,035553017 ;
x7 = -0,000122901 ;
x8 = -0,139558759 ;
x9 = 0,63289541 ;
x10 = -0,040388869 ;
x11 = 0,161839595 ;

x12 = -0,002408882 ;
x13 = 4,3439E-05 ;
x14 = -3,73529E-08 ;
x15 = -0,009348889 ;
x16 = 1,00102E-05 ;
x17 = 0,259087904 ;
x18 = -0,010106474 ;
x19 = -0,090758507 ;
x20 = 0,002770596 ;
x21 =-3,26291E-06 ;
x22 = 0,026311865 ;
x23 = -0,00170523 ;
x24 = 0,057829917 ;
x25 = -0,000190269 ;
x26 = -0,278450291 ;
x27 = 0,050511166 ;
x28 = 5,86621E-05 ;
x29 = -1,08488E-09 ;
x30 = -0,041792948 ;
x31 = 5,7281E-05 ;
x32 = -2,18132E-08 ;
x33 = -0,033933381 ;
x34 = 0,000184845 ;
x35 = 1,971444416;
x36 = -0,401763801 ;
x37 = 3,417807829 ;
x38 = -1,60701075 ;
x39 = -0,048572426 ;
x40 = -0,001714864 ;
x41 = 0,000628046 ;
x42 = 1,71234E-07 ;
x43 = -0,422328754 ;
x44 = -0,002316355 ;
x45 = 0,016454073;
x46 = 1,597285182.

**6.** Procédé selon l'une quelconque des revendications précédentes, dans lequel un score de risque calculé :

inférieur ou égal à 1 indique un très faible risque de mortalité,
égal à 2 indique un faible risque de mortalité,
égal à 3 indique un risque intermédiaire de mortalité, et
supérieur ou égal à 4 indique un risque élevé de mortalité,
dans lequel un faible risque de mortalité signifie un risque de mortalité inférieur ou égal à 20 %, et un risque intermédiaire de mortalité signifie un risque de mortalité inférieur ou égal à 40 %, alors qu'un risque élevé de mortalité signifie un risque de mortalité d'au moins 50 %.

**7.** Procédé selon l'une quelconque des revendications précédentes, dans lequel le score de risque est indicatif de la mortalité du patient à cause d'événements cardio-vasculaires ou à cause d'événements cardio-vasculaires fatals et non fatals combinés, de préférence dans lequel le score de risque est indicatif de mortalité cardiaque, de mortalité cardio-vasculaire, de mort subite d'origine cardiaque, de mort due à un infarctus aigu du myocarde, de mort due à une insuffisance cardiaque, de mort due à un accident vasculaire cérébral, de mort due à une opération de pontage, de mort due à une angioplastie coronaire, de mort due à un cancer, de mort due à une infection, d'infarctus du myocarde non fatal, d'hospitalisation due à une cause quelconque, de nécessité d'une angioplastie coronaire, de nécessité d'une chirurgie de pontage coronarien, d'autres interventions pour traiter une coronaropathie, d'attaques ischémiques cérébrales transitoires, de déficits neurologiques ischémiques prolongés réversibles ou de toute composite de ces issues.

**8.** Procédé selon l'une quelconque des revendications précédentes, dans lequel le traitement médical pour réduire le risque de mortalité du patient est sous forme d'administration de médicaments pour traiter l'hyper-lipidémie, pour réguler la pression sanguine, pour modifier le rythme cardiaque du patient, pour fournir un contrôle glycémique, pour inhiber la coagulation sanguine, et/ou sous forme de changement des modalités de l'hémodialyse, de préférence par allongement du temps de dialyse, et/ou par amélioration de l'état nutritionnel du patient, de préférence par augmentation de la prise de protéines.

# Figure 1

## 1 year

| Biomarker | Hazard ratio with 95% CI | p Value | c Statistics |
|---|---|---|---|
| Basic Model | | | 0,71 |
| Transthyretin | | 0,002 | 0,74 |
| Fetuin | | 0,006 | 0,74 |
| Homoarginine | | 0,059 | 0,72 |
| Retinol | | 0,056 | 0,73 |
| Free Trijodthyronine | | 0,089 | 0,71 |
| Troponin T | | 0,057 | 0,72 |
| Aldosteron | | 0,008 | 0,73 |
| NT-pro-BNP | | 0,006 | 0,73 |
| Carbamylated Albumin | | 0,003 | 0,73 |
| Full Model | | | 0.83 |

Hazard ratio with 95% CI

# Figure 2

## 5 years

| Biomarker | Hazard ratio with 95% CI | p Value | c Statistics |
|---|---|---|---|
| Basic Model | | | 0,67 |
| Vitamin D | | 0,004 | 0,68 |
| Transthyretin | | 0,019 | 0,69 |
| Free Trijodthyronine | | 0,032 | 0,67 |
| Retinol | | 0,086 | 0,69 |
| Aldosteron | | 0,085 | 0,68 |
| Parathyroid Hormone | | 0,056 | 0,67 |
| Assymetric Dimethylarginine | | 0,058 | 0,67 |
| Copeptin | | 0,02 | 0,68 |
| Carbamylated Albumin | | 0,016 | 0,68 |
| C-reactive Protein | | 0,003 | 0,67 |
| Troponin T | | < 0,001 | 0,68 |
| NT-pro-BNP | | < 0,001 | 0,68 |
| Full Model | | | 0,71 |

Hazard ratio with 95% CI

# Figure 3A

1 year - Survival

# Figure 3B

5 year - Survival

# Figure 4

**A**

**B**

## REFERENCES CITED IN THE DESCRIPTION

### Patent documents cited in the description

- US 20120103072 A1, Kamyar Kalantar-Zadeh
- WO 2011098519 A1, Maerz
- WO 2012075222 A2, Kotanko
- WO 2007001969 A1, Thadhani
- WO 2013010085 A2, Berg

### Non-patent literature cited in the description

- **WANNER et al.** *N Engl J Med,* 2005, vol. 353 (3), 238-248
- **CARRERO et al.** *Kidney International,* 2011, vol. 79, 749-756
- **BABER et al.** *Am J of Cardiology,* 2009, vol. 103, 1513-1517
- **BALON et al.** *Wien Klin Wochenschr,* 2010, vol. 122, 63-67
- **HUANG et al.** *International Journal of Cardiology,* 2013, vol. 168, 4155-4159
- **DRECHSLER et al.** *Eur J Heart Failure,* 2011, vol. 13, 852-859
- **DOWDY ; WEARDEN.** Statistics for Research. John Wiley & Sons, 1983
- **SCHNABEL et al.** *Eur Heart J,* 2010, vol. 31, 3024-3031
- **BONOW.** *Circulation,* 1996, vol. 93, 1946
- **SMITH.** *J Endocrinol,* 2000, vol. 167, 239
- **MUELLER.** *Clin Chem Lab Med,* 2004, vol. 42, 942
- **WU.** *Clin Chem,* 2004, vol. 50, 867
- **ALA-KOPSALA.** *Clin Chem,* 2004, vol. 50, 1576
- **SCHNABEL et al.** *Eur Heart J,* 2010, vol. 31, 2024-3031